# EUROPEAN PATENT SPECIFICATION

(11) **EP 2 808 396 B1**
(45) Date of publication and mention of the grant of the patent: **06.07.2016**
(21) Application number: 13169548.8
(22) Date of filing: 28.05.2013
(51) Int. Cl.: C12Q 1/68

(54) **Method for identification of honey bees [Apis mellifera] with improved tolerance to brood diseases**
Verfahren zur Identifizierung von Honigbienen [Apis mellifera] mit verbesserter Toleranz für Bruterkrankungen
Procédé d'identification d'abeilles mellifères [l'apis mellifera] avec une tolérance améliorée aux maladies de couvain

(43) Date of publication of application: 03.12.2014
(73) Proprietor: Länderinstitut für Bienenkunde Hohen Neuendorf e. V., 16540 Hohen Neuendorf (DE)
(72) Inventor: Prof. Dr. Bienefeld, Kaspar, 16540 Hohen Neuendorf (DE); Dr. Spötter, Andreas, 13189 Berlin (DE)
(74) Representative: Gulde & Partner

(56) References cited:
- DATABASE EMBL [Online] 6 March 2013 (2013-03-06), Honey bee genome project: "Axo, Apis mellifera", XP002709972, Database accession no. H9KLP5
- DATABASE EMBL [Online] 6 March 2013 (2013-03-06), Honey bee genome project: "Ser, Apis mellifera", XP002709973, Database accession no. H9KDJ9
- A. SPÖTTER ET AL: "Development of a 44K SNP assay focussing on the analysis of a varroa-specific defence behaviour in honey bees (Apis mellifera carnica)", MOLECULAR ECOLOGY RESOURCES, vol. 12, no. 2, 29 March 2012 (2012-03-29) , pages 323-332, XP055073364, ISSN: 1755-098X, DOI: 10.1111/j.1755-0998.2011.03106.x
- Y. LE CONTE ET AL: "Social immunity in honeybees (Apis mellifera): transcriptome analysis of varroa-hygienic behaviour", INSECT MOLECULAR BIOLOGY, vol. 20, no. 3, 24 June 2011 (2011-06-24), pages 399-408, XP055073366, ISSN: 0962-1075, DOI: 10.1111/j.1365-2583.2011.01074.x
- PETER R. OXLEY ET AL: "Six quantitative trait loci influence task thresholds for hygienic behaviour in honeybees ( Apis mellifera )", MOLECULAR ECOLOGY, vol. 19, no. 7, 1 April 2010 (2010-04-01), pages 1452-1461, XP055074128, ISSN: 0962-1083, DOI: 10.1111/j.1365-294X.2010.04569.x
- ROBERT PARKER ET AL: "Correlation of proteome-wide changes with social immunity behaviors provides insight into resistance to the parasitic mite, Varroa destructor, in the honey bee (Apis mellifera)", GENOME BIOLOGY, BIOMED CENTRAL LTD., LONDON, GB, vol. 13, no. 9, 28 September 2012 (2012-09-28), page R81, XP021108544, ISSN: 1465-6906, DOI: 10.1186/GB-2012-13-9-R81
- C. SCHONING ET AL: "Evidence for damage-dependent hygienic behaviour towards Varroa destructor-parasitised brood in the western honey bee, Apis mellifera", JOURNAL OF EXPERIMENTAL BIOLOGY, vol. 215, no. 2, 15 January 2012 (2012-01-15), pages 264-271, XP055073365, ISSN: 0022-0949, DOI: 10.1242/jeb.062562

## Description

Honey bees are exposed to a number of damaging pathogens and parasites. The most destructive among them, affecting mainly the brood, is *Varroa destructor.* A promising approach to prevent its spread is to breed for *Varroa* tolerant honey bees. A trait which has been shown to provide significant resistance against the *Varroa* mite is hygienic behavior, a behavioral response of honey bee workers to brood diseases in general.

The reproduction of *Varroa destructor,* an ectoparasitic mite of the honey bee (*Apis mellifera*), takes place only in capped brood cells of bees and the damage caused to colonies is mainly a consequence of the infestation of pupae. In the capped brood cell, mites puncture the host's integument in order to suck haemolymph. In doing so they are weakening and shortening the life span of pupae upon which they feed. Additionally, several viral diseases are transmitted by *Varroa.* Among others, levels of acute bee paralysis virus (ABPV), slow paralysis virus (SPV), black queen cell virus (BQCV), deformed wing virus (DWV), cloudy wing virus (CWV), and Kashmir bee virus (KBV) have been correlated with mite levels in colonies. Consequently, *Varroa* poses a serious concern and threat to the *A. mellifera* beekeeping industry. In fact, infestation of colonies by *Varroa* is generally considered the most serious problem for beekeeping worldwide.

Different strategies are employed as treatment against *Varroa.* The first is to keep the mite population within tolerable boundaries with the help of acaricides. Their efficiency, however, depends strongly on weather conditions and the time of application. Moreover, residues of these substances accumulate in bee products and mites are developing resistance against them, calling for permanently increasing doses. Additional disadvantages connected with these chemical treatments are high costs and labor.

The second strategy is to breed honey bees for tolerance against *Varroa.* An important factor in these efforts is the honey bees hygienic behavior, which is defined as the ability of honey bee workers to detect and remove pupae infected with brood diseases before the causative organism reaches the infectious stage, thus, limiting the spread of infection. There is evidence that the stimulus for hygienic behavior against *Varroa destructor* is based on odors originating from infected hosts. It has long been known that hygienic behavior confers resistance against American foulbrood and chalkbrood. More recently, it has also been demonstrated that hygienic bees detect and remove brood infested with *Varroa destructor.* The achieved effect is an interruption of the reproductive cycle of the mite.

The heritability of hygienic behavior was estimated in the literature to be in the range of ∼ 0.2 to ∼ 0.6. The estimation of heritability in honey bees is methodically difficult due to reproductive peculiarities as polyandry and haplodiploidy. However, despite the different results of the mentioned estimations, they all show that the trait is heritable to some degree and suggest that there is some potential to propagate the expression of this trait by selective breeding of bees.

Unfortunately, conventional breeding methods are less suitable to reach this aim since the behavior is observable only in workers (exclusively queens and drones are fertile), occurs in a very low frequency and is difficult to measure. A promising way to address this problem is to take advantage of the honey bee genome sequence (Honeybee Genome Sequencing Consortium 2006) in connection with molecular genetic methods. The strategy of choice is to genotype a high number of genetic markers and apply them in linkage or association studies to identify genomic regions implicated in hygienic behavior and ultimately the causative genes themselves. First steps were already taken in the molecular genetic unraveling of hygienic behavior. Some QTL studies with moderate marker numbers exist (e.g. Oxley et al., Molecular Ecology, vol.19, p.1452-1461, 2010). However, to date no study employing a large scale SNP-assay was accomplished and no quantitative trait loci (QTL) or candidate genes identified have been confirmed by other independent studies. Numerous studies have tried to identify informative loci and different results have been achieved. Nonetheless, it is agreed in the literature that there is a strong genetic component involved in the control of hygienic behavior and that variation in this trait is controlled by a small number of loci influencing possibly a bee's sensitivity to the stimulus and setting a certain threshold for every worker when to engage in hygienic behavior. The genetic predisposition for a heightened detection of abnormal brood odors may, therefore, facilitate the expression of hygienic behavior in a colony. Consequently, and even though hygienic behavior can also be influenced to some extent by environmental factors, there is a strong need in elucidation of gene variants controlling this behavior and appears to be a promising approach in the fight against Varroa destructor.

The present invention is directed to a method for the identification of honey bees with above-average hygienic behavior as defined in the claims.

According to the present invention a method is provided that allows for identification of honey bees [Apis mellifera] with above-average hygienic behaviour. Said method is characterized in that, it is determined whether the genome of a honey bee or a group of honey bees comprises:
at least one allele Axo' of the Axo gene, wherein said at least one allele Axo' is characterized by presence of at least one sequence variation as defined in claim 1 compared to the nucleotide sequence of the Axo gene with SEQ ID No. 1;
and/or
   at least one allele Ser' of the Ser gene, wherein said at least one allele Ser' is characterized by presence of at least one sequence variation as defined n claim 1 compared to the nucleotide sequence of the Ser gene with SEQ ID No. 2.

The inventors demonstrate for the first time that the genes Axo and Ser not only reside within the genomic regions correlated with a hereditary component of hygienic behaviour in honey bees, but that sequence variations within these genes are significantly correlated with the trait 'hygienic behaviour'. It has surprisingly been found that the presence of at least one or more particular sequence variations in the Axo gene and/or in the Ser gene correlates well with segregation of improved hygienic behaviour. Honey bees which exhibit at least one such sequence variation in their genome show significantly improved likelihood of above-average hygienic behaviour. Thus, demonstration of the presence of at least one of said alleles exhibiting a sequence variation compared to wild type Axo gene and/or wild type Ser gene appears to be predictive for above-average hygienic behaviour and, as such, for identification of honey bees with improved tolerance to brood diseases like e.g. varroatosis. Upon identification and selection these honey bees may be used in breeding for honey bees with improved tolerance to brood diseases like e.g. varroatosis.

The method of the invention is directed to identification of honey bees with above-average hygienic behaviour. Hygienic behaviour as used for the purpose of the present invention is defined as the ability of honey bee workers to detect and remove pupae infected with brood disease preferably before the causative organism reaches the infectious stage. This understanding of hygienic behaviour as well as methods of determining said behaviour have already been described in the literature, see e.g. Spivak et al. (Spivak M (1996) Hygienic behavior and defense against Varroa jacobsoni. Apidologie, 27, 245-260) and Bienefeld et al. (Bienefeld K, Reinsch N, Thakur RK (2001) Selection for uncapping of Varroa infested brood cells in the honeybee (Apis mellifera). In: Proc 37th Intern Apimondia Congress, Durban. Bucharest: Apimondia Publishing House. pp 12*)*. Above-average hygienic behavior is characterized by a hygienic behavior that is improved compared to the average hygienic behavior observable in honey bee workers of brood disease-sensitive colonies, in particular compared to Varroa-sensitive colonies.

The method of the present invention is characterized in that it is determined whether the genome of a honey bee or a group of honey bees comprises:
at least one allele Axo' of the Axo gene, wherein said at least one allele Axo' is defined by presence of at least one sequence variation as defined in claim 1 compared to the nucleotide sequence of the Axo gene with SEQ ID No. 1;
and/or
   at least one allele Ser' of the Ser gene, wherein said at least one allele Ser' is defined by presence of at least one sequence variation as defined in claim 1 compared to the nucleotide sequence of the Ser gene with SEQ ID No. 2.

The term "sequence variation" is used in its broadest art recognized sense. Thus, the term refers to any alteration of the genomic nucleotide sequence of the respective gene compared to a given reference or wild type sequence. A sequence variation encompasses at least one alteration in the nucleotide sequence which represents a deletion, insertion or point mutation like e.g. a transition or a transversion. The sequence variation may occur in an exon, an intron, the promoter region or in the 3'-untranslated region of the respective genomic sequence. The sequence variation may be silent, i.e. the amino acid sequence of the encoded protein remains unaltered, or non-silent, i.e. the amino acid sequence of the encoded protein is altered compared to the product of the reference or wild type gene. In case of a non-silent sequence variation, the variation may lead to a protein with an altered amino acid sequence like e.g. to a protein with one or more altered amino acids and/or to a prematurely truncated protein.

According to the method of the invention, it is determined whether the genome of a honey bee or a group of honey bees comprises at least one allele *Axo' of* the Axo gene. Said at least one allele *Axo'* is characterized by the presence of at least one sequence variation compared to the nucleotide sequence of the Axo gene with SEQ ID No. 1. In other words, it is determined whether at least one of the alleles of the Axo gene in the genome of the honey bee under investigation exhibits a sequence variation compared to the reference or wild type nucleotide sequence of the Axo gene with SEQ ID No. 1. It has been shown that the presence of such a sequence variation is informative for the hygienic behaviour of said honey bee.

It occurred that specific sequence variations are particularly informative in this context. Thus, the at least one sequence variation of the allele Axo' is one of:
- Axo'883 C→T: = substitution of C with T at position 883 ofAxo allele SEQ ID No. 1; this sequence variation leads to an altered amino acid sequence of the encoded protein, at position 63 of the amino acid sequence of wild type Axo protein with SEQ ID No. 3 His is substituted with Tyr, the resulting Axo' protein, Axo' His63→Tyr, has the amino acid sequence SEQ ID No. 5;
- Axo'899 G→T: = substitution of G with T at position 899 of Axo allele with SEQ ID No. 1; this sequence variation leads to an altered amino acid sequence of the encoded protein, at position 68 of the amino acid sequence of wild type Axo protein with SEQ ID No. 3 Gly is substituted with Val, the resulting Axo' protein, Axo' Gly68→Val, has the amino acid sequence SEQ ID No. 6;
- Axo'1412_→G: = insertion of G at position 1412 ofAxo allele with SEQ ID No. 1;
- Axo'1424_→C: = insertion of C at position 1424 of Axo allele with SEQ ID No. 1;
- Axo'1435_→C: = insertion of C at position 1435 of Axo allele with SEQ ID No. 1;
- Axo'1435_→CGAA: = insertion of CGAA at position 1435 of Axo allele with SEQ ID No. 1;
- Axo'2537 T→C: = substitution of T with C at position 2537 of Axo allele with SEQ ID No. 1; this sequence variation leads to an altered amino acid sequence of the encoded protein, at position 260 of the amino acid sequence of wild type Axo protein with SEQ ID No. 3 IIe is substituted with Thr, the resulting Axo' protein, Axo' Ile260→Thr, has the amino acid sequence SEQ ID No. 7;
- Axo'3759 C→A: = substitution of C with A at position 3759 of Axo allele with SEQ ID No. 1; this sequence variation leads to an altered amino acid sequence of the encoded protein, at position 410 of the amino acid sequence of wild type Axo protein with SEQ ID No. 3 Asp is substituted with Glu, the resulting Axo' protein, Axo' Asp410→Glu, has the amino acid sequence SEQ ID No. 8;
- Axo'3867 T→C: = substitution of T with C at position 3867 of Axo allele with Seq ID No. 1;
- Axo'3909 T→C: = substitution of T with C at position 3909 of Axo allele with Seq ID No. 1;
- Axo'4666 T→C: = substitution of T with C at position 4666 of Axo allele with Seq ID No. 1; this sequence variation leads to an altered amino acid sequence of the encoded protein, at position 610 of the amino acid sequence of wild type Axo protein with SEQ ID No. 3 Phe is substituted with Leu, the resulting Axo' protein, Axo' Phe610→Leu, has the amino acid sequence SEQ ID No. 9;
- Axo'5299 G→A: = substitution of G with A at position 5299 of Axo allele with SEQ ID No. 1; this sequence variation leads to an altered amino acid sequence of the encoded protein, at position 658 of the amino acid sequence of wild type Axo protein with SEQ ID No. 3 Arg is substituted with Lys, the resulting Axo' protein, Axo' Arg658→Lys, has the amino acid sequence SEQ ID No. 10;
- Axo'6139 G→A: = substitution of G with A at position 6139 of Axo allele with SEQ ID No. 1; this sequence variation leads to an altered amino acid sequence of the encoded protein, at position 878 of the amino acid sequence of wild type Axo protein with SEQ ID No. 3 Glu is substituted with Lys, the resulting Axo' protein, Axo' Glu878→Lys, has the amino acid sequence SEQ ID No. 11;
- Axo'7717_→A: = insertion of A at position 7717 of Axo allele with Seq I D No. 1;.
- Axo'8566 G→_: = deletion of G at position 8566 of Axo allele with SEQ ID No. 1; this sequence variation leads to an altered amino acid sequence of the encoded protein, at position 1309 of the amino acid sequence of wild type Axo protein with SEQ ID No. 3 a frame shift occurs and the protein is truncated, the resulting Axo' protein, Axo' Thr1309→fs, has the amino acid sequence SEQ ID No. 12;
- Axo'8566 GT→_A: = substitution of GT with A at position 8566 of Axo allele with SEQ ID No. 1; this sequence variation leads to an altered amino acid sequence of the encoded protein, at position 1309 of the amino acid sequence of wild type Axo protein with SEQ ID No. 3 a frame shift occurs and the protein is truncated, the resulting Axo' protein, Axo' Thr1309→fs, has the amino acid sequence SEQ ID No. 13;
- Axo'8567 T→A: = substitution of T with A at position 8567 of Axo allele with SEQ ID No. 1; this sequence variation leads to an altered amino acid sequence of the encoded protein, at position 1310 of the amino acid sequence of wild type Axo protein with SEQ ID No. 3 Leu is substituted with Met, the resulting Axo' protein, Axo' Leu1310→Met, has the amino acid sequence SEQ ID No. 14;
- Axo'8851 G→A: = substitution of G with A at position 8851 of Axo allele with SEQ ID No. 1; this sequence variation leads to an altered amino acid sequence of the encoded protein, at position 1384 of the amino acid sequence of wild type Axo protein with SEQ ID No. 3 Ala is substituted with Thr, the resulting Axo' protein, Axo'Ala1384→Thr, has the amino acid sequence SEQ ID No. 15;
- Axo'10755 C→T: = substitution of C with T at position 10755 of Axo allele with Seq ID No. 1;
- Axo'11325 G→A: = substitution of G with A at position 11325 of Axo allele with SEQ ID No. 1; this sequence variation leads to an altered amino acid sequence of the encoded protein, at position 1668 of the amino acid sequence of wild type Axo protein with SEQ ID No. 3 Val is substituted with Met, the resulting Axo' protein, Axo' Val1668→Met, has the amino acid sequence SEQ ID No. 16;
- Axo'12909 G→A: = substitution of G with A at position 12909 of Axo allele with SEQ ID No. 1; this sequence variation leads to an altered amino acid sequence of the encoded protein, at position 1855 of the amino acid sequence of wild type Axo protein with SEQ ID No. 3 Asp is substituted with Asn, the resulting Axo' protein, Axo' Asp1855→Asn, has the amino acid sequence SEQ ID No. 17;
- Axo'12924 A→G: = substitution of A with G at position 12924 of Axo allele with SEQ ID No. 1; this sequence variation leads to an altered amino acid sequence of the encoded protein, at position 1860 of the amino acid sequence of wild type Axo protein with SEQ ID No. 3 Asn is substituted with Asp, the resulting Axo' protein, Axo' Asn1860→Asp, has the amino acid sequence SEQ ID No. 18;
- Axo'12924_12926 AAT→GAC: = substitution of AAT with GAC at position 12924 to 12926 of Axo allele with SEQ ID No. 1; this sequence variation leads to an altered amino acid sequence of the encoded protein, at position 1860 of the amino acid sequence of wild type Axo protein with SEQ ID No. 3 Asn is substituted with Asp, the resulting Axo' protein, Axo' Asn1860→Asp, has the amino acid sequence SEQ ID No. 19;
- Axo'13984 G→A: = substitution of G with A at position 13984 of Axo allele with SEQ ID No. 1; this sequence variation leads to an altered amino acid sequence of the encoded protein, at position 1899 of the amino acid sequence of wild type Axo protein with SEQ ID No. 3 Val is substituted with Ile, the resulting Axo' protein, Axo' Val1899→Ile, has the amino acid sequence SEQ ID No. 20;
and/or combinations thereof.

The at least one allele Axo' may contain more than one sequence variation compared to the nucleotide sequence of the Axo gene with SEQ ID No. 1.

Alternatively or in addition to determining the presence of an Axo' allele, in the method of the invention, it is determined whether the genome of a honey bee or a group of honey bees comprises at least one allele Ser' of the Ser gene. Said at least one allele Ser' is characterized by presence of at least one sequence variation compared to the nucleotide sequence of the Ser gene with SEQ ID No. 2. In other words, it is determined whether at least one of the alleles of the Ser gene in the genome of the honey bee under investigation exhibits a sequence variation compared to the reference or wild type nucleotide sequence of the Ser gene with SEQ ID No. 2. It has been shown that the presence of such a sequence variation is informative for the hygienic behaviour of said honey bee.

It occurred that specific sequence variations are particularly informative in this context. Thus, the at least one sequence variation of the allele Ser' is one of:
- Ser'17193 G→A: = substitution of G with A at position 17139 of Ser allele with SEQ ID No. 2; this sequence variation leads to an altered amino acid sequence of the encoded protein, at position 66 of the amino acid sequence of wild type Ser protein with SEQ ID No. 4 Ala is substituted with Thr, the resulting Ser' protein, Ser' Ala66→Thr, has the amino acid sequence SEQ ID No. 21;
- Ser'40832 T→C: = substitution of T with C at position 40832 of Ser allele with Seq ID No. 2;
- Ser'43680 C→T: = substitution of C with T at position 43680 of Ser allele with SEQ ID No. 2; this sequence variation leads to an altered amino acid sequence of the encoded protein, at position 1090 of the amino acid sequence of wild type Ser protein with SEQ ID No. 4 Leu is substituted with Phe, the resulting Ser' protein, Ser' Leu1090→Phe, has the amino acid sequence SEQ ID No. 22;
- Seer'43709_43711 CGC→TGT: = substitution of CGC with TGT at position 43709 to 43711 of Ser allele with SEQ ID No. 2; this sequence variation leads to an altered amino acid sequence of the encoded protein, at position 1099 and 1100 of the amino acid sequence of wild type Ser protein with SEQ ID No. 4 wherein Tyr1099 and Ala1100 are deleted, the resulting Ser' protein, Ser' Tyr1099_Ala1100→del, has the amino acid sequence SEQ ID No. 23;
- Ser'43710 G→A: = substitution of G with A at position 43710 of Ser allele with SEQ ID No. 2; this sequence variation leads to an altered amino acid sequence of the encoded protein, at position 1100 of the amino acid sequence of wild type Ser protein with SEQ ID No. 4 Ala is substituted with Thr, the resulting Ser' protein, Ser' Ala1100→Thr, has the amino acid sequence SEQ ID No. 24;
- Ser'43711 C→T: = substitution of C with T at position 43711 of Ser allele with SEQ ID No. 2; this sequence variation leads to an altered amino acid sequence of the encoded protein, at position 1100 of the amino acid sequence of wild type Ser protein with SEQ ID No. 4 Ala is substituted with Val, the resulting Ser' protein, Ser' Ala1100→Val, has the amino acid sequence SEQ ID No. 25;
- Ser'43939 C→T: = substitution of C with T at position 43939 of Ser allele with SEQ ID No. 2; this sequence variation leads to an altered amino acid sequence of the encoded protein, at position 1176 of the amino acid sequence of wild type Ser protein with SEQ ID No. 4 Pro is substituted with Leu, the resulting Ser' protein, Ser' Pro1176→Leu, has the amino acid sequence SEQ ID No. 26;
and/or combinations thereof.

The at least one allele Ser' may contain more than one sequence variation compared to the nucleotide sequence of the Ser gene with SEQ ID No. 2.

The method of the invention may comprise the following steps:
- isolation of a biological sample of one or more honey bee individuals;
- determining whether the genome of a honey bee or a group of honey bees comprises:
   at least one allele Axo' of the Axo gene, wherein said at least one allele Axo' is characterized by the presence of at least one sequence variation as defined in claim 2 compared to the nucleotide sequence of the Axo gene with SEQ ID No. 1;
   and/or
      at least one allele Ser' of the Ser gene, wherein said at least one allele Ser' is characterized by the presence of at least one sequence variation as defined in claim 2 compared to the nucleotide sequence of the Ser gene with SEQ ID No. 2; and
- selecting one or more honey bee individuals, wherein its genome comprises at least one allele of Axo' and/or of Ser'.

The skilled person is well aware of suitable methods to isolate a biological sample of one or more honey bee individuals. The method of choice will depend on the assay used for determining the presence of at least one of the alleles Axo' and/or Ser'. The biological sample may comprise biological material that allows conclusion as to whether the biological sample is derived from a honey bee with a genome comprising at least one allele Axo' and/or at least one allele Ser'. Such biological material may be e.g. genomic nucleic acids, transcribed nucleic acids and/or translated proteins. Preferably, the biological sample is isolated in such a way that the donor honey bee survives the procedure and is available for subsequent investigation or breeding. It is particularly beneficial if in the method of the invention the biological sample is isolated from a honey bee queen and/or drone and that said isolation is performed such that the respective donor individual survives the procedure and is available for further investigation and/or breeding.

There are numerous methods readily available to the skilled person in order to reliably determine whether the genome of a honey bee or of a group of honey bees comprises at least one allele Axo' and/or at least one allele Ser'. The method of the present invention is not limited to a particular assay to determine presence of the said alleles Axo' and/or Ser' but can be practiced using any known method suitable for detection of a particular allele. Selection and adaptation of a known method is well within the scope of routine work of a skilled person. Basically, it is possible to determine the presence of the at least one allele Axo' and/or the at least one allele of Ser' by a nucleic acid-based or an amino acid-based assay. The presence of such alleles can be determined in a more direct way based on genomic nucleic acid material. Using suitable assays, the skilled person can determine directly whether the genome of a honey bee or of a group of honey bees exhibits at least one Axo' and/or Ser' allele by analyzing genomic nucleic acid material as such. It is also possible to determine presence of said alleles by analyzing nucleic acid material which comprises transcribed nucleic acids, provided the sequence variation is present within the transcribed part of the Axo' allele and/or Ser' allele. Axo' and/or Ser' alleles wherein the at least one sequence variation yields a protein with altered amino acid sequence can also be detected based on amino acid-based assays. The presence of the at least one Axo' and/or the at least one Ser' allele can preferably be determined by a method selected from PCR-based, hybridisation-based (like assays based on specific hybridisation to mobile or immobilized bait or catcher molecules, e.g. to specific complementary nucleic acid molecules that may be immobilized to a solid support like in chip-assays or blotting techniques) and/or sequencing-based methods and/or by heteroduplex-formation, SSCP (single strand conformation polymorphism)- and/or RFLP (restriction fragment length polymorphism)-analysis. There is ample support to guide the skilled person in adapting a particular method to the detection of the alleles specified as Axo' and/or Ser'.

In a further aspect, it is disclosed an isolated nucleic acid which hybridizes specifically under stringent conditions to a nucleic acid sequence of the allele Axo' or a reverse complement thereof but not to the allele Axo with a nucleic acid sequence of SEQ ID No. 1 or to its reverse complement.

In another aspect, it is disclosed an isolated nucleic acid hybridizing specifically under stringent conditions to a nucleic acid sequence of the allele Ser' or a reverse complement thereof but not to the allele Ser with a nucleic acid sequence of SEQ ID No. 2 or to its reverse complement.

The isolated nucleic acids may be used as detection means in a method of the invention in order to determine presence of an Axo' and/or Ser' allele in the genome of a honey bee or a group of honey bees.

The term "hybridizing specifically under stringent conditions" is to be understood broadly and means hybridization of the nucleic acid to its respective target sequence specifically while it is not hybridizing to its respective reference target sequence. Suitable hybridization conditions are described inter alia in Sambrook J, Fritsch E F, Maniatis T et al., in Molecular Cloning-A Laboratory Manual, 2nd edition, Cold Spring Harbor Laboratory Press, 1989, pages 9.31-9.57 or in Current Protocols in Molecular Biology, John Wiley & Sons, N.Y. (1989), 6.3.1-6.3.6.

An "isolated" nucleic acid is one that is substantially separated from other nucleic acid molecules, which are present in the natural source of the nucleic acid (e.g., sequences encoding other polypeptides). For example, a cloned nucleic acid is considered isolated. A nucleic acid is also considered isolated if it has been altered or manufactured by human intervention. Moreover, an "isolated" nucleic acid, such as a DNA molecule, can be free from some of the other cellular material with which it is naturally associated, or culture medium when produced by recombinant techniques, or chemical precursors or other chemicals when chemically synthesized. Specifically excluded from the definition of "isolated nucleic acid" are: naturally- occurring chromosomes (such as chromosome spreads), genomic libraries, and whole cell genomic DNA or whole cell RNA preparations of naturally occurring sources (including whole cell preparations that are mechanically sheared or enzymatically digested). Nucleic acids and/or peptides may be provided in isolated form, i.e. purified from their natural environment, preferably in substantially pure or homogeneous form or free or substantially free of nucleic acid or genes of the species of origin other than the desired sequence.

Nucleic acid may include DNA, RNA, mixtures and/or functional substituents thereof, particularly cDNA, genomic DNA and RNA and may be wholly or partially synthetic. The nucleic acids comprise single stranded or wholly or partially double stranded poly-nucleotide sequences. The term "isolated" encompasses all these possibilities. For the purpose of the present invention, where a DNA sequence is specified, e.g. with reference to a particular SEQ ID No., unless the context requires otherwise, the reverse complement thereof is encompassed. The nucleic acid may be produced by any means, including genomic preparations, cDNA preparations, in vitro synthesis, PCR, RT-PCR, and/or in vitro or in vivo transcription.

In a further aspect, it is disclosed an isolated peptide encoded by an allele Axo' or Ser' with the proviso that the at least one sequence variation of the allele Axo' or Ser' yields to an altered amino acid sequence of the encoded protein. Preferably the isolated peptide comprises or consists of an amino acid sequence of:
Axo' His63→Tyr which has the amino acid sequence SEQ ID No. 5
Axo' Gly68→Val which has the amino acid sequence SEQ ID No. 6
Axo' Ile260→Thr which has the amino acid sequence SEQ ID No. 7
Axo' Asp410→Glu which has the amino acid sequence SEQ ID No. 8
Axo' Phe610→Leu which has the amino acid sequence SEQ ID No. 9
Axo' Arg658→Lys which has the amino acid sequence SEQ ID No. 10
Axo' Glu878→Lys which has the amino acid sequence SEQ ID No. 11
Axo' Thr1309→fs which has the amino acid sequence SEQ ID No. 12
Axo' Thr1309→fs which has the amino acid sequence SEQ ID No. 13
Axo' Leu1310→Met which has the amino acid sequence SEQ ID No. 14
Axo' Ala1384→Thr which has the amino acid sequence SEQ ID No. 15
Axo' Val1668→Met which has the amino acid sequence SEQ ID No. 16
Axo' Asp1855→Asn which has the amino acid sequence SEQ ID No. 17
Axo' Asn1860→Asp which has the amino acid sequence SEQ ID No. 18
Axo' Asn1860→Asp which has the amino acid sequence SEQ ID No. 19
Axo' Val1899→IIe which has the amino acid sequence SEQ ID No. 20
Ser' Ala66→Thr which has the amino acid sequence SEQ ID No. 21
Ser' Leu1090→Phe which has the amino acid sequence SEQ ID No. 22
Ser' Tyr1099_Ala1100→del which has the amino acid sequence SEQ ID No. 23
Ser' Ala1100→Thr which has the amino acid sequence SEQ ID No. 24
Ser' Ala1100→Val which has the amino acid sequence SEQ ID No. 25
Ser' Pro1176→Leu which has the amino acid sequence SEQ ID No. 26

Further, antibodies are disclosed which bind specifically to an isolated peptide. In a preferred embodiment, the antibodies bind specifically to an isolated peptide but not to a peptide encoded by a nucleic acid sequence with SEQ ID No. 1 or 2. The antibodies may be used as detection means in a method of the invention in order to determine presence of an Axo' and/or Ser' allele in the genome of a honey bee or a group of honey bees.

In a further aspect, it is disclosed a kit for the identification or selection of honey bees with improved tolerance to brood diseases. The kit comprises an isolated nucleic acid, an isolated peptide of the invention and/or an antibody together with a container and/or a user manual.

It has surprisingly been found that the presence of an Axo' and/or a Ser' allele in the genome of a honey bee is predictive for the expression of the trait "hygienic behaviour" of said honey bee or its relatives. Since the trait "hygienic behaviour" is predictive for the tolerance of a bee colony to brood diseases, the present invention also refers to the use of a method of the invention for identification or selection of honey bees with improved tolerance to brood diseases. Basically, the method of the invention is suitable for use in identification and/or selection of honey bees with improved tolerance to any brood disease. However, preferably the method of the invention is preferably used in connection with the brood diseases varroatosis (infestation with Varroa destructor), American foulbrood (infestation with Paenibacillus larvae) and/or chalkbrood (infestation with Ascophera apis), preferably with varroatosis.

### FIGURES:

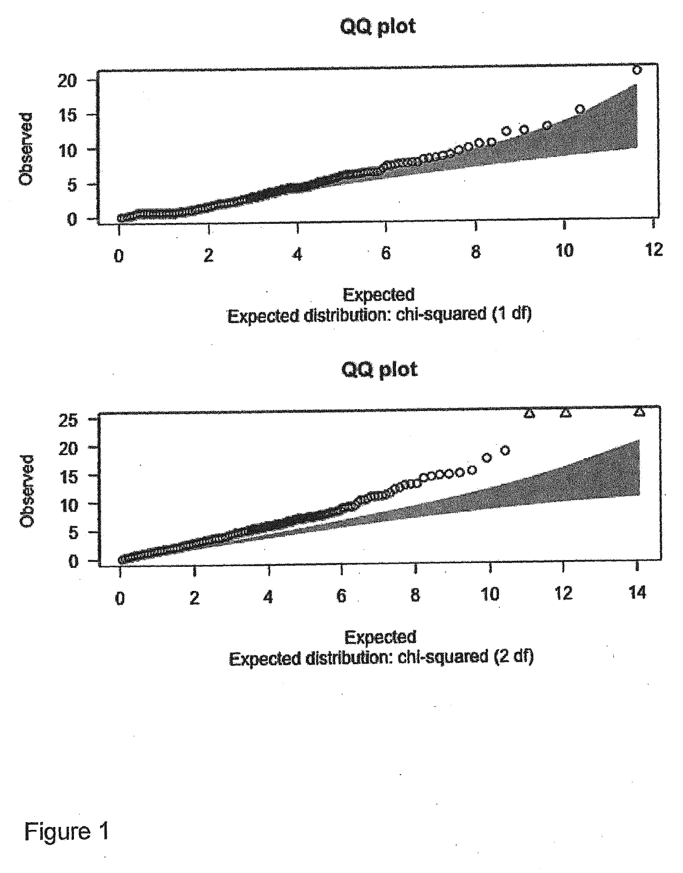

- Figure 1:: shows quantile-quantile plots of allele- and genome-level associations

### EXAMPLES:

In this study, an association study is described for hygienic behavior trait 'uncapping of Varroa infested brood' (also called "defense behavior") employing an Affymetrix 44K SNP array unique for the honey bee (Spotter A, Gupta P, Nürnberg G, Reinsch N, Bienefeld K. (2012) Development of a 44K SNP assay focussing on the analysis of a varroa-specific defence behaviour in honey bees (Apis mellifera carnica). Molecular Ecology Resources, 12, 323-332). Information concerning the SNPs used for this array were deposited in the Dryad repository (DRYAD entry doi:10.5061/dryad.8635cs4h). The array is obtainable through AROS Applied Biotechnology AS, Aarhus, Denmark.

### Materials and Methods

### Defense behavior bioassays and phenotyping

In order to phenotype single worker bees for defense behavior against *Varroa destructor a* special bioassay was developed in the Institute for Bee Research Hohen Neuendorf, Germany (LIB). For one run of the defense behavior bioassay 2000 worker bees were marked individually after hatching and transferred to one caged experimental comb (Bienefeld et al. 2001). These combs were derived from unrelated and undiseased virtually *Varroa*-free colonies. The observation area of each of these combs consisted of ca. 170 brood cells (ca. 10 x 10 cm). Of these cells 45 were artificially infested with one *Varroa* mite by cutting a slot in the cap, inserting the mite with the help of a small paint brush, and resealing the cell. Between five and nine cells were empty, between 73 and 77 cells were left untreated, and 45 cells were opened and resealed without inserting a mite (controls for the effect of cap manipulation).

The caged experimental combs were integrated into the hive of a colony in order to provide the experimental bees with sufficient warmth and an environment as natural as possible in these experiments which were carried out in 11 runs over the years 2009 (5 runs), and 2010 (6 runs). In front of the comb an infra-red sensitive camera (Panasonic WV-NP1004 megapixel color network IP) was installed and the actions on the observation area of the comb were recorded for seven days. Infra-red LEDs (OSA Opto-Light GmbH, Germany, Type: OIS 330 880) were used for illumination rather than visible light, because bees do not respond to this part of the light spectrum (880 ± 10 nm) and are not disturbed during long periods of observation. The recordings were inspected by two independent persons and scanned for hygienic behavior. Bees that started to open a cell were designated as "beginner" and bees which expanded an existing hole were designated as "helper".

### Animals

The honey bees (*Apis mellifera carnica*) used in this experiment were obtained from a special mating design: 10 queens of a line bred for hygienic behavior in the LIB were mated to drones of a non-hygienic line. 10 F1 queens of these crosses were artificially inseminated with pooled sperm from ca. 250 drones from various origins which were unrelated or marginally related among each other and with the queens. The resulting worker-offspring of these queens was analyzed for hygienic behavior against *Varroa destructor* in the years 2009, and 2010 using the defense behavior bioassay described above. To compensate for age effects on 'uncapping of Varroa infested brood' freshly emerged worker bees (0-12h old) were individually labeled and used for the defense behavior bioassays at the age of four days. The first four days the bees of the same run were allowed to get used to each other. They were kept together in a caged comb which was integrated into a colony in order to provide warmth and a natural environment. After this time the bees show a calm and normal behavior when inspected with the help of an infrared camera.

Colonies used as sources for experimental animals belonged to a line selected for *Varroa* tolerance since 1997 in the LIB. Whatsoever, success of these conventional breeding efforts, based on phenotypic records alone, was limited so far.

In 11 runs of the defense behavior bioassay a total of 22,000 worker bees were phenotyped. From this population, the 122 top performing beginners were selected for genotyping. These bees were the best 122 regarding the number of their uncapping actions directed against *Varroa* infested cells. They started at least one uncapping of a *Varroa* infested cell and helped in at least one other uncapping event. Furthermore, the number of their uncapping and helping actions directed against *Varroa* infested cells were at least twice as high as their actions directed against control cells.

For the control sample, for every bee from the case sample, a worker was selected that descended from the same queen, but never showed hygienic behavior. This design was chosen in order to counterbalance differences between case and control sample, which are only due to population stratification. Consequently, by a comparison of these samples, only differences in hygienic behavior but not in other traits should be identified.

### Statistical analysis

SNP genotyping was performed with an Affymetrix 44K SNP array, developed in cooperation with AROS Applied Biotechnology AS. The algorithm BRLMM-P (http://media.affymetrix.com/support/technical/whitepapers/brlmmp_whitepaper.pdf) was used for genotype calling.

The SNP-array contains 32632 SNPs which yielded non-distorted and thus reliable genotyping signals. From this marker-set, SNPs were chosen for the statistical analysis. The statistical analyses of the SNP-array genotyping data of the 244 honey bee DNAs were performed using R statistical software (http://www.r-project.org/). Marker inclusion criteria for the association study were: 1) An Affymetrix Genechip call rate of more than 95% across the 244 samples at confidence p-values below 0.1, 2) A minor allele frequency (MAF) of greater than 0, and 3) No departure from Hardy-Weinberg Equilibrium (HWE, Fishers Exact test P-value < 0.05) in the control group. Genotype and allele-level associations were tested using Fishers Exact test, and the resulting P-values were subjected to FDR correction.

In order to assess the normal distribution of the data, Quantile-quantile (Q-Q) plots were constructed by ranking a set of values of their association statistic (Chi-square) from smallest to largest (the 'order statistics') and plotting them against their expected values using the program "snpMatrix" from the R package. If the two distributions being compared are similar, the points in the Q-Q plot will approximately lie on the diagonal. Plots were generated for association values on allele- and genotype level.

In order to estimate the fraction of heritability explained by significantly associated SNPs compared to that of all SNPs tested, we used the GCTA software package.

### Identification of positional and functional candidate genes for varroa tolerance

For SNPs showing an effect on varroa tolerance we carried out BLAST searches (http://blast.ncbi.nlm.nih.gov/Blast.cgi?PAGE_TYPE=BlastSearch&PROG_DEF=blastn&BLA ST_PROG_DEF=megaBlast&SHOW_DEFAULTS=on&BLAST_SPEC=OGP_7460_9555) with the SNP flanking sequences as queries in order to determine their position on the NCBI Map Viewer of the honey bee genome (http://www.ncbi.nlm.nih.gov/projects/mapview/map_search.cgi?taxid=7460). The genomic regions around these SNPs will be scanned for putative candidate genes.

### Mutation analysis of candidate genes for Varroa tolerance in honey bees

Functional candidate genes in the vicinity (up to 100 kbp distance) of significantly trait associated SNPs were subjected to a mutation analysis, i.e. their exons were sequenced and scanned for protein changing polymorphisms. Such changes serve as evidence for a function of the respective gene in the regulation of Varroa tolerance. The mutation analysis was carried out employing two samples of 15 worker bees with extremely differing Varroa tolerance. One sample showed a very high degree of hygienic behavior against Varroa and the other a very low degree. Genotype and allele frequencies were tested for significant differences between the two samples, which should provide additional indication for the involvement of genes in the control of the investigated trait.

### Results

### Statistical analysis and Identification of positional and functional candidate genes for varroa tolerance

A total of 1561 SNPs met all of the filtering criteria described above. These SNPs were included in the association tests. After FDR correction two SNPs showed a significant genome-wide association with the trait "uncapping of Varroa infested brood cells" on genotype-level. These SNPs accounted for 8% of the observed phenotypic variance in hygienic behavior in our population. Further information about these SNPs is provided in Table 1. Information about all SNPs used for this assay were deposited in the Dryad repository (DRYAD entry doi:10.5061/dryad.8635cs4h). No significant effects on allele-level were found.

**Table 1: SNPs showing a significant genome-wide association with the trait "uncapping of Varroa infested brood cells"**

| SNP | Nucleotide change | Chromosome | Position (bp) | Minor allele frequency | FDR corrected allele level association | FDR corrected genotype level association |
|---|---|---|---|---|---|---|
| AMB-00484462 | A→G | 8 | 4303286 | 0,1894 | 0,2017 | 0,03565 |
| AMB-00805602 | T→C | 5 | 7541012 | 0,3151 | 0,7165 | 0,03565 |

Data points in Quantile-quantile plots (Figure 1) of allele- (1 df) and genotype-level (2 df) associations approximately lie on a line, which is indicative of the similarity of the two distributions being compared (observed and expected) and thus demonstrate the quality of our data. Departures in the extreme tail of the distribution in the genotype-level plot are due to markers associated with the trait.

Two candidate genes, spatially associated with the two significant SNPs have been selected based on functional evidence. Axotactin (Axo, chromosome 8; SNP: AMB-00484462 in intron 15 of Axo) and Serrate (Ser, chromosome 5; SNP: AMB-00805602 ca. 600 kb distance to Ser). The detailed reasons for their selection will be discussed later.

### Mutation analysis - Identification of possibly causative polymorphisms in candidate genes for Varroa tolerance

For the candidate gene Axo 24 polymorphisms were found. Of these, 16 are protein changing (16 substitutions), e.g. Axo'3867 T→C and Axo'3909 T→C showed a particularly significant effect of genotype, whereas Axo'10755 C→T showed a particularly significant effect of allele on Varroa tolerance in the analyzed sample. For the candidate gene Ser 7 polymorphisms were found. Of these, 6 are protein changing (6 substitutions) and e.g. Ser'40832 T→C showed a particularly significant effect of genotype on Varroa tolerance in the analyzed sample.

The identified significant effects are highly interesting, because they confirm the results of the association study of the SNP-Chip genotypes. So, our results are supported by evidence from two different sources, which makes an actual involvement of Axotactin and Serrate in the regulation of Varroa tolerance highly likely. Moreover, these experimental results are backed by information about the gene function of Axotactin and Serrate in other insect species, which is outlined in the discussion.

### Discussion

Hygienic behavior is an important and efficient factor in the fight against *Varroa,* since it interrupts the reproductive cycle of the mite. Its initiation depends on the olfactory sensitivity of a bee, and odour profile along with stimulus intensity of abnormal brood. Results of Schöning et al. (Schöning C, Gisder S, Geiselhardt S, Kretschmann I, Bienefeld K, Hilker M, Genersch E (2012). Evidence for damage-dependent hygienic behaviour towards Varroa destructor-parasitised brood in the Western honey bee, Apis mellifera. Journal of Experimental Biology, 215, 264-271.) suggest that bees show selective, damage-dependent hygienic behavior against *Varroa destructor.* Consequently, efforts to breed for increased hygienic behavior presumably result in bees with an increased olfactory sensitivity. This would not only be useful as a mechanism of disease resistance against *Varroa* but also against other brood diseases like American Foulbrood and chalkbrood because the respective pathogens damage bee brood even more obvious and thus should pose a stronger stimulus to the bees than *Varroa.* This renders the molecular genetic elucidation of hygienic behavior a prior-ranking aim of honey bee breeding. Our research makes a significant contribution to the achievement of this aim.

Candidate genes in the vicinity of significantly trait-associated SNPs were chosen mainly based on functional evidence. The Axo gene encodes a protein which is a member of the neurexin superfamily. It appears to be a key element that mediates the effects of glial cells on neuronal membrane excitability and synaptic plasticity. Possibly, Axo affects the sensitivity of bees to external stimuli produced during Varroa infestation.

Ser is a member of the Notch signaling pathway. This pathway is involved in the regulation of genes that control multiple cell differentiation processes during embryonic and adult live, such as neuronal function and development. Evidence for an involvement of the Notch pathway in the control of varroa tolerance of honey bees is already provided in the literature. It has been found that the gene fringe, a positive regulator of the Notch signaling pathway, appears to be differentially expressed in bees tolerant and sensitive to *Varroa.* Ser is also involved in antennal development in the flour beetle, Tribolium castaneum. It could have a similar function in honey bees. Antennae are their main organs for the reception of olfactory stimuli, which are assumed to trigger varroa defense behavior.

## Claims

1. Method for identification of honey bees with above-average hygienic behaviour, **characterized in that**,
it is determined whether the genome of a honey bee or a group of honey bees comprises:
- at least one allele Axo' of the Axo gene, wherein said at least one allele Axo' is **characterized by** presence of at least one sequence variation compared to the nucleotide sequence of the Axo gene with SEQ ID No. 1, wherein the at least one sequence variation of the allele Axo' is one of Axo'883 C→T, Axo'899 G→T, Axo'1412_→G, Axo'1424_→C, Axo'1435_→C, Axo'1435_→CGAA, Axo'2537 T→C, Axo'3759 C→A, Axo'3867 T→C, Axo'3909 T→C, Axo'4666 T→C, Axo'5299 G→A, Axo'6139 G→A, Axo'7717 _→A, Axo'8566 G→_, Axo'8566 GT→_A, Axo'8567 T→A, Axo'8851 G→A, Axo'10755 C→T, Axo'11325 G→A, Axo'12909 G→A, Axo'12924 A→G, Axo,'12924_12926 AAT→GAC, Axo'13984 G→A and/or combinations thereof;
and/or
- at least one allele Ser' of the Ser gene, wherein said at least one allele Ser' is **characterized by** presence of at least one sequence variation compared to the nucleotide sequence of the Ser gene with SEQ ID No. 2, wherein the at least one sequence variation of the allele Ser' is one of Ser'17193 G→A, Ser'40832 T→C, Ser'43680 C→T, Seer'43709_43711 CGC→TGT, Ser'43710 G→A, Ser'43711 C→T, Ser'43939 C→T and/or combinations thereof.

2. Method of claim 1, comprising the steps:
- isolation of a biological probe of one or more honey bee individuals;
- determining whether the genome of a honey bee or a group of honey bees comprises:
at least one allele Axo' of the Axo gene, wherein said at least one allele Axo' is **characterized by** the presence of at least one sequence variation compared to the nucleotide sequence of the Axo gene with SEQ ID No. 1, wherein the at least one sequence variation of the allele Axo' is one of Axo'883 C→T, Axo'899 G→T, Axo'1412_→G, Axo'1424_→C, Axo'1435_→C, Axo'1435_→CGAA, Axo'2537 T→C, Axo'3759 C→A, Axo'3867 T→C, Axo'3909 T→C, Axo'4666 T→C, Axo'5299 G→A, Axo'6139 G→A, Axo'7717 _→A, Axo'8566 G→_, Axo'8566 GT→_A, Axo'8567 T→A, Axo'8851 G→A, Axo'10755 C→T, Axo'11325 G→A, Axo'12909 G→A, Axo'12924 A→G, Axo,'12924_12926 AAT→GAC, Axo'13984 G→A and/or combinations thereof;
and/or
at least one allele Ser' of the Ser gene, wherein said at least one allele Ser' is **characterized by** the presence of at least one sequence variation compared to the nucleotide sequence of the Ser gene with SEQ ID No. 2, wherein the at least one sequence variation of the allele Ser' is one of Ser'17193 G→A, Ser'40832 T→C, Ser'43680 C→T, Ser'43709_43711 CGC→TGT, Ser'43710 G→A, Ser'43711 C→T, Ser'43939 C→T and/or combinations thereof; and
- selecting one or more honey bee individuals, wherein its genome comprises at least one allele of Axo' and/or of Ser'.

3. Method of one of the preceding claims, wherein the at least one allele Axo' contains more than one sequence variation compared to the nucleotide sequence of the Axo gene with SEQ ID No. 1.

4. Method of one of the preceding claims, wherein the at least one allele Ser' contains more than one sequence variation compared to the nucleotide sequence of the Ser gene with SEQ ID No. 2.

5. Method of one of the preceding claims, wherein the presence of the at least one allele Axo' and/or the at least one allele of Ser' is determined by a nucleic acid-based or an amino acid-based assay.

6. Method of one of the preceding claims, wherein the presence of the at least one allele Axo' and/or the at least one allele of Ser' is determined by PCR-, hybridisation- and/or sequencing-based methods or by heteroduplex-, SSCP- or RFLP-analysis.

7. Use of a method of one of claim 1 to 6 for identification or selection of honey bees with improved tolerance to brood diseases.

8. Use of claim 7, wherein the brood diseases are varroatosis (infestation with Varroa destructor), American foulbrood (infestation with Paenibacillus larvae) and/or chalkbrood (infestation with Ascophera apis), preferably varroatosis.

## Patentansprüche

1. Verfahren zur Identifizierung von Honigbienen mit überdurchschnittlichem hygienischem Verhalten, **dadurch gekennzeichnet, dass**
bestimmt wird, ob das Genom einer Honigbiene oder einer Gruppe von Honigbienen umfasst:
- wenigstens ein Allel Axo' des Axo-Gens, wobei das wenigstens eine Allel Axo', verglichen mit der Nukleotidsequenz des Axo-Gens mit der SEQ ID Nr. 1, durch das Vorhandensein wenigstens einer Sequenzvariation gekennzeichnet ist, wobei die wenigstens eine Sequenzvariation des Allels Axo' eine von Axo'883 C→T,
Axo'899 G→T, Axo'1412_→G, Axo'1424_→C, Axo'1435_→C,
Axo'1435_→CGAA, Axo'2537 T→C, Axo'3759 C→A, Axo'3867 T→C,
Axo'3909 T→C, Axo'4666 T→C, Axo'5299 G→A, Axo'6139 G→A, Axo'7717_→A,
Axo'8566 G→_, Axo'8566 GT→_A, Axo'8567 T→A, Axo'8851 G→A,
Axo'10755 C→T, Axo'11325 G→A, Axo'12909 G→A, Axo'12924 A→G,
Axo'12924_12926 AAT→GAC, Axo'13984 G→A und/oder Kombinationen derselben ist;
und/oder
- wenigstens ein Allel Ser' des Ser-Gens, wobei das wenigstens eine Allel Ser', verglichen mit der Nukleotidsequenz des Ser-Gens mit der SEQ ID Nr. 2, durch das Vorhandensein wenigstens einer Sequenzvariation gekennzeichnet ist, wobei die wenigstens eine Sequenzvariation des Allels Ser' eine von Ser'17193 G→A, Ser'40832 T→C, Ser'43680 C→T, Ser'43709_43711 CGC→TGT, Ser'43710 G→A, Ser'43711 C→T, Ser'43939 C→T und/oder Kombinationen derselben ist.

2. Verfahren nach Anspruch 1, das folgende Schritte umfasst:
- Isolation einer biologischen Probe eines Honigbienenindividuums oder mehrerer Honigbienenindividuen;
- Bestimmen, ob das Genom einer Honigbiene oder einer Gruppe von Honigbienen umfasst:
wenigstens ein Allel Axo' des Axo-Gens, wobei das wenigstens eine Allel Axo', verglichen mit der Nukleotidsequenz des Axo-Gens mit der SEQ ID Nr. 1, durch das Vorhandensein wenigstens einer Sequenzvariation gekennzeichnet ist, wobei die wenigstens eine Sequenzvariation des Allels Axo' eine von Axo'883 C→T,
Axo'899 G→T, Axo'1412_→G, Axo'1424_→C, Axo'1435_→C,
Axo'1435_→CGAA, Axo'2537 T→C, Axo'3759 C→A, Axo'3867 T→C,
Axo'3909 T→C, Axo'4666 T→C, Axo'5299 G→A, Axo'6139 G→A, Axo'7717_→A,
Axo'8566 G→_, Axo'8566 GT→_A, Axo'8567 T→A, Axo'8851 G→A,
Axo'10755 C→T, Axo'11325 G→A, Axo'12909 G→A, Axo'12924 A→G,
Axo'12924_12926 AAT→GAC, Axo'13984 G→A und/oder Kombinationen derselben ist;
und/oder
wenigstens ein Allel Ser' des Ser-Gens, wobei das wenigstens eine Allel Ser', verglichen mit der Nukleotidsequenz des Ser-Gens mit der SEQ ID Nr. 2, durch das Vorhandensein wenigstens einer Sequenzvariation gekennzeichnet ist, wobei die wenigstens eine Sequenzvariation des Allels Ser' eine von Ser'17193 G→A, Ser'40832 T→C, Ser'43680 C→T, Ser'43709_43711 CGC→TGT, Ser'43710 G→A, Ser'43711 C→T, Ser'43939 C→T und/oder Kombinationen derselben ist; und
- Auswählen eines Honigbienenindividuums oder mehrerer Honigbienenindividuen, wobei sein/ihr Genom wenigstens ein Allel von Axo' und/oder von Ser' umfasst.

3. Verfahren nach einem der vorangehenden Ansprüche, wobei das wenigstens eine Allel Axo', verglichen mit der Nukleotidsequenz des Axo-Gens mit der SEQ ID Nr. 1, mehr als eine Sequenzvariation enthält.

4. Verfahren nach einem der vorangehenden Ansprüche, wobei das wenigstens eine Allel Ser', verglichen mit der Nukleotidsequenz des Ser-Gens mit der SEQ ID Nr. 2, mehr als eine Sequenzvariation enthält.

5. Verfahren nach einem der vorangehenden Ansprüche, wobei das Vorhandensein des wenigstens einen Allels Axo' und/oder des wenigstens einen Allels von Ser' durch einen nukleinsäurebasierten oder einen aminosäurebasierten Assay bestimmt wird.

6. Verfahren nach einem der vorangehenden Ansprüche, wobei das Vorhandensein des wenigstens einen Allels Axo' und/oder des wenigstens einen Allels von Ser' durch PCR-, hybridisierungs- und/oder sequenzierungsbasierte Verfahren oder durch Heteroduplex-, SSCP- oder RFLP-Analyse bestimmt wird.

7. Verwendung eines Verfahrens nach einem von Anspruch 1 bis 6 zur Identifizierung oder Auswahl von Honigbienen mit verbesserter Widerstandsfähigkeit gegenüber Brutkrankheiten.

8. Verwendung nach Anspruch 7, wobei die Brutkrankheiten Varroose (Befall mit Varroa destructor), amerikanische Faulbrut (Befall mit Paenibacillus larvae) und/oder Kalkbrut (Befall mit Ascosphaera apis), vorzugsweise Varroose, ist.

## Revendications

1. Procédé d'identification d'abeilles mellifères présentant un comportement hygiénique supérieur à la moyenne, **caractérisé**
**en ce qu'**il est déterminé si le génome d'une abeille mellifère ou d'un groupe d'abeilles mellifères comprend :
- au moins un allèle Axo' du gène Axo, ledit au moins un allèle Axo' étant **caractérisé par** la présence d'au moins une variation de séquence par rapport à la séquence nucléotidique du gène Axo avec la SEQ ID N° 1, ladite au moins une variation de séquence de l'allèle Axo' étant une variation parmi Axo'883 C→T, Axo'899 G→T, Axo'1412→G, Axo'1424_→C, Axo'1435_→C, Axo'1435_→CGAA, Axo'2537 T→C, Axo'3759 C→A, Axo'3867 T→C, Axo'3909 T→C, Axo'4666 T→C, Axo'5299 G→A, Axo'6139 G→A, Axo'7717_→A, Axo'8566 G→_, Axo'8566 GT→_A, Axo'8567 T→A, Axo'8851 G→A, Axo'10755 C→T, Axo'11325 G→A, Axo'12909 G→A, Axo'12924 A→G, Axo,'12924_12926 AAT→GAC, Axo'13984 G→A et/ou des combinaisons de celles-ci ;
et/ou
- au moins un allèle Ser' du gène Ser, ledit au moins un allèle Ser' étant **caractérisé par** la présence d'au moins une variation de séquence par rapport à la séquence nucléotidique du gène Ser avec la SEQ ID N° 2, ladite au moins une variation de séquence de l'allèle Ser' étant une variation parmi Ser'17193 G→A, Ser'40832 T->C, Ser'43680 C→T, Set'43709_43711 CGC→TGT, Ser'43710 G→A, Ser'43711 C→T, Ser'43939 C→T et/ou des combinaisons de celles-ci.

2. Procédé selon la revendication 1, comprenant les étapes suivantes :
- isolation d'un échantillon biologique d'un ou de plusieurs individus de l'espèce abeille mellifère ;
- détermination si le génome d'une abeille mellifère ou d'un groupe d'abeilles mellifères comprend :
au moins un allèle Axo' du gène Axo, ledit au moins un allèle Axo' étant **caractérisé par** la présence d'au moins one variation de séquence par rapport à la séquence nucléotidique du gène Axo avec la SEQ ID N° 1, ladite au moins une variation de séquence de l'allèle Axo' étant une variation parmi Axo'883 C→T, Axo'899 G→T, Axo'1412_→G, Axo'1424_→C, Axo'1435_→C, Axo'1435_→CGAA, Axo'2537 T→C, Axo'3759 C→A, Axo'3867 T-C, Axo'3909 T→C, Axo'4666 T→C, Axo'5299 G→A, Axo'6139 G-A, Axo'7717_→A, Axo'8566 G→_, Axo'8566 GT→_A, Axo'8567 T→A, Axo'8851 G→A, Axo'10755 C→T, Axo'11325 G→A, Axo'12909 G→A, Axo'12924 A→G, Axo'12924_12926 AAT→GAC, Axo'13984 G→A et/ou des combinaisons de celles-ci ;
et/ou
au moins un allèle Ser' du gène Ser, ledit au moins un allèle Ser' étant **caractérisé par** la présence d'au moins one variation de séquence par rapport à la séquence nucléotidique du gène Ser avec la SEQ ID N° 2, ladite au moins une variation de séquence de l'allèle Ser' étant une variation parmi Ser'17193 G→A, Ser'40832 T→C, Ser'43680 C→T, Ser'43709_43711 CGC→TGT, Ser'43710 G→A, Ser'43711 C→T, Ser'43939 C→T et/ou des combinaisons de celles-ci ; et
- sélection d'un ou de plusieurs individus de l'espèce abeille mellifère dont le génome comprend au moins un allèle Axo' et/ou Ser'.

3. Procédé selon l'une des revendications précédentes, où ledit au moins un allèle Axo' contient plus d'une variation de séquence par rapport à la séquence nucléotidique du gène Axo avec la SEQ ID N° 1.

4. Procédé selon l'une des revendications précédentes, où ledit au moins un allèle Ser' contient plus d'une variation de séquence par rapport à la séquence nucléotidique du gène Ser avec la SEQ ID N° 2.

5. Procédé selon l'une des revendications précédentes, où la présence dudit au moins un allèle Axo' et/ou dudit au moins un allèle Ser' est déterminée par un test à base d'acide nucléique ou un test à base d'acide aminé.

6. Procédé selon l'une des revendications précédentes, où la présence dudit au moins un allèle Axo' et/ou dudit au moins un allèle Ser' est déterminée par des procédés basés sur PCR, hybridation et/ou séquençage ou par analyse d'hétéroduplex, de SSCP ou RFLP.

7. Utilisation d'un procédé selon l'une des revendications 1 à 6 pour l'identification ou la sélection d'abeilles mellifères avec une tolérance améliorée aux maladies de couvain.

8. Utilisation d'un procédé selon la revendication 7, où les maladies de couvain sont la varroose (infestation par Varroa destructor), la loque américaine (infestation par Paenibacillus larvae) et/ou le couvain plâtré (infestation par Ascophera apis), de préférence la varroose.


### SEQUENCE LISTING

<110> Länderinstitut fur Bienenkunde Hohen Neuendorf e.V.
<120> Method for identification of honey bees [Apis mellifera] with improved tolerance to brood diseases
<130> P09948EP
<160> 26
<170> PatentIn version 3.5
<210> 1
   <211> 14501
   <212> DNA
   <213> Apis mellifera
<400> 1
   ttggtgcatc aagtgtgaat agaagagtgc aatttgcgtc gatggaattt gagattcttc      60
   ttcagttaga gacttgggat ttttttcaaa agaaaactgg tgtcgtgatt taaccttttg    120
   ataaaattac gaggaattcg gaaggacgga agttgaaaag gacgattatc ctttcatctt     180
   ggaagagttt tgagaagtca gagttttcgt agttcgatag atttaagcaa tttcagtatt     240
   gataacattt aacagtttct caagtttgtg gaactatcct tgccggttta aaaaaccaat     300
   ctttcttggg atttttgaga attagctgtt tgtcaaaaat tgtgaaaaga ttaaaaactt     360
   gattatctat cgatattctc cgtcgatatt tatctcttta ctttcttaca tctttcgaat     420
   aattttgacg atacgatgaa cgtaaaataa cgccacgttc tctttattta aaaagaaaga     480
   aagaaaaaaa aaatctccga gaatagtagc cgaaaattcg ggaaggaaat ttgagaacga     540
   aacatcagag gaaacgttgg ttcatttttc agcaaggaaa caaggatgat cgaccagaaa     600
   agatggatct tgaggatcct cgttttggca aacgttcacg ccgcgttcgc ctcgatcaac     660
   ctaacagatt catcggagat cgatcaatcg aacgagatgg actcgttcac aaccacgaag     720
   aggacgtttc ttgacaggtg tctggtgaag accgagccag gcccgtgcaa acagtacgtc     780
   accaggtgga cgttcatcaa gactgagggg aaatgcaaga ctttctctta cggcggctgc     840
   ttagggaacg agaatcgttt caactcggaa gccgagtgtc tacattattg cgtgggtggc     900
   cctgaacgta aggagaatca aataaatttt gtcactcgtt caattttcgt ttcattatcg     960
   aattaacata cgaagagaac atcgcgagaa aatggtggaa aatcgagata ttagatcatg    1020
   atatttcttt tgattcgatt tccgttgaac gacgacaaga taatctctat tagatttttg   1080
   aaagattctt tgtaaaaata tcgtttctaa gatgaaaatt taataaaaaa aaaaaaaaag    1140
   aaagagaaat attttgtata ttttttattt tattttttta atcttgccaa tatctttatc    1200
   ttgttttcgg attttcttag acacactgcc tcatatggtc acgaacggga acgtacttgt    1260
   aacgagcaca gcaaccacga acacactgcc ttctaccacc gccatcactt cacggccaac    1320
   gttttcgcca ccagaaccga cgaaaccgcc tgtaccaaag tacaaacgag gaaaggtaaa 1380
   atttatttcc tatgaattat ttcctctctc cttaacttaa tgtatttcaa atgtcgagta 1440
   agaaaaattt atgagatacg agagttcacc ccagaaaaat tgatcgacag gagctcacct 1500
   tcatggaatc tggctacgag aaaactttta tgttcgccca aagcaacacg tttatacaac 1560
   tcgacggtag cggtataaaa ccgtttcaac ttcggtaagt aatcttgact ttacgcgaaa 1620
   ggattatttc gaaacgaggg aacgagagat atcaatctgt caaaagaaaa gacgatgaaa 1680
   atggggtagg tttttttttc gcttcaacat tgaaaaatgt taacgtattc aaaattcgat 1740
   cctcgttaaa acgaggataa aatttgaaat taaaaaaaga aaagagaaaa tatcaaattt 1800
   gacgaatgaa aagggataaa ggaatgataa taatataatt ggaatacgaa gatttttcaa 1860
   atcattcttc caagaataaa aaagaataga ttaatgattc taatttccca aaatagatat 1920
   tttctataga attattccta ttattttcaa cacatattta tttattgtat tttatagaaa 1980
   gatcaattat tttactattt aatcactgac attcttcgtt aataaatatc ttatcaattt 2040
   tcaaataact tttcttcctt aaataataat gattccagat atgatcaagt caaatctaaa 2100
   accgaatttc ccctcccctt ttccaaataa ttcaaaatat cctaaattaa atccgaacat 2160
   tcattccttc gagcttaata ataaattgaa gaagaatcat ggattgttat aatcatttca 2220
   atttgcagac tgtgtcgcga gatatcgttc aaatttcgca cgaaattacc ccacggtttg 2280
   ctggtgtatc acagcgtgaa ggatcggcca gagatccggt tagacccgta tgctctttac 2340
   gtgatcgtgg agaaagggca gctgaaagtg gtccacgtgt tcgggaaacg atcaacaagc 2400
   ctgacggtag gcgaggggtt gaacagggat gaatggcaca gcgttttggt gaggatcgac 2460
   gtccacgggg cgaaattgat cgccagggtg gacgataaac aagaagagac cacgcttgaa 2520
   gtcttggaac acgtgatcaa ttatggagtg tccgaggaac tggcgtctgt cgtccttatt 2580
   ggtggtaaaa tatacaacga catatcttca agttttttaa gatgaataga attttaaaat 2640
   ttcgaaagga gaatataatg cccccctttt cgatgaatat taaaatcctt gaaattcttc 2700
   catgaattga tacattatcg aatcatattt tttgaatttt caaaatatca atcgagttat 2760
   aatttctatt cttctattta tcacaaagat attaagcaaa atataataag gatctggttg 2820
   actggattat cgacgaagat aaaatgatgg aattattttc cttgttattt aataataaat 2880
   tatttttagg gttgagttcc gaagaaagat tgcacggcgt gaaatacata atagaatcat 2940
   tcgttggctg tatacgagat atggttctca gctcgggcaa atcggccagc gatttgctgc 3000
   ccattcaacc tctgattgcg acaaaacacg aaaacgtgag agagggttgc atagacaagt 3060
   aagcagataa tcttttaata attaaagagc gttatgttat tttataatat tacttgtttt 3120
   cattgatcgt acgatgaaat ttcagatgta gaacacgaga aaatctctgt ttcatctcga 3180
   gtcaatgcgt gaatcactat aatagtttaa cttgcgattg ctttggaacg aagtatgaag 3240
   gagaacgatg cgatgtatat agtacgtaat catttcaata caacaatatt tttcttctaa 3300
   taaaaaggga cacacgtttc attccgtatg aaaattaatt gggacaattg gattgaaaga 3360
   aaacttggaa ttctataaaa aaaaaacttt aacgatttta aaattaatgt agacaattac 3420
   tttccttaga atatatctgt aacatcgata taatgattca aaattatatc caatttctta 3480
   tatattttca tatatataag aattcctgtc caatcaattt tcaaaatata ataaaaagat 3540
   taaacgttcg aatgcattcc agcggccacg atcttaacgt taagaggttc ctcgtacgta 3600
   tctttccggg tttacgactg gaaggacaga gttcactcgt ccgtcaacag gataagcctt 3660
   gcgttcaagg tatttgataa atcgatcgat tacgaatcat attctcgccc gtgaacgatg 3720
   aaattaattt ttatattcgt tggaaagacg aaatgggacg actcggctct gttctacgct 3780
   tccggggaaa tcgacggtac gccgcactac gtggcagcct ccatagtgaa cggatcggtg 3840
   cacatcgagt tggatttcgg ccacaattcg aagatcgcca ctgtactcgg cgattatatc 3900
   acgtcgaatc attggaacaa tctgacgata ttccacaacg gatcgttggt cttcgtcagc 3960
   ctggacgacg agattaaagt gctcgagata cccggtgaaa attataatat gatcatcgat 4020
   cctgagatct acataggcgg tggcccggaa ttgtacaaga agaagggact ttcgtcgcac 4080
   aataatttcg caggtgagaa taataataaa taataataat aataattaaa attcgatctt 4140
   tccatttgta ttggaatata gataaaaaag tcgttgaatt aattaaagcg tcttttttaa 4200
   agacgttaca attttcttgg cgataaaatt tcatggagat attaacaaat gatagatgcg 4260
   tcgtcgattt aaaagagaat ttctaaagga aacatttcta agggaaacgt gaatctatac 4320
   atttaatcta tacattacct cccctcaccc ccctaaaaca cgacttttta agattgatga 4380
   agatcaattg aatcggtttc aggctcgctg aaatacgttt tcttcaacga caagtccatt 4440
   atttatgagt tgaaacgttc caaccccatg gtgcattata tcggtgtgct cgagcccgaa 4500
   tactacgagg cggatgttga tgtgattcca ataacatatc cgttcgcgac cagccacatt 4560
   tggtggccaa tagcgcggac cgactccctt aaattgaact tcgacttcaa gagttcgaag 4620
   cctgtggccg tggtcgcgtc tggggacgtg aaatgcaatc gtagcttcgg ttactgggag 4680
   gtacgtttca actttgagga aatattggct gctcgaaaat agcggtcgaa cgccgacagt 4740
   aaatttatgt aaatttgaat aaacctgatc gtcgaacaga ataaatcgat atattttcct 4800
   tcgttgggaa gaaacaatca tttttcaatc gattgaaatt atcgaaactg tgttaatcat 4860
   ctttgaataa catacgattc gtagatattt gtgaatcgta atatacaaca agatttctat 4920
   cttttaataa tttgtcgacg aattaaagaa ttaattaaat tctaataaat tcgaaaaact 4980
   tattaattgg acacatatct tcaaatttac gaatttgcat ttaataattt ttcgacgaat 5040
   cgaagaatta acttaaattc taataaattc gaaaaactta ttaattggac acatatcttc 5100
   aaatttacga atttgcgttc aaagttgaac atctttcaaa tccgattatt ccaattttcg 5160
   atgcacagct ccaactggtg aacgacgaaa tccgttttca actgatcccg gtgttgggcg 5220
   aaaacgtcac ggtcacgact gcggtgaagt tcccgcctta caacacgtct tggcacgcgg 5280
   tcgaattaaa ttacacgaga ggggagatca gcgttctggt ggattatagg aacaagcaga 5340
   gcaaattatt ctcgatgacg ttcgagctgg gcgacaaagt tatcatagga agtgggaaga 5400
   acaacacggg caagtttctc tcaaaaatct cgcatgataa taacgcgaca ataatatata 5460
   ttctatatca ggtttggtcg ggtgtatgcg cgagatacgg gtgaacgagg agaggataga 5520
   gcctagatac gtgatgaaca ccgacagagt tatgggtgaa gtagcgttgg acaattgcca 5580
   attcatcgat ccttgcacca ggcccaacac ttgcgagcat ggaggcaaat gctcggtgaa 5640
   aaaggacgag atcagctgcg attgcaccga caccggttac gtagggaaaa attgtcattt 5700
   cagtgagtga attcgatcga gagatgtcat ctcgataagt aattatttac gagtaaattc     5760
   tgggagggga atgaattgag aagaattgaa gaatcgtgat aaattttaac ttaaatttca     5820
   gctaaattca gaaaaacctg cgaggaattg gcattgttag ggtacaccca ggacgatgtg 5880
   tacaaaatcg acatcgacgg taacggtaga tttcctcctg ccctggtgaa atgcgagttc 5940
   caatccatcg agaactcgac gaagaccatc gtggagcaca atttgccctc gcaagtcgac 6000
   gtcagatcga tcgcagaatc tgatttctcg ttcaacatca agtacaggca attcaccgcc 6060
   gagatgcttc aagaattgat ctcgcactcg ttgtattgca gccagtacgt caagtacgat 6120
   tgctacaagg cgccgttgga attgcacagc gccacgtggt tccttaactc gaagggaaca 6180
   accgtggatt acattggaaa tgtgaacaga ggatcttgtc cttgtggaag tgagtaaaat 6240
   cgaagagttt aagtcaaatg atgataattg aaattatcat tccgttttta tatcaagatt 6300
   ttaaaagtat aaatttgcca aaataatatt tagaaatttt atattaaaaa gtgtttaaag 6360
   aaatgatgaa tttggaaaat ataatgatca ataaagattc cagttgatcg ataatcaaat 6420
   tgattagtaa aatcaaggaa ttttgattat atgcacagtc tagaattaaa ttaaattatt 6480
   caaggaattg acaatctgtt tgtgtttagt gaacaagaca tgcattaatc caaatttaag 6540
   ctgcaattgc gatttcaccg gaaacgggga taaatggctt tccgacgagg ggtattacga 6600
   aacaccggat tctttgggta tcacgggaat ggtattcttg caacaaaggg atctcgagga 6660
   agacgcacaa gggcgaatta ccttgggtcc attagagtgc gtcgaaacga gtaaataata 6720
   tttttaccta atactcaaaa tattataatt tatataaata tctagcattg tttaaaaaat 6780
   taaattcctt gcaaacatta ttttatcttt ttaatatctt tcaacttaaa aagtatttaa 6840
   atgaagaaaa tcctaatatt gaaatgaatg attaaagttg attgattttt atacagatac 6900
   acagaagtac gttgtaacat tcaccacttc gcaatcatac atcgaagtac caggttggag 6960
   aaaaggtgat atagcattca gttttcgaac gactggggag aaagctatcc tactctatca 7020
   accgcccatc agaaacaatt atccatcttt catggttgct ttgacctcgg agtatcggtt 7080
   gacgtttaat ttcaccctga gcacgggtgt tataaagggt atagaggtgg agagtaaaag 7140
   aaaattgaac aacggggaat ggcagaagat atggatcgat tataacaatt atcacgtcag 7200
   attaatgatc aacaccgatt ctcaaatgct cgacctcgaa gacaagtttg gaccgttcga 7260
   gggctctatg tttatcggtg gagccactgc gtgagtatta ttctcaaaaa tacgatacga 7320
   ttaggtgtaa tacattgtta tcgaatattt tgattttgtc ttggaaatta ttatcttgaa 7380
   aaatcaaaat caaagtaaaa atgttatggt tttcaaagga aatataaaat aaaattttaa 7440
   acaggaaata tttatcgaat gaacgaaata ttaaatttta acttaggcat agaatattgt 7500
   aaaatatttc gaagaattat atataaaata taataataat atttcgaaga actatataaa 7560
   aataaaataa attttaacaa tgacacaaga tattttcaga aatagaaaat aacataaatc 7620
   aaatcattgt aaaaaatgtt taagttctca taaagtaaaa gaaaagaaag aatgaaaata 7680
   tgtaaaagct tgttgcatag atcatcacac gcgtgtcaaa aaaaacacga taaaatcaga 7740
   aagaaaaatt ttatctctca gagaacattt gagaacttct tcagttcagc aaggactcat 7800
   aggctgtttt cgaggtttgg tcgtgaacgg agagatcctg gacattcaca gttacatgtc 7860
   tgttcatttg tccgagatca tcaaagattg caaacctttt tgtcaaccga acaaatgcca 7920
   gaacggtgca aggtgcgtgg aattgtggag caactacaaa tgcgtctgtg agaacaaatg 7980
   ggcccatttt ggcgactatt gcgagaatag taagccaatt ccaatttcaa ctacctttga 8040
   taaatttcgc gatcgaaata attcctttcg tccattgaat tctgcctttt tttttttttt 8100
   tagatataaa caacgaggcg ttgacattta cctcgcaaag tgctttcctg aagaagaatt 8160
   acttcggcac ggacgaaaag gacgaagaaa aatctctgtt gaagaatata ctgatgaaaa 8220
   atatcctaat caatctgaga acatacgaca cttattcctc cattttgtac gcgaacgata 8280
   acttgaacaa ctttgcccat ctttacatct cgaatggcac cagtatagtt tatctgttca 8340
   acgctggtaa cgagattaag aatatcaccg tggaatatcc gggtaattaa aatatttaag 8400
   aaaattcaat ggaattttat gaattcgatg attcaatctc aaatataata atttttcgac 8460
   aaatattttt tgaaaatttt cagtgaacac agggatctcg atccaaatcg cgatcattcg 8520
   gaacgagagc tcgaccacgc ttcacgtgaa cgagtacaat tctacgttga acgccacgct 8580
   tgtattactg gacacgtatt cgaacaaacc ttggataaat ccggagaaag aggtgttagc 8640
   tcctcagagg ccgccggcct cgcccaccag ctacttccag gtgagatatc atgagtttca 8700
   aatcgaatcg aatgatcagc aatgatcagc aatcttatat aggtgaatct agggggtttc 8760
   gatccggatg atttgctgag agtcggcaaa gaggacgtgc aaattaaagg gtacgtcggc 8820
   tgtcttggcg gactaatgat tggagaatac gcggtcaatt tgtccaagct tgcaaacgaa 8880
   gctaatcacg agggtaacaa ggaaatactt cctaattgcc aaacgaaatg cgatgcaatg 8940
   ccttgcaaga atttggggat ctgcaccgag gattttgaga ggcaggaatc attgtgcaat 9000
   tgcgagttga cgtcgtattt cggggagcat tgcgccgatg gtgagatgtt ttagtattaa 9060
   taataataaa ctggattaac tggagttttc ttttttcaaa aggccattaa atttgtataa 9120
   ttattattgg tacgttaaat aatttactta tctcaactgt ctattaaaat cttatctcga 9180
   tcatttttcg caaagagaat ttttaaaaaa agaatttatt aaaaatagat tccagtaaat 9240
   attataataa tggtataaat gcattttgat ttttagaaac ttaatagaaa gtattttgac 9300
   tataatttat agagatatta ctgaaaattg ttattgttga gaaatagagt ttggaaattt 9360
   ttaaacctat tattcaacag atttaatttt atctgttttt tcctgacctc gataataaat 9420
   accggtatta ttaataattt tgaaaatatt ttgaaaataa tacgattttg gaacaatcta 9480
   gaaatttctt ctattatttt ttagaaatac ctgtaccatt aaataatctt taaacgatat 9540
   gcattttaac cattatttat ctatcgaatt tttatttgtc cttatctgac tcaataacag 9600
   atactgttac tgttactatg cttactgttt taatttttat taaactcttt cttcaaaatt 9660
   atataatttt gaaacaattc agaaatttct cacaatgtta aatatgattt aaataaacac 9720
   agatattatc gacttatcgt taaatttctc cccaaattcg cgaccattct aacatttccc 9780
   agatacgata ccaaggaatt tctattttct gttagaaaaa ggggcagact tcagtggcga 9840
   gagcgtcgtt caacgggagt tcgatctcgt tggaaaagtg aatcaagtga aagttcaact 9900
   cgcattttcg accaacgagc tacgacaacg taccacagct cttctactct tacagacaga 9960
   aaataagtaa gttctccctt ccttcctgtc tgaaaattca tcgctcgatt tcatccgttt 10020
   acgaacgcac aaaaatttcg aatcgcttac ctctcgaatc cccttttcaa aagcatttct 10080
   tttttttttt ttttctttct ccctttcaga agaagctact acctgctcgt tgctttaacg 10140
   tcagagggtc aattgatctt tgaggaagac agagaaggat ccgcgttcgg agtgcgttta 10200
   aacgatataa attttttgaa cggggccagg cacagcattt actacgttcg agacaatagc 10260
   accgtcacgt ttctggtacg tcaaatgtaa taattcgaga ttgctcgaac attcctgcaa 10320
   tttctccttc gattatagat cgatcgtgaa ccagtgacac tgttaccgat cccagcgttg 10380
   actctcgggg aggacgagag ccccggggtg accgagatcc aactaggcgg tctgaacacg 10440
   acagactcgc gattcagcgc gtacaaggga tacaccggtt gtctgagcag tgagttccaa 10500
   atcgttaaaa atcgtccgac gattcgaaac aatcgaaaga attgcgaagc gacgaaaaga 10560
   aaaagagacg agatggaaaa gaattcgtcg acagaggaat ggctctaaca gaataccatg 10620
   tgtttcttat gttagacgaa acgatcgtca gccacgttcc gattacgaaa tgacggatac 10680
   cctcttcttg tagacgtcgt ggtatcgatc aacgggggac ccgacatgaa gccgctcgag 10740
   gagtatatgc tattcacgaa acagggaagc gaaaccgtga gggcgacgat gcctgccggc 10800
   gtcaggagcg cgcagtgcgc cgttttccac gtccaaccgc acggccttga cccacccaag 10860
   aacgatagca acgtggtaag caatcctttc gaatttttcc ctttccgaat ttttcgatcg 10920
   aattaacatt gagattctct tgctacttgt tggatttttg tgaacaaatg actctcgaca 10980
   atcctcctgc gagattacaa ttttcaaatc ctcttaataa gaattctgac agaaacgatt 11040
   cttacgtaaa gaaaaatgtt cgatctttgc acgaaattaa attactcacg tagccgagaa 11100
   acattagatt tttatgaaga atctccctta taagtaatgc aaaaagtaag aaatttaata 11160
   tacttatgac acgtgtaatg gattgataaa taaagtggaa atacatcaca ccgtgacgaa 11220
   acaaactttc aaagtttcga gggaaggaag aaggaaggaa aagttttaag agaaaaaaat 11280
   atgtaagggg cggaaaaaac tttgtgtcga agggtgtcga caaagtgtgg gtggaggacc 11340
   ccccggagag gataccgtac aaatcgcaat acacgaacgc gacccaggag gagcaaggtg 11400
   ctggtactta catcttcatc gcgttgtgtt gcgtattcgt gacggcggtg atcggttgca 11460
   tctacgaggt gtggcggagc gcgaggaaag atcggcgccg aaggcgcgca tcagtcgcct 11520
   ccacggtacc cgcgcccgga tcccagagat ggcaatcgca acagtacacg gatccattga 11580
   cgggtggagt gaaaacggtc gggttcaaga acgtggccga ggacgagaaa agaccgaacg 11640
   gcacgcacat gaaggccgcg gtcaaggaat ataaaccgct tgccaacgcg gagtcgaaag 11700
   aattgattaa cgataaaaga gttcatatca taggtaggct gattttgaaa tatcgatcaa 11760
   tcaatttagt ggaaaatata tatatatata tagatgtgtc gtttgtgaaa cgaggattcg 11820
   aatttatgat tataattttt tttatagcag atgaagaacc agagaagaag gagctcctag 11880
   gggtaaatac aggcatcatc actaaatctc cgaaactgaa tccattcgta aagatttttt 11940
   ctttttttta taaaaattat ctatatttta agtttgaagt aagcctgctg aatagatctt 12000
   tcaaattttg caaatttatg atatctatat acgaatttaa gaaagagaac gatgatcgaa 12060
   tcgatgattg agtgttttag taatttgatc gttgtctact ttgcaggctc gcttttcaac 12120
   cgatttgaaa cattcgaaat aaatgaaaaa tccattgatg aataaagtag taaaccaatt 12180
   aggaatattt tcatgaagaa taatttataa ggaaaaaaat ttttattttg tttaattaat 12240
   taattaattg attaattttg ataagttcaa gtattaattt aaaatgtaca tatacaagat 12300
   gttcacatta actatcatga atttcgtgag attattagaa tacgaattaa gattttttat 12360
   tcctatacga ctatttccaa attcatttcg tctcaaaacg atagagatat aaaaacgaac 12420
   tcaatttttc attttcgctt tattctctcg cataaaatct ataggattgt accatattca 12480
   aattatacac ccttacagat attctctaaa catcgaatgt ataaaagtaa aatccaaaaa 12540
   gttaagatat gataaaataa actcaattaa acctaaatgc aggattgtac caatgtatcc 12600
   aaaatataca tcctgaatat agtgaaatta aaattaagat tgaaataaaa aaaattagaa 12660
   taaaacaaac agaattgaaa cagaacagaa accactagaa ataactatat agatatattt 12720
   tgtgaacatc gaacgtatac gtatgaaaga taaatttaaa agaattagaa aaaaaaaaca 12780
   attgaaacaa tgaaaaataa attttatttc aacgtctcaa atcgatctac ttccagtcga 12840
   tggaagatct acaggaggaa cctgaattgg aagaatgcga ggaagaggaa ccagatgaag 12900
   aagatgaaga tgaagatgaa gataatccca aagaacaaaa tacagacgag gaggagatac 12960
   atcaaaaaac agaacgtact gatgataatg attttgtcaa atcggtaaga aaaacatcga 13020
   gaagttattt cttgaataga taggtatatc tgtccttgaa atatgaatca tttgatataa 13080
   tattgaaaga aatagcaaat ctatttttct aaagatctca aagtacatat catatctgta 13140
   taataataag tgaaaaatgt tcagattatt gattttgttg aagatctaaa aattgataga 13200
   gctttaaaaa aatattataa atttatgact tttattttaa aaaatatata taaattgata 13260
   tatatttaaa tatttaatat tctttaattg aactttataa tgcctaataa taattttcta 13320
   aacaaaagat taaatctata tagcaatgtt caaaatttca acaaaatctg taattcaaaa 13380
   atttagaaat tttcttataa atcaaattaa gaaatggaaa ttaaatttta ttattctttt 13440
   ctcttttctt tgtaattttt aaattaatca aattgtcatt caattttatt aagaaaaatg 13500
   tttatttcaa agacagattc gatttcacca taagctatgt tcgaattttg ttggatatat 13560
   tcattaatat tttgattgcg taatacacca atgttattca catccattat catcacacac 13620
   atttgttatt tatctttgtc ggctttgtat gtatatccct cctacacctt ttttttatat 13680
   acatatacat atatatatat acataaatat aaatatgcaa tattgattat gttatacgaa 13740
   cgaataaaac atccttaaag aaatcaattt ttcaaacgtt gtaacatctc attcctcgta 13800
   aatccattaa atacattctc cctacaaatc atttcattcc gataaaccga ttaggaaatc 13860
   atctcataga atcgattcga gaaatgcgaa gagataaaag ggatcgggat gcaacgaaca 13920
   ataatcgaaa atatatgata ctttcagcca gaaaattcgt ctaggataag tgtcgaagag 13980
   agtgttgatt ccttaaacaa caatccgaaa cacgatgaaa caaaaacgca atacgaagtt 14040
   taaaggtatt cagtattatg gtatagcttg ttgcatcctt tcttcttctg attagccatt 14100
   tgctttttga ttataatgca aatgtaattt gcattcattt tgcaaaatat ataacgcgaa 14160
   taattttaat aaaaaggaga agtataaaga tttgaaaata ataaaatatt aaaatattat 14220
   tttgaattat ataatgcgaa ttacattagg attattatca cagaataaga acattagtgc 14280
   cattaatttg ttgtatttca agtaattatt ttattgaaaa tcattgcatg attctgtatg 14340
   attagtataa aaaaattgcc tttttctgct ctccgttttt tatagatatt aattatctac 14400
   tcctatatta aaaatgaatg ctttaaataa agaatttttt ctacatcttt ttagtaaagt 14460
   gaaaaatata atttgttaca actataacaa aaaagatata a 14501
<210> 2
   <211> 46501
   <212> DNA
   <213> Apis mellifera
<220>
   <221> misc_feature
   <222> (27732)..(27900)
   <223> n is a, c, g, or t
<400> 2
   ggaggtcgtt cgagaagatc caggaagaag gagcccgttt atcgagttgc gcgcgtcacg      60
   tgaacacaac ggacgcgcgg ctccatcctc gattaattac cttttcgatc gaggtgaaac     120
   gggccgagag aagggaaccg acagagagag agagaggagg gaaaaacgaa gaggatcgta     180
   acgacctacc gatatcgcac gatcaaaggc aaccgtgcga gggaaaggtg tacctgtcgc     240
   gacgcgagag tgcacgtaaa catcttcaga ggcccgtccc gcgcatcctg gatcgtcctc     300
   gttagaaagt tcgatctttc caacctgaac cgcgagaaaa ccgactctct atcgatatct     360
   atcccacgtt atccgcgtat ccaattatat tttcagtttc ctcttctaaa cgttcccaac     420
   tacgctactc ccacacgtat tgttacgtat aatataaaac aaaacagtgt taaacagtga     480
   ttgaatagtg tcctcgaaat atcgttaatt tcaacgggag attcaatgtt attatattac     540
   ttacgaagtg gagtaccgtg agatatatat atatatatat gtaacagtgg ctcgtattta     600
   taagtgtgtg cccgcggacc ggaagtaaat aataagaagt gaagtgttaa atcgatcgaa     660
   agaagaaaac gggagcggga taatcgataa tcgttccgtg aacttgtgaa cagtgctgaa     720
   tcgatttcaa actctcttgg aattacgagg gatatatata tatatacaaa cggagacgcg     780
   cacgtatttt tatatatata tatatatata aaataggaaa ggattacgca ggaaggattt     840
   tcgttggatt ggaaaatttt tagttgggag aggagctgga gaagagctag agaaggaggt     900
   gacccacggg gggaatggtg atgcggtagt cgagggattc ctcgaggaaa gacatgaggg     960
   cggccgccgc ctacgtcctg ttccttgccc acctcataca ggtaacgtgt acgctttatc    1020
   tgccctattc gcgacaacgg atcctcttcc gtgttccact ccgtgcatgt tcatgtacct    1080
   ccggagagtg ccgacggaga acgtcttctc ttgtctcccc tccgctgcga cctttgcttt    1140
   cgcccgaatc tctgtcaaat tttcccgatt aatcgataat aataacagcg aggttttgaa    1200
   agaaatctac gacgagaatg aaaatttatt tctagataat ttaataattc gccagttttt    1260
   tttttttttt cacgaaccag gaaattaact ttccggtgga tggaggcgat taactttgga    1320
   gaaagtctcg tctcgttgac tttttcctac gaatattcac gtcgacgtga cctttgctcg    1380
   gaaatctcag gatttttagt tagattgatg tatggaatac ttggaaatga aattaataat    1440
   ctttttgaaa ctccatctgg atatacgagc aattataaac cttacacaaa gtattgtaag    1500
   gagatatata aacgaaaatc ttggattcaa acagacattc ccgaaaaata agagattcgc    1560
   gagatctaaa aaaaaaaata aaattctaat aataatcctt cgatgagacg agttttatcg 1620
   atatagaaaa attaaacggg attattttcg aaacgctcct gtctctattt aaaagactgg 1680
   gaaaagtcta taattaataa atcgcgttag aaacacagcg ttaatctcct atcgaattgc 1740
   ttctcatttg gaaaaaaaaa aagaatagtt aacttggata tgatcgaacc gcgagagacg 1800
   gagcaattac tcgccgatcc tgaacgataa gttcgcgagt ccagataagc ctagtaatta 1860
   tcgataaacc agtcgactgt gtatcgatgc cagatataaa taaacgaagg gaaaaaaaag 1920
   aaggagaaac gattacatat taattacatg cattagggtg aacgaagcgc aaactgtcgc 1980
   aaagtggaac gattagataa gtagcggccg gctcgaaacg atgaatcatt gggacgaggt 2040
   ttattaactt atcttagacg ctaatattat tcgatgaacg tgtaaagact gtcgactgaa 2100
   cagtgtatat atacatatat atatatatat atatatatat cacgtataca ctctgtgcac 2160
   atttcgtctc tgccttcaaa tctcgatcct cgaattattt ttccaaatat atgcattatc 2220
   tatacaataa tttactttcc cttctgtatt attatatttc tccatttaca ccaaattctt 2280
   cattctttct acttctttca tttaacattc tccatttcac caacaaagtt tccatccaaa 2340
   tcacagaatt tcttttctaa cactgaaata tacactaaaa cttgccaatg cgaaaaaatc 2400
   gtccatccgc tcaaactttt ccacccgatg aattttacaa acgagtcgca acgagtatcc 2460
   acgttataaa aacattgtaa aacttgcaac cggttgcatt cggttgcatc gcgcatcacg 2520
   taaaacggct tgcaaaccca gcacggttcg tctcacgacg agtttcttct tcctcctcct 2580
   cctcctcctc ctcctcctct tcctcttctt cttcttcttc gtgtcgttgc gcgaggataa 2640
   agagggcggc accaaagcat catcctcccg cttcttgttc tcatcaagcg gatacacggt 2700
   gcaaccgcgg cccttttttc ccgccgatct cgcctcctcc tcacccacac ttatcatccg 2760
   ctccacgcat ctccttccct ccttcttatc ctcctcccgg aggacggaga gttattccag 2820
   cgccgcgtgt atccctccat tgtgcgagcc ggtcgaaaaa tgtgtaacac gttgaatcgg 2880
   tgtaatcggc cacggccctt atttgtatcc ccctgctcgg ttattattac acgcgtacgt 2940
   tctatgcgtg tatattcgtg cgccacaggg atgatcgaag acggagattc cccgctaacg 3000
   cgccgcaatg aattcttttt caagatgata tccaatcgtg gttacttcgt aaattgttat 3060
   tgacaaattt ttttggcctg gaaatttcga atattttttt tgtttcgtta gaattctcga 3120
   ttcgttgtta aacgcagttt gcaacaagta aaacgatgat tcttttttaa tcgtttatcg 3180
   tcatcattta tcctggggaa tgaagtgtga gaaaaagtgt gttgcttttg aaattacgaa 3240
   agggaattag tctgtttccc ctttgttaat ccgttttatt ttagagaaga cgcgcgacat 3300
   gttgattcgt ctggatacga gagacaagcg agagaattat aactgggcta atgtcggtgt 3360
   atcgtatgac caacgggtgg atacgttggc tgcaatgcag gcaaaacgtc tggatataat 3420
   tcatattgaa cgtggaagcg agtagaatac gagtggaata agtaacggct gtgtaaatcg 3480
   tagccgtgaa agctttaaaa ttcttgatat aagtattgaa gtattacgtg taatctaact 3540
   agcaatttgt gaaagcaaga tagaatcaaa acgttcatga aaacatacgt aacaattgat 3600
   gttcgctttc gagaaattca taaggtgtta ataaaagaaa aaaaaaaaaa aagaaaaggg 3660
   gccatttgta atgcaaaaca ttggaattcg tttattttcg atgggggaat tacgtgtttt 3720
   gcaaaaaaaa aaaggaatta attgttttaa ggcgtaaaag tgaaaagtgt ctgtttaaaa 3780
   acggaacgaa gaaatatcta agttaaagat atacacgttc tatgatattc aatctaataa 3840
   ttttacaaat atatatatac tcgtaatagt cgattattga ttaaaatttt tgcaaaaatc 3900
   taattcacga tttttctcat tccaattcgt gtattcgatc tctaattgtt tccaatttcg 3960
   tgacacgcgt ccaaccgtat tacattacaa ttcgagtcga attacacctc gagaaaaaaa 4020
   agaaataaaa ttcaattctt ctttgtcaca gaaccgaaca aaattctttc tctctctcgt 4080
   gtacgagaag ttcgtaaaca acaagtttaa atacctcaac aacctctctc tcgagacgat 4140
   attctcagta ataagggctc gagtaagaag gaaagatgga accactcgaa atctttctcg 4200
   aacccggctt ccagatccca gggagtgaaa gattttttta ctcgttcgaa aaaacactgg 4260
   acgacgggag ctcgcgaaac gcaatctcgt tgacggctaa acacagcctc cgcggccact 4320
   cgaaacggtg cttccaaaaa cgatgcctcg gatgcaaccc cggctgccga gatttcgggg 4380
   gtaattgggg ctcgaaagtt cggagtttgc tgcgcgagtt atgagaaagg ggaggccctt 4440
   tcacaaatga ctttcgagga agagcggcgc acccccctct ttccctcccc tccctcccgg 4500
   atgatttcga cctagcgcaa ttaaccgccg cttctgctct ctttcaatta cagcccgagc 4560
   cggaattacc gttataacgc gcgagatggc tgccgtttgg aaaaaagaag aaaaaggtat 4620
   tccggccgaa aagtattccg agaaaccgat acctcgccga tccaactcgc tacgtgtcac 4680
   tttacgcctc gtatgtgcgt gtgcgtgtgc aatcttgaat aatttctccc gatttaatat 4740
   cgtcaacatg gaggagtggg ataaaattca ttcgacaact caatctattt attgaaattt 4800
   tcgaatcgac ctttcttttt taggggagga agtgtttaaa atttagttta gaaagatgaa 4860
   ggataattca tttaactcgg atgatgaagg aaaactgtct catataagat acgcgtttat 4920
   attggtttaa agacaggtga tcgatacata tatctcgaac acgatggttt gtccgtcacg 4980
   attttccacc cctcccctca cgaatatgga attcgagcat cgaaattgcc acgggagtgc 5040
   tcgagcgcaa ttatcgtgaa tccccgttat ggagatcggc ggggacgaga gatcgatctg 5100
   cggccgtgga atcgtgcacg cgaaatctcc gctcgtacac gtcttggatt tttctcggcc 5160
   cggctcgatt ataattcatc caccgtctcc accgtcggcg cgtcttaatt atccaggctt 5220
   ggaacgtgga acgaaggatg gatcgagaga gagccggctt ctcttccccg tggaagaaga 5280
   aaaagcaagt ggtggagggg aaaccgattg gaggaaggga agatcggtca cgcctggatc 5340
   gcctctaaac gcatatctcg aattaataat cctatatttc ttttttttaa atcattcagg 5400
   taataaagat cgagtcaaag cttctctgca tttccatttc tttttcgttc tttacatttt 5460
   ggcgattgaa taggatagga tttcttcggt taaaatcggc aataaatttg aaggattcta 5520
   gatttcgaga atataagagc gtatgttaaa ttagtcacga atctttaaat aggctccatt 5580
   taagagcaga actggcgagg gaagagaata tcgacgataa taaaatattt ttttatatga 5640
   agatcttcgt aaaaaaaaaa agaaacattc gatatattta tacagaaacg gtacgggttt 5700
   tttcacacga aaattgacat tcgcgaggcg aggatcgcaa aaaaaaggaa cactggcgcg 5760
   taattaatca acgccagcgg atgaaactca ttggagtgaa ctcaataacc ctgtcgagga 5820
   aaattagctc tcgcgtacaa ctctcgcgcg caaatttctc tctctctctc tctcttctcc 5880
   cgaccaggtt tttacgggag cccggtaaca acatcttaac cgccgttatc aagaaatcct 5940
   cttccagaaa cgagaggcga gccgaatccc gccaggaaaa ctctattact ctatcacgcc 6000
   gcaagtacga actcgatcaa tcatcccttc ctcgacttta tatgaaaaaa aaaaagaaag 6060
   aaagaaagaa atatcctatc aaacaccgtg gatcacaact tcttcctcct cgaggagacg 6120
   agcccgaggc tacgaattaa taaaagtaac gatcccataa tcataatccg aaaacggaaa 6180
   aatacatacc agggtggtgc gtaacggcaa tggtaaatca catcaaggtg aaagaagcga 6240
   agcttcatca gcaagaggag agagagaaag ggttgagaaa aagacgcgcg catgccccgc 6300
   ggcggtcgac gacgacccgt ttagcgcatt agcgcgccgc ttccgctttt accctgggaa 6360
   cctatctctc tctcttcaac tattcacaca cacacacacg ctacatccct ttcatacaac 6420
   gaacacgatc gcgttcttgc cgttcctctt cttgctatca ccgcggccaa ttatcagccg 6480
   ccgtctcgcg ggaagttgca gggatacaga gttggataat agttacatag tgcctacttg 6540
   cacgtggtac ggaagctggg acgaatatcg agggagggga aggaaagaga tatcgatggc 6600
   gcggcgtggc gtgacgcgga aatgataaat aagacgcagg ccgtggaaca ggtggagacg 6660
   caaggacgat ggccgtcgag tggaatgaga gcgcgctgtg gataaggcgg aatgcaaata 6720
   gattggagag agagagagag agagaaaaga agggataaat tattcgaacg acattgagat 6780
   ccagcgtctg tgacccaagc tgaacaccag aagatcggct tcgggagagg aaggggtggt 6840
   tgtgcacgtg agcagagaaa gagaaaggga ggaggtgaaa atacggaggg ggagcgggtt 6900
   aaagcgaagc gtttagcctg gcggtcacgc gaacctccta taaaaatcgg gggcctcggg 6960
   gctccggcga tggtccggcg gatgctacgc cagatagacg gataatacgg tctcgtggct 7020
   cggcgttaca ccggctgctg cgtgatgttc cgtgtgtaag ctgtgctgtg gcctgggctg 7080
   gaaacggtgg ctggtggcgc acaggtgcgt gtccgagagt ccttcttctc agcccggctc 7140
   ttcccgccat cctcgacatc ctcggtggcc gaggaacgaa gagagcaggg ctgggaaagt 7200
   ggttaagggt tttacgagtt cggggaatcg ggcgaggagg cgcgcaccac tcgagagtaa 7260
   tttcgtgata aacagggata agtttttggg gcggatgaag tttgttcgtc aacacgtgag 7320
   aagggaagta aacatctcga attctttctt ccttctttct ctttttttct tcttttttcc 7380
   ccctgacgaa aggaaaggat cattggaggt gtcgcacgag tcgaaactct ctttttaaat 7440
   agcgttaatt cctgggaata atttttctaa tgagtttgct ttttcttttt aacgcgatga 7500
   aaaagattgg ggcgggtaac cgaggtaata gagatggaat agcgatttta taagttagcg 7560
   cgttaaaaag tctcggggag cggacgtttt tatcgcgttg gagattgaaa ggaaagggca 7620
   tggtatgtta ggctagttaa agacaaagcc cgctgtcgcg tttaccgcgg ttaaactggc 7680
   ctgttaccgt tcatcgttgc ctgttctttt tcatacctcg tttttcgccg tcgtttatca 7740
   agtttgagag cagaagtggt gtaatttcac gggtgtccgc gggaacgggt gacaaaaccg 7800
   tgacaacgtt tagttacgta gcaaccggcc tggctaaggc ggtctctcca cggtagcatt 7860
   ttttcgaacg gtagaagcat tttactccag actcgacatt cctctgtcga cttcgattcc 7920
   ctgcacaact ctctgctctt ttcacacgcg tatctcgctc tttcgttccc cattttcccg 7980
   aataaaaaca ctttcaccga tacttcatcc aagattaaaa aaagcctcgt aaaagacgct 8040
   gcgatttttc ttttccaatt tttattatta gcaagagaga cgatccggaa tatttccaat 8100
   ttttttctta aaaatttctc gacaatggct cgaaaaaaaa agagacatcg tagaatacca 8160
   gtcaatatcg gatactaccg cccctttgtc tccgtcgaat ggtatttacg aaaaaattcc 8220
   aacggagtgg ccgaccagct ggtcgtcccc tcgtcgaata tcaattggca cgtagtagca 8280
   tcgtcatttt tacgtcggcc gaaccctgtc atcatcgatc ttcaataaac gcttcaatct 8340
   taacaacttg gacaaacccg ttttaattta ttgtcgcacc gagcgcaata tcggtcactg 8400
   tcgccgcgcg cttacgcaac gtgtaatcgc cgctgatcgg gcaaacgatc tatcgcctgc 8460
   ttgatcagag accgtggaag ccgccctctt ttcaacagcc ccacgatcga ttgtttttca 8520
   cgcgtcctcg catgcgaaaa aaatgagaaa aacgagtcga gagaaaattg gccggcaaat 8580
   ttgcgtccat cgatctcttc cttcctcgaa aattacaatt tatacatcat tatctccaga 8640
   gaggaggatc atttgtttaa ttttctttta caaattcgta gattttttaa aaataaaatt 8700
   aaccaattat ttttccaaca acataaaaac atcgataatc ttccatatgt ataaacggct 8760
   cgattacaat ttcgtatcct cttcgaaggg gttatagttc agagaccttc gagttgcaca 8820
   aagtttttgg atgtacaggg gtatcgagtc aatagagcga gacactaacg tctgagtgtc 8880
   ttgactcaca cctgttctcc ttgcttgccg agacgttcga tcccccggcc atcaggtaga 8940
   tcaggttgag gaaactggat gatccatctc tctccctccc ccaccgatcg tcgatcgtgt 9000
   ttcatccacc cttgaccggg ccaacaaaag ctcgatcgat aatcgagcag cctgcctgtt 9060
   tttagtagcg gcgacaagtt cccatggctc ttcccacggg gaaacacgat acacccacgc 9120
   aacgattact cctcaaatcg gctgagaaaa tgccgatcgt ggtgtaccac gccacggtaa 9180
   aaatttaatc tccggccggc cggtggggga ggggggacgg tcagaaaatt attgcgtcga 9240
   gttccgctgt gtgtacacag cgggaggagg gggagggagg atcccctctc tggagataat 9300
   ttggtaaaca cgaggagggt aaactacgag gagcggtgcc gctcgaggag atgaaaattt 9360
   ttgtttgaat ttctatattt gcactttttc caagtgaatt cgatatcgtt tcaaattacg 9420
   gttattaatt tttctctctt tttacaactt taaacaatat cccattcgat gttcgatctc 9480
   tgataaaata gcacacatat acgtatacat atatatatat atgtatatta ttccagcgaa 9540
   agcgtatagg acggctctat tgctgaacct aattagagaa ttaccggcac ggccgatctt 9600
   atcgcatcgt aacttaatta gtccgttgaa ctaaacctcg agccttattc catcaattta 9660
   ataacacccg ataacccgtt taccaattat ccaacgcgaa ttattataat tacaaattaa 9720
   tttgcccgaa atatatttca cggactcgtg tcaatgaaaa taactcgata atcacctccc 9780
   ctctctctct tcccatcaag attttcgctc gaaaacaaga gaaagaaacg gggaaagtag 9840
   gattaaaaat ggaacgatat ccttctcatt aatttattcc acccacctgt ttagagattc 9900
   gcgattaaaa tcgcgcaaat tcgatcgtcc ttcgttcctt cgatcgtgga atcatcggcg 9960
   gtagtcgaga gaaaggctag gagggggagg gagcttccca cgcgagctaa cagtttttaa 10020
   ttatttcggt aatttgtgtg cgccgatgga ggggccggcc ggagggatcg ggcgaagggt 10080
   agcgaataat attaagtata ggcctcctcc cgcgagaagg gagggggagg atattgcgga 10140
   ggatcggagg aaacgtgtgg ttcggaaagg aggaggcggc ggcggcggcg gcggcaatcg 10200
   tggatacgtg gtccgtggtg aaattaaatg gtgcttcggc ggccccatcg agtctatttc 10260
   ggccgggcag gtagcggcca ccggtaatta ctaccttaga acttaggagc tcagaggatg 10320
   aaatgaaagg gcccgggacg attttcgaaa cggaattcgc gtcagttgtt gctcttcctc 10380
   gcgttccccg acaggggaga acgcgttttg ctccttttcc ggttcgactc tgggtcgcgt 10440
   gagcgaactg cttctttttc ttggtgccca gttcgaaaga ggatctcgat ggtcatcgaa 10500
   gatggaacga tcgatcctcc gctgcattct ttgttgcaaa aggattctcg atcggtttta 10560
   ttcaacgtgg agaaactata gaaaaggacg attcctttct gctctgcttg gatttaattc 10620
   gaagaacgat taaaaaaatt agggttttca agcttctcct taaggttaaa tcggttaatt 10680
   tgctggattt tgaagaacga ctcgtgttcc aaatattcga gaaacgagag cgggtggagc 10740
   tcgaataatt aatatccgac gataatggta ccttctcatc gatcccccat cccttcccat 10800
   aacagtttag ctggttttca attaagatat taagcgggca cttgccactc gagcttttgc 10860
   ataattgcat ccagctctaa ttaagcattt acataattac aggtattcca tacgaacggt 10920
   agtagtaacc tcccctcccc cctctgactt attcgcgcta atgagatcgc tgataacaat 10980
   taacccgaac ccgtcccaag tgcaccgttt accctcgatt atccgccgat gatcgccgcc 11040
   gttttccaca gtcatcctgt tattaagcca ccctttgatt gaagagcact ccggccatag 11100
   aaacagccat gaaagatgaa taaagcttgt tttatcaata atccattccc atgagatccc 11160
   cgcttccaac ctgtttctat cctcgagcgc tcaattattt tccaggaagg acgaacgaga 11220
   cgttcaacat tgtctcctcg agattctctc agacccgttt catatgacgt ttattggatc 11280
   gttctggatc cccctgtaaa ttgtggaaca ttgaaactga cgaagagggt agtaagagct 11340
   acggtataac gacatcgtct ccaagagccg tatccaataa acgtaataat aaacgttcgc 11400
   atgcaaaatg aaatatacaa gtctatctgt taccaggtat cgatactcga gatttttcag 11460
   aaccaattta ttgttacttt ttcctcttca cctgaaccga aatttatatc tccaaaagcg 11520
   tgcgtaattt atagcacata ttctataaac gaaatataaa ccatcgtaac aattcccacg 11580
   gaacgcctag cgcgatattt tattatcaaa cgttccacgt tgctcagttt acgacatcgt 11640
   gcggccggaa atcgagtcga aaataaataa aaaaaaaaga aaagaaaaaa gaagagcgag 11700
   ggaagagata aatgagatgg cgggaaagag ggtcgcgaat atatcccatc gatatcccat 11760
   aggatcgcga gaaattgcat gagaaccgat acgaaaggat acgctaggct cgttcctcag 11820
   tacgcgtagt gtgcgccgtt ttttcgctcg aatgagttat tcgtattaac tggcgcgatc 11880
   gttggacgcg aggaagagga ggctaagcga gcgtatgcct cgctgcatcc tggctatgcg 11940
   gaaaggaaag cctgttctcc cgcgaatcgg cgcggcgcgg ctcacggaca gcgaagagga 12000
   gaggagagaa acggctcatc ctcgtcgtgg cgggtcggcc gaggctcgta acgagtcacg 12060
   agcaaagatg tatgtgtgcg tggacggcaa gggccttaaa acggatgtgt aaaaccctct 12120
   cgtgcaccag gtgtccagaa atcaatctcg cgacagaaat cccgcttctt tgtctctgcc 12180
   gatttttttt ctcttttcag agaatagaat tttccaggct cgaaaaagtt cttgaaattt 12240
   tgactttgtc gcaagaggat tgtatcacga aatatacaga attatcataa atatttttta 12300
   tatttttcag acgaatcttt tatttttttt ttttaaataa gcgataaaat cgtcgtaaaa 12360
   gtttcgtttc gcgctggtgg aggaaatttt gaataagcgc gggaattcgt tgcgaagaag 12420
   gatgatgccc ctactaaata gatagttgtt aagtagttgg aggtagttga cacgttaatg 12480
   actgatggaa ctgatgggct ctctgcccgt cgtcgtttcc ttacgcattt aaccggttgc 12540
   tgcgttaatg tctgtttata ttacggtacc gtcgcatacc gatcgctcgt atattcgcac 12600
   gaatcggaca cggaaaaatc gagactcgcc tcgcctcgcc atcggttatt ctgcatcgtt 12660
   cgacaatcag tttgacagtt tgctaaacta aggattaacg aattaatcct ttataaccgt 12720
   gagacgatcg atggaacgtt tcgactggat atgcatattt catattataa cgatggataa 12780
   aagaaatata tataaatctt ctgcgatcgt tcgcattata acaatatcaa taataataat 12840
   atgtcgatca atcattttta gttatttcat tcgatattcc gactgtgctg tgttttatta 12900
   aatcattcga ttgatcgaga aaacattcga atatgtatat tgtacgtatt tgcgaccatt 12960
   atctgaattg ttcgaataat ttcaatgctc gagacaatct ctaatagttg gattgttgag 13020
   gaatttgtac gaggaatagg atattatgga acatgaataa cgttaccaaa gaaacaatcg 13080
   acgctccaca aaggagattc cccggcgaaa aatgagcatt cgcacaattg acccgataac 13140
   cgtgaaaggg agcaactcgg gcaaagcgat tgttgaagag gcgcggtgca ggatgggagg 13200
   tggacgttaa tacgaggtgt ccacctttat tacacctctc gacagctctg aggatcttca 13260
   attatggctg ggtggcggcc gggaccccgg ccatatggga aaataacgga tcgtggctgc 13320
   tcgtgaatgc tccaacatca tcaagacacg tgaatgtcat ttcctggttc tcgtataaat 13380
   ctcgtataaa tatgctcaac atattttgag ttagtgttta aatattcgac gaaaactttc 13440
   ccagtagatt ttcaaaatcc tcttcgttta ttccttcgaa attctgagat ttcaacaaag 13500
   aaaagtcttc tatcttaatc taaaataaaa cgtaatcctg cgtaatttct tcttcaacgc 13560
   ggaccgttcg aagatcgttc cacgaaacca ttttcgaacc tcgtttaaaa aagaaagaga 13620
   gagagagaga gaaaaaggaa aacgtcggta tcccgatgag aaggggcttt gaaactgacc 13680
   gcagttacac caataaagta cacagcaatt ttacactcga gccttaccat accttgtctc 13740
   gcctatctcc tatatataga ccaaagatca cgatcgatcc acgaagatat ctgccagaag 13800
   atgactaaaa agagagaaaa agaagaagaa gaagaaaaaa acggcgaaaa ttcctaaaaa 13860
   cgaaacatct aatcgtccca attaatcctt tatgagagaa ccggatcacg cgtcgcgtcg 13920
   cctcgcatag gttcaccgcg attcgaccgc gtgagaaaga ggcctccggc ctcgatcggc 13980
   tgttcaaaat tccagcctcg tttttccccg caacagacac acatacccat cccctataca 14040
   cgcacacata tgtatacata tatacacgca tcatcgaacc ataccatacc gtgccaactt 14100
   gtcgtacacc gatcccagtt tcgaaaaagg ccggtgctct tccgcatcgg cggcggcggt 14160
   ctgaaaagcc atctccgcga caccggccaa tttcacgtcg gcacgtgagc ggactgtgca 14220
   aactcgggcc gcgcaaactc cctccctctt tcgccctggc caacagtcgt tcacggtttt 14280
   cagctgcttt cggaggcaac gggttaccgg cgagtgcctc cagaataagt atgcacggct 14340
   aataaaacaa cggaggagag agcgagagag agagagagag agaaacggtc gttacaccgt 14400
   cgagttggtc aaacacggtt ccgagctgag ggcggaaacg agcaccgctc cttctctcgt 14460
   tccttctctc cgatcgtaaa cgacggcgac gacgactacg acgacgacta cgacgacgac 14520
   ggcgacggcg atcgagcgcg cggaaacggc tcgagggaac cgaaagacgg cgaaagaaaa 14580
   cgcacgctgc tcgttttacg gccgcgcgac acgctccgcg tttgcacgct cgattgatat 14640
   attctcccct cgggaggggg tggggagaga gactccggtg gatatattgc cggagggaga 14700
   attatcggtt tttagcttgg aaaagttttt tttttcttct tcttcttctt cttctagttc 14760
   tttttagaaa gagaagcttg tgtacgactt ggggaaggga gggagggggg gagggagaag 14820
   gtgtccagag cgtggccacg gcgcaagtta gtcacgattg ttttagaaga aaattcatac 14880
   tctatgacgg tttgtacgac ggttgtataa aagcatggag agtggagaat gttcacaatt 14940
   gtgtgattta tgctgtgatg cgaatataac gtagttagtc acggataaca cgtgcaaatc 15000
   gcgtgtattt ctctcgggag aaagagatat tggcaaatgt tgctaagtat tattggattt 15060
   tatttaactt gggaaaattg tttgagaaca gagagatacg agagtcgtgt gttaaattgt 15120
   tatatgttgg agaggaaaaa aaaaagtaag aatgttgttt tttctttttc tttttttttt 15180
   ttttcccccc cttttgcgat gagatgggaa cgtgagaatt ttaagtttct gtactccagg 15240
   tggggaaaaa gagaaaaata gaaaatgcaa ggatgatata tgcttcgact ttggggaaaa 15300
   ttttcttcct agaggagggg aggaggaagg ggaatgaatt atatttgatc gaattttcaa 15360
   gatagccgaa ctgctgcctt atttctattt tgaaagttat accgattaat aaccgcttcg 15420
   aaaagtgaaa cttctatttt tattgcgtat aacaacataa gtgaatataa taaactacgt 15480
   ttttgtacac atagagtgta tcgacgaaac gcaaatcctc tttcacgacg aataattatt 15540
   accatccaat ttatgaatcc tcatcaatac tcctattctg gaaaattctg aaaatttatt 15600
   cctatgcata caagatcccg atcgaaaaaa aaaaaattca attcaattca aaaacgacaa 15660
   acgtgtccat ccatcccaga agatacgaac tcgatacgaa atcagtttcg ttttaacaaa 15720
   gattctcgcc cccctcctcc tctccatcat ttcctgcacc cggacattat ccgtaattca 15780
   ctgctcaatt tcctcgagag attcatcctc ccaggccctc gcctcgttcg gccgcgaaac 15840
   cgcagaagct cggatcccag gcaaaatgtc actcgaagag tggaacgtta tataacatag 15900
   taggaagcgg tttgtggaaa gcaaaggcca gcagacgatc ctccgcgctt tctaccttcg 15960
   atcttactct tacggtaatt acacacaggc ccccgggggc ccgtgcacga tcccctcgct 16020
   cgcttcgtgc acacgcttat gaccgagcgc cgtacatacg tacgtctctc cctccctcct 16080
   cctcctcccc cgtatacaca cacacgtagc gtagaatgca cgtgcccgat tcagaagcgt 16140
   gcattcgaaa ccgataggtg taggtgagag atagaggtgg agaggaggga tttcgtaagg 16200
   ctcgaaagga aggctctccg agcgttcgaa aacgggacga tttcgtacgc cgcttttgta 16260
   tcgtcaatcc gcgtgcaatg caacggatgg gcgaacgaaa aagcgagagg cgaaatgtaa 16320
   aattatgttg gttgcggcat cgattgcgcg atcccatttt gaccgtgaat gggaaagtag 16380
   atagtaggtg aactttcgcg cggaatagat cggattttcc agagaaatcc tccgagatta 16440
   ttcgtgtcaa ttgctccctc gatttatcga gctcgagttt tgaaattgaa aatgtaaaat 16500
   tgaatgtaaa agttctttca acgattttga acagtcgtat tttttgctga gagaggtgat 16560
   aaataaagtc atcgggtaaa aggtactttt agaagaaaat ggcggggcac gaaacggtgt 16620
   ttcgaaaccg tacgatacga ctttttccat cgtatcggtt cgaagctgcc tcgtaataga 16680
   cactcgaggg gcctttcgcc tcctggggcc gcggagactt ccgttttcgc cagagaccca 16740
   acctggaaaa tcccgctgtt ttattatgcc cattagaaac gaacgaaaca tcgtgctccg 16800
   ctccgacgtt ttctctccgc gtaacgagcc cgtaataaat ccgttggaaa aaatgtagga 16860
   taggattcaa cacaggtatc gatatcgaaa tgtcgaggat ctcgtcgatg aaattgaatt 16920
   acgttttgca gagatattga tttcgaaaaa ttggaataac actctcgaga aagtattctt 16980
   cgattgtgaa tgtaataaaa gtgaagagat cgtaataaat tttttttttt ttgttatcgg 17040
   agcaggcgac aaacgcgagc ggattcttcg aggtgcaaat cctctcgctt acgaacaaca 17100
   ggggcaccct ggtggacggt aggtgttgcg gcgggggagg cgatggcgga ggaaaaggag 17160
   aattaccacc gtgcacgaca ccgtgctcga cggccttctg gctctgcttg aaggaatacc 17220
   agtcgaatgt cacagccata ggctcgtgta gctttggcaa cgtatctagt cacgccctcg 17280
   gccagaacac cttcaccctc agcgaaccgg tcaccctgca actgcacttc acctttcgat 17340
   ggacggtgag tggatcttct tttattttat tttattacac gctattctat tatatcgtct 17400
   aaaattgtat aattcggatc gttgaataaa atagtttgga gagaaacgaa ggaaggagaa 17460
   aattcttcgc gattcgaacg tgaaaatatt tatcttcttc gttacgtttc gagcaaacaa 17520
   atctctcgtc ccagtctcga attatcacaa atggcgtaaa tgatattttc tcgagcaaag 17580
   gaacgaaact ctcgccttcg aatggtcggt taaaaataga aagaagaaaa gaaaagaaaa 17640
   acattcgacc cgattgtaag tcatacgggt tcaaatacgc gagttatcgc gtaaaatttc 17700
   tctccttcgt gaccatcgct tctaaccgct tcgaggtgcg agaggcatcg accattgatc 17760
   cttccttcgt gaaaagaatt tttcaaatgg gacaaataaa attctcaaga ggctcgggta 17820
   taatcaatgt ttcgagattg tctcgctcgg aaacgattat acacgtgcat atattacacc 17880
   gtattcaatc acggaaggag atgctgaatt atcggcagac gtaatgaaaa tcaagtttta 17940
   ccgtttttca tggtggcaga aatgatttta taattattac aataccagta tatcatggaa 18000
   attacataag ttaaaccgtc cttaccgtgc atttttcagc tgttgagttg aaagttccat 18060
   ttatccatta tctgaagaaa tttaattatt attccaataa ggaaacgaga cgtagaaatt 18120
   ccataataca cgattctctt ccattttcat gaatcggaaa gattcgaacc aagctctctc 18180
   tctctctctc tctctttctt tctctctcaa gagttttgat actcttctga tactcttctc 18240
   agattcctcg cacgaaaaaa aaaaaaagaa aacaagaaga aggagaaaaa aaaagaaaga 18300
   aaaaaatacc acggcgttaa cgctccaacg cttaacgctt accctcgaaa aaaaaaatcc 18360
   cgtgtcggcg tgcacgtgta tttactttcc atatatattt ttttattgcg cgcccatggc 18420
   cccataattg aattagcccg ggcagcgcta aatgctcgaa gaagcgtaca cacacacaca 18480
   cacacatacc cgttcgtgaa ccgtcctagg acgaaaaggg ggagcggagg gggagggaga 18540
   gaggagagag agagaaagac agaaaaaagg aacgaaaaaa gggaggagag gaaaaaatgc 18600
   ctgacggaaa ggaagcaatt taagggtctc tgtttatgcc cctcgagaag agaggagagg 18660
   ggggatacca tcggacccac cctttgtcgc ccctcgaaga aacggacccc tcatcgggtc 18720
   cctcttacag cctcgagcga gcgagttatg aaatatacaa acaggctagt gctcttcaaa 18780
   taatccacca ctggaccgtc cgtcgcgcgt gtgtgtgcgc gagcaaacgt ctatataatc 18840
   tctcgataca tatgcacgcg cgtatgtacg tacgtagaag aaagcagagc tcctcgattc 18900
   ggtccgttag cttcccttgt tgttcgagga cccttgacgt atacgctctg accgatgtgt 18960
   atacctcttg ggcgcggaaa cacgtcgtca tgcttggctc tcgagcatcc cacgagcatc 19020
   tcatcgaaat cggccgttcg acgattatac gctcgacagc ggcccgacct ggctgcccgc 19080
   cccggtgaca cgtcacgtgg gtggatgcaa tgctgatcgt ctgctcgcgt cttcttcgtt 19140
   ctctactcaa cagtttttaa ttacatatat atcggtgata taatggtaat tggaaaccga 19200
   tttctaataa tttctaatga aagggaatcg cgggtctctt cgattcgtag cttcttcgtc 19260
   tcttgggatt cgaaagattt atatagtggg aagttttgcg ccgattcttt ttacgtaacg 19320
   gaaatttcta gaagaaaaga aaatttcgag gtaggtcgaa gaggaatgaa tgactaagaa 19380
   tttctggaaa ggaacacgac gaaaggaagc gtccaagttt acattattca tatttcggtc 19440
   gtttcaaaaa aaaaaaaaag aaaagagaaa cttcgtcgaa aaaggaggcg tcgagaagaa 19500
   ggcgtgaagt gccaagtggc aaatctttct ctccgttctc gcgccaaaaa cttcttcgta 19560
   cgtcgtttca tcgatggggg tcggaaattg gggtgttgcc agaagagaag aggtttggac 19620
   tcgagaaaga tttctcgggc gaaaagaaag gaaggaagga aaggaaagga gaaatagagg 19680
   aacgcgatga aggcgttgcc aagcgactgg aaccgaattg gaattctacg taacgactgc 19740
   tcgccgatag aggagcaaca gaccggaaat cctttggatt cgatagctct cgcgcgtccc 19800
   gcatcgacaa aggaatcggg gctagaaatt tgcgatcgac gtcgcgatac aatgcctccg 19860
   tgtgcaagcc tttttccaac ggagaagtcc ccccctctct ctcttatatt gttgcgaaat 19920
   tgcctctccc ccgagaaaag attcactcgg ttgcctttca acgatttcga gaatgagaac 19980
   aagaggccgc cgtttaatag aggcaggggc cgcctctaaa tgagatttct cgcgatccga 20040
   gctcgaaatg gaacgcggaa tacgtttata tcttgaaatc tcgttactat tgtacggaaa 20100
   aattctgggc aatgatctag attcacgaag ataatcaagt tattagtttc aaagtatcaa 20160
   aaatcgagca aatatagaat acaaaatatt gtgtgaaaag caatcgatta cttacggcca 20220
   aatgtttgta aataattgaa taaaatacaa gacacgaaag gaacgcggat acctttacat 20280
   ttcgaaatct cgttacctaa taacagaaaa attacagaaa aattctagac gcttcagaat 20340
   tttcacgaag atagtaaaat tatgaatcgc ttatcctcta aatttcgagt agaatagaaa 20400
   attctctgtg aaaaccggta ctgaaaagag caagtgtcgc cacgaataag cgaatataaa 20460
   tacaaaaatc gtacgatcat cgacgcgcct aattccaaag catcgcaata tggacaaaag 20520
   attgggagaa cattgaacaa acccttacac ccttgcgtat aatctacctt tcaaccgcct 20580
   tatatactgc tccacgcgtt taagaccgca gtcctccaag ttaataagca cgccattcga 20640
   taccggttac cagccgaagg aagaaacaaa catacgtgta cgcgtatcca catcgcacgc 20700
   cggaataatc gtccttcgac tttctccgtt tgcgatttca atagcggcgt aaacaggcat 20760
   acgtaagaag ggaatacaag ccggcttttc agcggtagca taatggcaca ggaaacgatc 20820
   accgagatga gagttagccg tgcctttgca caatcctggc cgcagcgtct acgcgcagac 20880
   gccttcgacg tctctccttt cgactcaccc ctacgcgtag gtgtccaaaa atgctctgca 20940
   tttttctcca ggacctccat ccaggaagtt tccccttctt tctttcccac aatcgttcgc 21000
   caattttttt tctttctcct tgcaagacat ggaatcgcga ttctactatt gaatcaatct 21060
   ttttattttt catcgtcgat ctttataatg taattcgtcg agacgcgatc ttcgtcaatt 21120
   ttattgcgtt tattgcgggg agagaatggt agatggatcg actttcttct ctttcttcgt 21180
   tcccttttta gaccgcgatt ttcgtcgatt ttcgttttca ttttgcaacg agtcgcgcga 21240
   ataggtggat cgatagtcta ttcttggcac aatatccttc aatgtgggcg cctatcaatg 21300
   acgtgacgtc acggttggtg ggcccacgtg gatctcactg ggcattgtgt cggctgaatt 21360
   agcgagtagc cggcacgaac gtgttggctt gcgcgagggt gacgagattg aaaactgttg 21420
   gatgattcag ttgcggagat tgtcgttgta tgtattgacg agagcaatgc gtaaaaatag 21480
   ataattgttt atagattcgt gcattcatta tcactaatta tgatttattc tcggcacgaa 21540
   aatgatcgaa tgattattta taatcgtaaa agagatggaa ttggttaaac gtacgattta 21600
   cggtatattg atcgaaggat tgaatgaata aatacttgga attaaacaca ttataattcg 21660
   acgaggaata ttcttggttt attgttggaa acgttattgg acgcgtcagg attgaataat 21720
   aatcgatcgg cgcataaatc ataggccgag aaaaagaata acgacgtatc tatatgattt 21780
   aataaatgat actcgtatta tcaagaagcg aaagctatat accaaaagtt tagaatcaaa 21840
   aaatatatat atattcttcg ttcccttcaa actcacgaat cgtcattaca tatccaaccg 21900
   gaatcaaaaa taatcttcgc aaaaaaaaaa gagaaaggaa aagaagtttt gccgctttcg 21960
   aactgatcga tcaatcgata tctccacgtc gcacaatcgc gaagaaaaag aaaaaggtga 22020
   aggaaaggga aaaaatggaa attattctcg aagtcgggac gggtctggcc gaaggacatc 22080
   ggatgatcga tgcgacgttt aaacggccgt gtcacgcgtc aaacggcatc attcaattta 22140
   aaccgagccg ctattgagtt ccaaaccgcg gcgtcgtgta agtagattag ccgaggcggc 22200
   tttaatcacc tcctggaggg gactttctca cgccttaata aagttcgtcg ttacggttcg 22260
   ccgtgcctca aaggctcgaa tctttcaaaa ccgttgatcg acgtaactgg gccaactttg 22320
   gaaaccggag accgaaaact tcttaacttc attctccccc ctcctctaac gagctaataa 22380
   taaaaaatct cttaaatttc ccaggtgatg tcgcccgtat ttatcgacac cctcctgaat 22440
   aatcgagcta attttcgtgg aaggggaaag agaaaagccc gaattggtga gaaaccccgg 22500
   gctccccctc cccccgtggc gcgtgataaa tttcgataat tatagcctcg ataatgggcc 22560
   gtgtaatgat aaacgatagt cgtctaaacg gtgttcgacg gcggagggaa tggataaaaa 22620
   ccgcgggggg acgaaaatga aggagcaagc gtgaggaggg cacgcctcga atccccgcag 22680
   gggatcatcg tcgtgagtca ggatccaaca ggtgtattct tcgcgtcctc tcctccaaaa 22740
   ccattctctc tctctctctc tctctgtctc attctccgct cgaaggggtc cctgcatact 22800
   tttcgggact cgtttcctga ccccctcctc ccctccttcg accaatctcc tccctccctt 22860
   cgtcttcgtc gatatcgcat tccattcggt cgcctcgcaa gaggccggtg aacgatggtg 22920
   gcctcgtttt tctagaatta tctgtgaaca ttttggaaac gtggcggtaa ttcgttcata 22980
   ttaatcgttt ccggttgctg acgcctcgtg acggcgtgca aattttatac agcaacgcgt 23040
   ataacaattg tcacaattcc gcttgatatg tgtgtgtgta ttttcgccag cagttgaaat 23100
   tattgaatat ttctttgatt cgtcgttgag aatcgttgaa aaattaatcg aagaattcca 23160
   tccctcccct tcttcctact cgacggatta ttattaatcg atcatcaccg attcttcttt 23220
   cttacgaatg aaatcgtaaa agccggacga tcgaagcgag gaagagagag agagcggttt 23280
   accaacggat gtaccaacta gatagagata ttcgtggcgt gtcatttata ttttcgtaaa 23340
   gggggggtcg attcctaagc acgaataaat cacgcagcgg cgaagatacg gaagcaggat 23400
   cgatttgtca agggagatgt gcgcgagccg gtcgattaat cgagcgaacg aacggctcca 23460
   aaatcgctgt tcaaccgccc gcgggaatcg caatcagcct cgacttgttg ttggccgtcg 23520
   accgatcaaa gagatttacg tccaactcgt atgaaaaatt tcccatccgc ctttcttttt 23580
   ttctccttct ctttctctct ctctctacaa ttccctttga cgcgcctcct cgctaatgaa 23640
   cacgcgagtg cacgtccgag aatcgcataa acggcggtca ggtaattaga aattttgatc 23700
   gaacatccca ctttttccct cggccccata ttaacgacga tccgctacac gccgatcctg 23760
   ttccaccaac gtagaaataa tatcccctct cccctcctgc tttcacatcg tccacgtttt 23820
   tccgtttccg cgttcccaca tgcgtgaatt caatctagat tcctctacgt ggctcgctta 23880
   attcgaggcc gagcctcggc ccataattcg cctcgccgag attattaatt taacctcccc 23940
   tgagaaaagg agcgaaagtc atcgattaaa aatggcgggc caacgaaact gccctcgttg 24000
   acgcggcagg gggcccgaag agtggggagg gggtacatta accgtggaga tttacgagaa 24060
   cttatcgatc aggtgtggta cgtggctaca cgaggggcac gcaacgcaaa gtgaagcaca 24120
   caggtgtaca gtatcaagcg tatgcaaatg aaagaggagg aggaggagga ggaggaagaa 24180
   actgtcgccc cggtttgggc aatgcctcgc gagcttgtcc ttacctccta cctctccatc 24240
   ttcttccttc ttcttcttct tcttcttctt cgtcttcttt ttcttcttct tcccctcctc 24300
   ccgtttctcg tctctctttc tcaaagcaga agactggctt tgggctggct ttgggtcgtg 24360
   tgtgaacccg ctgcgcggac cgcataccaa tctcctcctc gtgatccctc ctcttcttgc 24420
   cttcgcaaca gtggatatcg ttcctcttcg ttctccttct cctccttttc ttcttcttct 24480
   tcttcctcct cctcctcctc ttcttcttct ccttcttcca tcgctagtta gcccgtggta 24540
   cgagtcgctg ccgttgccat cgtgtctccc gaaccacgtt ttccgttgcc tgtaagtata 24600
   caggtctcga ttcttcgctg ttgcgacctc gcgtgcccat ccgcgatcgt gtatatacgt 24660
   gtatatgtgt atatatatat gtgtgcgcgt actatgtggt attatgtcca cggtggaaag 24720
   aattcgcgta tgggtacctc ggcggatttt agaggcaatt tttgaatgcg ttatcgtcca 24780
   cgcttgaaat atcgccatta ttcccttgag cgtgctcaag ggagattgta atacgaatgt 24840
   ttagccagtg ggattttatg gccggaagag ttatggcgat tgaatattta acaacttttc 24900
   gcgggcttct ggatttatgg aacgttcgga caaagcgcga ctcgtgtacc tcataagcgt 24960
   ttaatcattc tctgggtttt tttctttttt ttttttcgaa agcgaactca aatttaaaaa 25020
   atgtcatcgt tttcgattca atcctctcta tttttccatc aaagctaagt tctacttcca 25080
   gagaacacct ctgaaattct attctttctc gtgggtactc tctgtgtgta cgaaataagc 25140
   cacgcatacc tgctcaacct cgatcccatg gaacgcgcat gggggacgaa ctcgcgtacg 25200
   tgtaaaatgg gcgcgagagc tcggctcgct aggctcgagg aggcatttaa cgcgaagaat 25260
   gtcaggttcc tcgcgatacg caggtagaga ggcacgacaa accaccgccg gcatcgcgag 25320
   ctggctcctc ctttaaaccg cgtctccttt aaccgagcga ctcctcgtcg cgacatcgtg 25380
   ccaaggcttc tcggccttgt gcccctgtga acagtggctt gaacagaccc cgctcgagaa 25440
   tcccctcctc tctcttcctc ccttcctccc ttccttcctt tctttctttc tttctttctt 25500
   tctttctttc tttccgcttc ctctcctcct tcgacgcttc gttttttcct ctctcgaggc 25560
   ggtcctcccc agctacgctc tctccctttc tctctctcca gagtctctcc acggttctcc 25620
   tcggggacaa cgcgagattt attagccaac cggttaattg ggaaccgtgg acgaggaaag 25680
   gattccagaa cgaaacttcc cttcgccttc aaacgttgga attttcaaag gaacaacccc 25740
   ttcttcttct tcttcttctt tttcttcttc tttctcctct tctttttgtt ttgtgccgat 25800
   gaatcgttct cctctctggc ggaggagagg ggggaggttt ataagtctgc gaatttatcg 25860
   catcgcgacg cctgacctcg agacccccga ttgttgcggc gagatttaag ggtcaagggt 25920
   aaacacagcg gcggtgctcg aaagcgatca aaagtcggaa ttccgccgag ggattcctcg 25980
   acctcgtccg cggctcgcgt tcctgcgaaa tagctctcga aagtaattga acggtggagc 26040
   tgtccggaat gcagctttcg gggaggatcg ccggccctcc tccaaattcc ctgcaatcga 26100
   atttatcgcg gatcgtggag acagagaggt ctggatcttc tcgagctgat tttcctaaca 26160
   tttgtaagaa agtttcctat ttcatattct atttcatcgt tctttaaaag gattttcttt 26220
   tttgaaattc acaagtgttg ttacgaagag agaatcctcc tccaaattct ctgaaatcga 26280
   atttatcgcg gatcgtggag acagagaggt ctggatcttc tcgagctaat tttcctaacg 26340
   tttgtaagaa agtttcctat tttatattct atttcatcgt tctttaaaag gattttcttt 26400
   tttggaattc acacaagtgt tgttgcgaag agggacagtg aatcctcttc caaattccct 26460
   ggaatcgaat ttatcatcgt ggagacagag aggtggatct tctcgagctg attttcctaa 26520
   cgtttgtaag aaaatttcct atttcatatt ctatttcatc gttctttaaa aggattttct 26580
   tttttggaat tcacacaagt gttgttacga agaaggacag tgaacaggcg tcgtgtaagt 26640
   ctaaaactcg aacgcaaaag aagaggaaag gggacacgat cgaacgtaaa cgataatttc 26700
   tccaatcgat aaacctcgtt aaacttaaaa tcgtcggcat tatttataaa ttcgtgtagc 26760
   gagatattag taataaagta gccaccgggc tcgtggtaac gagcaatcca attgaatttg 26820
   aaaatctttg gacacgcgcg tgcgggcacg cggggaatta tattgccgcg caagtaaaac 26880
   tttttcacgg aaaccattcg aaatattcga cacgaccccc gtttacgggg aatctgaatt 26940
   cgcaaagttt acggcgggaa gaaatttgca aattgttaac gctcgctcgg ccacgttttc 27000
   aatttatttt aatttctaat ccttggcgta tgaccatgca gcgccataaa acacgaaagg 27060
   aggaggatca ttgaaagaaa gaaagaggga aagctcgcgg aataaatcga caaaaggcat 27120
   aaaccattca aggcttcccg gatcggttgc gggcgtgctt ggttggattc gctcgctgct 27180
   tagtacgcgt cctcacgtac cgctcgttcg atcttttccg cgaaaccaaa gacacgttgc 27240
   ctcacataga gaaggagaag gcgttctatt cggcgtacgt aagtatctac gtgcagttac 27300
   cgaggcgcac gatcgacgtg taacgcgtct cccggcccat cggcgagcgc cacgacggaa 27360
   cgacggccag atggagccgc tcctcgacct cctctacgtg cttccgctga tccttttcgg 27420
   acacggtgca ttcatcgttt ctacgatcac cgttttcatt atcgtaaaag cgttcctgca 27480
   tttcgaattg cagctccaaa gttttttctt tctttttttt tttttttttt tcttttttgg 27540
   aatgagagaa agaagcgata ttggtagtat ctctgtaaaa ttaattttaa aggttatttt 27600
   ttttcttata aagattaatt caaaattgat gacgatttaa atattatcgt tcttaaattt 27660
   attttaagaa acgattgtta tttttttttt tttcttagaa tctcttcgaa tctaatgaca 27720
   ttccatgcaa annnnnnnnn nnnnnnnnnn nnnnnnnnnn nnnnnnnnnn nnnnnnnnnn 27780
   nnnnnnnnnn nnnnnnnnnn nnnnnnnnnn nnnnnnnnnn nnnnnnnnnn nnnnnnnnnn 27840
   nnnnnnnnnn nnnnnnnnnn nnnnnnnnnn nnnnnnnnnn nnnnnnnnnn nnnnnnnnnn 27900
   gaatctaatg acattccatg caaagtaacg gtgttgtacg atgtttgttc agttacttta 27960
   ccatcgtata aataacatat tactcgctaa tcgtcgaaaa gtgtccataa aagctcgagc 28020
   cgagtaacga ccgattcatc tctcggtatc caacgcgaaa taaatacata cccgatctgt 28080
   aacgcatcgt gcatatccat tatcccccct cctccacgcg ttatcacgcg tgaaatcacg 28140
   cggatatgta cgacgataaa tgtacgatga tcgagaagtc gcctcgatgc acgaatagcc 28200
   gagccggcga caggaaatta attaattgat ccacgcttgg aaccgcatct tgcggaatga 28260
   tatccgactc gatgaatttc ctacgcccac aacgcgaccc acgacgtccc tcgacccgga 28320
   tcgatcgatg atcgagagac acgcggtggt cgcggatcct gacttttacc ccgacttttt 28380
   atccatccga taatttattg cctggaacgc ctgtgcgagc tgttgcacgc tccgctcttt 28440
   aaatgcgttc gaacgaatag agagagagag agagagagag acgtggtttc ggccggtgaa 28500
   aggggaaatt cgctcctttt ctgcgctacg cccacgcgaa ttcctagatc catcgccacg 28560
   ctcgcttgga gatccatcgc ttcggttgga tctttttcgc gcggtggata ctttcattga 28620
   caatgggatg gaagaatata tataaattcg aaagtttcga aatttgcacg gtggaaatta 28680
   atttattacg aatccgagat attttacgcg gtttctaatt atattgtgat tataagatta 28740
   taattgtcga tatattaaac gtactcgatc ttggaatctt tttcagagtt atcgtttaat 28800
   taatttaaaa agatgcaaac agtgttaatg cgagagataa ataatccact tgctcgagct 28860
   gtgatatact ttcgttggaa acaatggaaa ttttgaaagt catatacatc ataataatta 28920
   caattattat tacagaaatt gaattttctt cttttttcca ttccatataa aatcgagatc 28980
   gatcatccgt ttcgaaagac aaaacgaaaa aaagagaaaa atctatcttg atcaaggaaa 29040
   tttgttccac tttcttcatt ttctctttat cctcgaaaga aaggaagaga ttcgatcgaa 29100
   aatgcccgaa taatagaaat cttatccaga tcatcccaat cccaacgttg actcatatcg 29160
   atcgagacgg ttgagagaag aaggtctgat cgacgaatgt atggacggac gaattagaat 29220
   atatatatat atgtacgagg tgcgagggaa aaaaaaggcg agcgaatgaa agtcgcgggc 29280
   gtgggaacgg cgggccccaa gctcgccacc aattaacacc aattaacgct catctgctct 29340
   aggtagtggc agagacgagt gaaccgactt tacatttagc gcggagacgc tcgtgttcaa 29400
   ccgtgatcgg ccacaacggt gctctttatg tccaggtcca aggatttttc gcaacccttt 29460
   tcaaaaaaga ttggatcgga cgagtttctc cgccatcttg ccaatcgact acacttttct 29520
   cttttataca attcgataca cgtatcttta aagagttgct taaaaatatt tattaattca 29580
   gtcaaaagaa ttaaataata atcgattccg aatcaatttc gagttgtact actttctcta 29640
   atttaattgc gagcgaatat cgaatatcgt ccaaagaaga agaggaagaa gaagaagaaa 29700
   aaagcatgga ggcggaattc gcatcaaagg cttcacctcc tgagtgaaaa tctcggggaa 29760
   tcgaacaggt ttttcaccat actttttttt ccctcctcca aatcggtcgc gttggggtaa 29820
   gaaaaaacct tctttccaac ggtggaagtg tcgcgccgct ttaggaagcg atttgtcgcg 29880
   cgagaaacgc gcgcgacatt agccgcgtta tctaagcatt taggctgccg gcgggggaga 29940
   ttgttttcgc taattcgccg atcaagcagt gcggtcacga tcgagcggtt agccgggcgt 30000
   gttgaacggg tgtcccgccc gtttttcaga aaaggaatcg atcaacggag gttcccacgt 30060
   gtatcgtccg ttgctaagga acggactgcg tccccttctt gatccaaact attgctcctc 30120
   gcgaaggatc gaaaaatcga aaaatggggg tggaaaataa gataccctta attttcgaat 30180
   tgccggttga ccgatcacgc gaaacacctg cgattctaat tgaaaaaaaa tggaagcgag 30240
   agagaggtga agctttcaac tgaagtccca acctcctctc attgtctacc aaccgagatc 30300
   cgaccgagtt cattaaaccg ggcgcgcgca cacacgagcc gggtacacgc gagatcaccg 30360
   atgcgcctcg atctttttct ctctctctct ctctcctctc ttttttttct ccttctctct 30420
   ccgttgcacg ggaccggttc catggggaac acttccagct ttctatcgta cgtaccttct 30480
   tggacaccct gtttgggatc cgtcgctcgg cttacctccg cttctggtct cctctctcgg 30540
   cagctctccc aacagatagc cgctgtcttc cgcgacagcg cgattggaat ttatttattg 30600
   cattagccga ccgtcactca cggcccttga ttggtggtag caatcgaggt acaccgcgga 30660
   gaggaagcgc ggtggaggcg aggagaggag gaggaggagg agagagcacg caaactctgc 30720
   cggctgtaat tattctgatt tgtggcggag ggacgacggt gaaaaatgtc gacggccacg 30780
   ccggccccct tccctcggta attatcggcg gcggtggttg gccacctctt tcgataatcg 30840
   cgtttgtttc acttcctccc tgattctgcc tccctgattc tccgagcgtc gcttccactc 30900
   tctcgaggag aggttgtcga gtactttttc tccattactt tatatcgatc aaccaccagg 30960
   agagggggtg gaatgatcga acgttgggtt taatccgcgt tggatcgaga aggattagga 31020
   tcgtgaggat ttagaagtga tttcatcgtt ggtgatcgat gaacatcgaa gataacttga 31080
   gtaaagtcta tgatatctta ttacatgtat gtaggttgtg tgacgtggga gatgcgcgag 31140
   tacgggaaac ggaagtgaaa aaattgataa tcccattatg ggagtaatag gggaggcaaa 31200
   aggtcgaggg agtgatgtgt aaatttgcaa aactccatct cgcgctggtg gccacgtcgg 31260
   ccacagggaa ttattcaagc tgggcaaggg ttaattgccg ccttcatcaa accacctctc 31320
   ttcctctttc gaaccagccc tagacaacac cgtgacatcg tctttccgta ttttcgatcc 31380
   gccggaacga gaaatgttct gcactcgata aacgatacat atatatatat gattcttcct 31440
   atataagata aggaaaaaat tgatataaaa atcacgacga ttccatcctt ctatcgttta 31500
   ttcgacttgt gtaattactt aaaatcattg actaaaaaat gaccggttgg aggatctctt 31560
   ggtgatccac aaattgagaa ccgatgaaag agccaagtaa ttttaatcca tgataatttt 31620
   tttccttttt ttcttttgtt taagagttat taatattttt ttaaggagaa gattcccata 31680
   gaattcccgt agagggacct aacataagat tcccataaga tggctcgtta catggtattt 31740
   ttctcggttc gcagaggcaa ttcacgctta tcctgcaagc gagagacgag gcgagcacag 31800
   gcgtgatcga ggaagcgagt ttcagcggca ttgtcctgcc aggacccacg tggcacactc 31860
   tcaatcatga agggagaaac gctcatctgg cttatcgcgt tcgggtccag tgcgcggatc 31920
   attattacaa cgcgacctgc acgaagttct gcaggccaag gaacgacata ttcggccatt 31980
   acacctgcga cgagaacggc gacaaggtgt gcatacaggg ttggaaaggc gccgactgtg 32040
   agataggtag gttgaacttt tgaaaaaatt tcatccaacc cttttgttat ctttacgcga 32100
   tggatacaaa atatttttgc cattccgtaa gaggaacgta cgtgaaattc ttcatctata 32160
   tgttggaaat attgtatatc aatattgtgt attaaatgta aatcagtgag aggattggtg 32220
   agatgatatt ttcaatgtgt ccacgatgtg tgatattctc tacatcacag tttagaacgt 32280
   gtattaagaa tttaaaaatt tgtgctctta aatacacgtg tatttcgtgg gaatcacgaa 32340
   aaattccttc gtgccacctc tcctcttatc atgttcttct aatgcagaat aatgtaaaat 32400
   ttccaaatgg aaaattgtgt ttgaattttg tatcaaaaga atatataatt agggagaaaa 32460
   gtgacgaaac tttttctcga gattatcgta acgtaagcgg aaatttttta ccaaatttta 32520
   taattttacc attagaagaa tatataatct gaaagaagac gtgaaaatct ttttggaatt 32580
   tgaattattc atccgtttta tatttatttt ttaaacgtag agcttatgct gtcgtatatg 32640
   gaaaaatgta attccttttg cccataagtt agacaaggaa agtaaataaa tagaaaattt 32700
   tagtaccggt ggcgccatct cgcgtttgga cgctggaagc tccgacttgc tactagcata 32760
   gcagaatgtg cgaggaggta ttcgtgacgt catcaaagag aaagagaaag caacattgcg 32820
   cgaaaaggac agaggatacg tgccacttat tttggttata cggctcctaa tacttcgcca 32880
   cctcttcgaa ctattaatac atcgataatt aatcaataat taatttttta tcgaaaatat 32940
   ttacactttt aagctcgcat cttgcagttg taacctaaaa aaaatttctt ccgttaattt 33000
   taacgtaaaa tttttatcaa ttgattattt attaacgatt aaaccgtcga atcgaatcat 33060
   cgatgccatt attatttaac gccgcaatat tctttaattt tcatcaattc ctcgttatca 33120
   tgggatctat ctctaaatat taatacgcga ttctgaaacg ttatttcctc tcctactttc 33180
   acgcgtacaa ttttcactcg caacacgttt ttcttttcta attcaactgc gaatcctatt 33240
   tcgaaataga ttcgtcgaca ttatcgacga taattcctac cttatgtaac aaaatctcga 33300
   agaaatataa gagaatctcg aaacgaagaa attggaaaat ggaatttcga aatgattgaa 33360
   actacccctg tatataaatt tatccttaaa tcatcctaag tcaccggaag cattaagaaa 33420
   accgagacaa agaacgcgtt cgcggcttaa tcgagcggaa cgatacacga atgaccaaaa 33480
   agaaggaggg ggggataagg atggaaacga aaagaaacgc gttacgatat atccgtaaaa 33540
   attagtagtc acggcaaaaa gcgtgaaacg gcgtggtaac acgcttttgt cgtcgacgag 33600
   atcttgttac gtatgcaacg aagcatcgcg caaggccttt tgccaggata tcgccggtta 33660
   aacgattcca ccgtgttttc ttttttcgct tgctccctcc ctccctccca tcccctgcct 33720
   ccgccggccg agggaataat taatcgatcc tcgaagcgtg aaatctcgcg tcgcaagcga 33780
   acgcgcgatc cgcttttctc acgaactcac gctgactcct cctcgtttaa tgagccgatc 33840
   gatcgcctcg atcgactcga attttcaacg gtgatacgct ctctcccgtt ttttctctct 33900
   ctctctctct ctctctctct ctctctctct ctcccgagaa aaagggatat tcgattccct 33960
   gaaggcaaga agaggtgatt cgagatcgat ggaaattttt tgctttacat tcaaattgaa 34020
   cgaatttttg tcccgtcggt cgcgagaaaa ataattagac gtcgtatcga ggaaaacaaa 34080
   gtgtgcctga tacacggtta taaatcttgt ccgatagagg agaagataag atcgattgta 34140
   taagtgagtt ttccaatgtc aagctgcaga aaatcacaga agagaagggg aaatgtcgga 34200
   tgttcgtttt cttacgtaaa atttatatca cattgtggat aattgcatcc tgaaataaac 34260
   aagtcttcct tcaagatttc tcaaagattt ccaatggcaa ttgtaaattt atccttcgag 34320
   ttagggattt aaaaagagga aacgaatctg tcgaagatct agaaatcttg tagaaaccct 34380
   tccaaatttt ctcccacccg agattttcga aattagagag aggagagatg aaaacagttg 34440
   caacggttta gaaatttgtc aagaaagttt gctttcgttc gggttaattt caacgattcg 34500
   taagtgtaat cattggaaga ggaaaactcg ttccacgtgt gtttcgtttc tgttgtcgga 34560
   cggaaaccgc gagtctcgcg cacgtctgat tggcaaatac gcaaacacag tgaaacacac 34620
   gaattatctc gaagcgtgcc tcaattggcg gacgttagac gcatgtcgta cgatcgtgca 34680
   taaaggtagc gagcagcatc acgccagatc ccctcgcaca atgctgcccg gattaatacg 34740
   tcagctcttt cttcgctgct taatcgttac ttccgtctcc cccgtgtgtg ccgcttatcc 34800
   gacccgcgct tatcgtctta tccctccctc cagaacattt tctttttttt tttttttttt 34860
   taagggatcg tcttaaatca tcccaaatcc gaagagatta tctttgtcga ggagaaagta 34920
   cggttcttcg acgatcgatt aaacgatatc gatatttcac gttgtgtcga gtcgatataa 34980
   ccgaaatcac aacattttaa tccgatttta tagtttagtg taggaatttt ttgaatttta 35040
   gaaaaataat aatacgtctc gagtgtatta aatttaagaa attaagtttg tggaagaatt 35100
   gtgctgcatt taatatatta aatatgtcgt aagagataat taatcgatat gtataaacaa 35160
   ttttacaatg ttataacatt tcaaacggag atataatttt gtttttctat gagaaaaatg 35220
   aatcgtgaga actcacgttc atgcatgatt aaataacaca gatcaaagat taattggaaa 35280
   ataagggtat aaaaaattat ttatcgagac tttttttttt ttatattaaa tatcgattgt 35340
   ccttgaataa agaattatat cataaaaact tagtttatcg tataaaatat atacgattta 35400
   aacgtgttta cttacaaatc gctatgctcg cgtttcgtat gcaaatatct tcgattggtt 35460
   aattatcgtg attcgaacgt gatcgagata tcaacgtagg cctggtttaa atttcgaaag 35520
   aatattaaat tgtgctcgaa tagttgaaaa ctggccagag aaagtaggac gagcaaacta 35580
   ttagactttt ctgcggtgat agtaattaga agccacggtt gatatcgtgt gaaactgaac 35640
   actgtgtttc tcgaattgtt tcgagggtat aataataata ataagaagaa atgtgacgaa 35700
   acgcgcgtcg aatttaaatc ccttgaattg ctccctgatt taagttccgc ttggaaaacg 35760
   gtggccgcca tcactcgagg acgaagccag tttcccttga cactgtgaac tttaagattt 35820
   tcttcgtttc gcttcgcgtt taatgctcgt ttaatatctt cttcttatcg acaataccat 35880
   cgatgttcat cagactaaga tacacgttga ttgatattaa agatttttca ggatgacttg 35940
   tacacgatgt tcgaaaaata caaagctttg caaaaacttt catttttgtt tattattaac 36000
   aagaaacgtt tcgaccgatc acggaatgta taacagtcga gcgacggtag tgttgggtat 36060
   gtacggcaat cgagcacaaa caagcaaaat gtgtcgaata aaattaatta ttcatcgctc 36120
   ttttctttct cccttcgtct taatataagt aatactttca attctgtcaa tttcaagaat 36180
   tataactcag atctataaat ccaaatctta tgaaacggaa gatacgttga aattaagaaa 36240
   ataaaaaagg ttccaaaatc cgtaaaaaat atatatataa aataatctac aaggaaatct 36300
   acaaagacgt taccttcctc tcctatatac agacgtataa atatattcac acggggaatg 36360
   tgttggaaag aaacgaaccg ggtcgcatcc ccttcgaatt attccggcgg tttaacgatg 36420
   cagcaggatc atattatcgg tatcacaatt agacacgttc cactttcgtc gaacgtggag 36480
   ggaagggggg aaaaaaggaa aggacaaacg atcccaatta gggactatcg gtggaaatcg 36540
   tgtactcgtg agacgacgag accggtcggt cggttacgat gagatgagaa gagaggatga 36600
   gagagagaga gagagggata gaggagagaa ctggctccag catttttgtc gcgctgtgaa 36660
   aggtcaatac gagaatttca ctcgtgcatc gtcttcacac gagttctatg caccgaccgc 36720
   atcatgcaac acaccgttcc tcctccgaga gtacaacgta gtatttctaa actcgtagcg 36780
   agaacgttcg tttgcatttg accccttttc accgtggaga tcgtaaaatc gttcgagatg 36840
   ctcttttacc tcaggattgg aaggattgga aaaggaaggc cgcatttcaa ataacacggt 36900
   atcaagatcg cgtgaaatta aaatcacgat cgaataaatc cttcccatcc gtatcaacca 36960
   ttattatttt ggaaggattt tggtgtaatc tattattatt tattattata cgttacatcc 37020
   acgaaagaaa tttatttgta cggatgtaga gagagataaa aaaaaataat tatccgagaa 37080
   aaaggttgga gtttgatgaa tgatagattt attggtaatg gcgcttaaga taaacgttag 37140
   ccattctacg gaaggattat cggcgctggt ggcaaaggta aaacggttgg ggatcgtgaa 37200
   agtcgtgaac cttcaacctg taatccgatt cttctcgcat ctgttccgtt caccagatcg 37260
   ggggatttat aagaaaataa aagaaatcgc gacggttgtg aacgaagatg aatcgcgcag 37320
   gttgattcgc ctactggcgg cggattaaca aaaaaaattc catagacgtg taactgtaga 37380
   tgttcccggc tagatttata gatcctgaac aacgaaagaa cggtactgta cgagaagcgt 37440
   tgttaatgtg tcggggaagg gggggggggg ggggttccta cacgcgaaac gtaagtacct 37500
   cgtgagattt gccgcggtga aacctctgtt aattgctttt cgcacgaccg gcacgccgaa 37560
   attcgacaag aaatttcaca attcttaatt aacacgtaac cgttcgacgg agaaatccgg 37620
   tcactaaagt actattctta cacgatacca ttaaaaagtg aaatatcttt tccatacgga 37680
   acttacgatt ttcttaaaat taatataaat tgaaagaaaa aaattagttt tccaagaaac 37740
   cgatatttcc agaaactttt aatctcaatt gcaatatatt gattcttcgt gatcaaatac 37800
   taacgaacct tcaaacaata aagtacaaca aacgtattta aatttatttc tcgttcttat 37860
   cgaccgagca aggaatcttg taataggcgg gttgcctact cacaggcagc gaaaacttcg 37920
   cgtttttaac aacagattat caacgattaa aatttcccat tcgcgataac tattcgtact 37980
   tttcgcaatc gattatgaaa cggaaagcca ttttccaata aaattcatta gatcatccat 38040
   aaaactcgtc aacgagaatc gttcgcgcca aggcaaagcc aagttattcg tcgcacgttt 38100
   cgattcgctc gattccacga gcaaaagagg gtaattggca gattaacaca tcctcaatat 38160
   cttcattcga cgaattcgaa ttcgaataat ccctaaacga agcgagaatc gtcggtcgcg 38220
   tgagatcctg ttccccgcgc ccacgcgcac ctgttgcttc gtcgatcagg aaaaagaggg 38280
   atggttgagg gatggtgaat tcacgagtga ttcatctaat tcaccgagca gactctcgag 38340
   cttctctctt ccgatttccg cgagggaccg taattgcgag ggaaatcctc gcgttccccg 38400
   tctttcacgc aataattgga aatcagccaa ttcccccctt ctcaatcgtg gaatgtggat 38460
   ggaagaaaac gagtggatcg atcattatta ccgtatggat tcgacgaaca tcacgccgca 38520
   gtctcataaa tatccgtaaa acgtgttggt cgtccacgcg agaacgagag agctcggcca 38580
   ttaatcatta aagaatccac aaggatgcaa ggggtcgaga gtccttcgag gagagtttac 38640
   tcctgtctcg tgtggagatg catattgcgt atgttaaaat atgcaattac gcgtcggtgt 38700
   acaccgtgcg gttaaggttg aaaaatagtc gacgcggaat agaagcgtag aagtgatcca 38760
   tagatttaaa cggacacccg attgacagag ggagtttacc gatcgagacg atgtgtgaaa 38820
   aagtgtttcg agaaggttcg attcttgcat gaattcaata tgttcgttcg tacactatta 38880
   atttaattgt gtatatctga cgtttcaatg aatcttatta ataatcttat accggtttac 38940
   cgatgacgtt ggcaataaag taattaatac cgatacgaat cgctgtccaa agtattccaa 39000
   agaatctatt cctcatccga tgatttttcc aaaccttaat cgactatatc gaaacgagct 39060
   gagattactc gtatatattc tatactcccg tagaatgtgt gtataccaac gtggagaatc 39120
   tacgtggagc cgtggtgtaa cacgcgattc cttcggttct ttggggccac gatgtgtaac 39180
   ggtaaagaga cggctaaaag tcgcagccct cccctgtaca cgtgtatgcg tgtgcgtata 39240
   tatatatgtg cacgcacgcg tgtgtcgccg cggcgtgagt tttcgtttca cggtcaaaag 39300
   gataatcctt ctctctgtca cttctcatcc gtctttctgt cccaccggct aattgagttg 39360
   ctccggaccg cggccacgga tctctgtcac gtttcatttt ctcccgtctc accgtttcac 39420
   cgtcacacaa tcgaccacga aactgtcgtt tccagggata cgatacgtat atatatacac 39480
   acaccttttc tctccatttc gatcatggac gaaattggat ttcgtcatcc ttagccacct 39540
   gtgtaagaag aaggcgaact tgcgttacct gttaacccgt ttctcctctt cttctccgtg 39600
   tattctctat ttttctcttt ctcgctcact ttgtcatcac acgaaggata cgccaatggt 39660
   ttccccaaga cgggacgaga cgagacgaga tatatgaaac gtcgttgatc gtataatccg 39720
   tccgggtgaa cgtggaaaat aatccgacac ttatgctttc gtggccgtgg gcggattaat 39780
   cgtttgtgtt ttaacttcca ttcacggggg ccttttgtgc cgcgttacgg agcgccacgt 39840
   ttattgttcg ccccggcgag attattcctt tggaaagtcg cttgaattat ggccgatggt 39900
   ggctcttctt ctttctacgt tattggggag atccggtata tatatgtaca cacggtggaa 39960
   agaacggatg aacggcgaat aaaatggccc gcaaccattg gtggttgcgg ttcgcggatt 40020
   gtaactcgaa gatacgcgat ctttattgta aaacgggcct gcgaacgaaa agggaggaat 40080
   acgcgttttt cgtacgtatg tataacgtcg gatataagag gaggtagatt aattacctgc 40140
   agtcggtaca gtggtttaaa aggtgtttaa ttttatacgt cttatctaag tggtttcggt 40200
   tattccggta agaggttgtt aaaggataaa ataagtctaa acaatttata acacgtctcg 40260
   ctttttatgt ctttctagcg ttaaaagccg gaattgaaaa tcgtcgttta tttcgttcac 40320
   gtctgaccat gacgtggcgc cgtcaacatt ttttaacaaa aatgtaatta gctacaacga 40380
   actttacaat gactcaacgt ttacactcgt attaacgtaa ttcttttttt cttttttcaa 40440
   cttcgtgaac ctcctcggtt ctcgctagca aacacgaaaa tttggtatat tgttaaaaat 40500
   aattaatatt aagtaaaaga aattcttata atcctgcata tatgtatcat ttttctgaat 40560
   ttatatagtt aaaaaatgct aaaatataca ttttaattta aaacaacaag aacgtaaaat 40620
   ttttcgttat atgtaacaaa ataatttaaa gtagaaaagc aatattgcga tcaaattgta 40680
   aaaatcgtta tactataaac taatggcgta attttcaatt accttatcat tgcttgtgcg 40740
   aataatcctt cctaactgac aggcgcggtt aatctgtttc agcggtgtgc aaggagggtt 40800
   gccaccccgt tcacggtcac tgcaacgtca gtggggaatg caaatgtcga cacggttggc 40860
   gcggtgaact ctgcgaccag tgcaccccct atcctggctg caagcacgga tattgcaacg 40920
   gttccagttg gcagtgtatc tgcgacacga attggggcgg catcctttgc gaccaggacc 40980
   tcaattattg cggcactcac gaaccctgtc aaaacggtgg gacgtgcgag aacacggcgc 41040
   cggaccagta caaatgcacc tgccccgagg gattctcggg accgacctgc gagaaggtgg 41100
   acaacccttg cgcctccaac ccttgcgcca acggagcgac gtgcagggag ctcggtgaga 41160
   gcgcgcactg cgagtgcgcc cctggattct ctggccctta ctgcgcgacg gacatagacg 41220
   agtgcgcctc ccaaccttgc caaaatgggg gcacctgcgt cgacgggaag aacgagtttg 41280
   tgtgcaattg tccaccggcc tggcaaggag tgctttgcca attcgacgtg gatgagtgcg 41340
   ccctcaagga atccccctgc aagaattcgt tgacctgtgt gaatcttgcc ggtgattaca 41400
   agtgaggaag aaaaaaattt gttttattat ttgacctgaa aatttagtcc atcggtggat 41460
   tatggaatgg attttttttt ctttaattaa aaaatctcgg cctcgtttca ggtgtcggtg 41520
   caggagcggt ttcacgggga agaactgtac gaagaacatc aacgattgca tcggccagtg 41580
   ccaacacggg gcgttgtgca tcgatctggt cgacgattat cactgcagct gcaccgcagg 41640
   ctattcgggc aaggattgcg acgtggacat cgacgagtgc gcctcgaaac cctgccagaa 41700
   cggtggcgag tgcagagatc tggtgaacgc gtacgagtgc gtatgtcctg tcggtttcac 41760
   cggataccag tgcgagatcg acaaggatca ttgcagcccg aacccgtgca gaaattcggc 41820
   gccttgtttc aacacgcaga cagattacta ctgccactgt ccggagcaat ggcaagggaa 41880
   gaattgttcc gagccagcct cccataatcc gcaattcggc atgatggacg aggagtccgg 41940
   gtgcgggagt gaggggacac catgcggcgg cagaggcaga tgcagcggtg gcagatgcat 42000
   ttgtgacgcc gggtatacag ggatgcattg tcacgagaac atcaacgatt gcaggggtaa 42060
   tccttgtttg aacggtggca cgtgtgtcga tttggttaac tcgttccagt gtatctgtag 42120
   agagggatgg accggagatc tgtgcgatca aggtgagcga ttgtttcaga gttcgtgttc 42180
   ttaatttgtt tcgccgtcgc gttaaatgcg atgaaaaaat tgtcacgaaa ggaagaaatg 42240
   atatctctaa taattctaac gatgcttctt tttttttttt tttttttaaa ttattcctta 42300
   tcgttccttc agacgtggac gagtgtacga actcaccctg ccgcaacaac ggcacctgcg 42360
   tggacggtgt cgcagacttc acctgcatct gcaggggtgg atggaagggc aaaacgtgta 42420
   ctcttcgcgc cggacactgc gaaccaggta cctgtcgcca cggtggaact tgccaagatc 42480
   gcggagatgg attcacttgt cactgtccac caggttggga gggagcagca tgccacatag 42540
   catctccagc gtgcgcgagc aatccttgcg agaacggagc tacctgcgtg aacaccgccg 42600
   atgggaatta cagatgcgtc tgtcgcgaag gtttcgaggg tccgaattgc cgtcgtgacg 42660
   tggacgattg ccagccattg ccttgcttga acgggggcaa atgcgtggac gggatcaact 42720
   ggttcagatg cgagtgcgcg ccaggcttca ccggcccaga ttgtcgtata aacgtgaacg 42780
   aatgcgccag cgacccctgc accactggct ccacgtgcgt ggacggtata gcgagttaca 42840
   cttgcatctg cccgccagga aggaccgggg tacgctgcga gattcgtaca gcaggtggac 42900
   caggatgccc ggtagctagc tgggacgacg attgcaacgt ctgcgaatgt cgtaacggga 42960
   agaaccagtg cagcaatatc tggtgcggtc ctggcaactg tctgaacggt acgtcgtgtc 43020
   tggctcacga agtgtgcgtg cccagtccga acgaaagctg tctagctcca ccgtgtccag 43080
   cgtggggcga atgccgcccc gtcgagactg gaagaagagt agggcctccc gctctgcccg 43140
   caccgccctc ctgttggccc ggacaagcca cgccgggtcc aacctgttcc aggctcacca 43200
   tcctgttgag aagagacact ctggccgcca gtacgtcggt agaaatcctc tgcagacggt 43260
   tgagaaaact gctgtccgat ccgagaaggc cacagtcgat cgttcttctc tgcgatctca 43320
   agcctggcga caacgataca gtggaagtga ccatattctc ggaggcagcc gcagatgcgg 43380
   ccagagatct tggcgaagcc cttagcagac ccctgggcag accactcgct ctggcctccg 43440
   tgctcgaggt caaagtggag acggccttgc tcagcgagcc gagttccgta aactcgagca 43500
   gcgcgggaag ctacgtggcc gtcctcggtg gagctctggc gaccgttttg gtcttggctt 43560
   tgttcggtgg actgtggtat cttcggacgg tgaggcaaag atcaagcctg accgcgacca 43620
   ccagcagcga gacgtcgttg catcgacacc gcagcgattt ggacgagaag tcgaacaatc 43680
   ttcaaaacga ggagaatctg aggagatacg cgaacccttt gaaagagcag gatcaaggag 43740
   agccgagggt gtccgtggtg agacctctgt ccggaacttc gttgggtgcg ctgggtgcaa 43800
   ccgaggagag tttggaaatg gtctcggacg agagtagaca tcggttgccg ccgctttaca 43860
   aagctcccag cgcggaagca aggaacaaca ccgctagttt cacgtacgag gaagggcctc 43920
   ataaaccgta cagcaaacct agattgcagg agccgacgta ttctcagcaa gcgagcagca 43980
   gtcagacatc gggaccgcat caagtactga cggtgcacgt gtaacgcgac agacttgcgg 44040
   atgtttcgtg aaaaatttca ctgaaaaaaa aaaaaaaaaa cacttgtgag aaagggacac 44100
   gaaaaacgtg tggcgaaacg tgtggaatta tcggactcgc tcgtaccaag tgttttcgca 44160
   acgattaatt gcaacgaaca gactgaacaa ttaataattc acgattcaac ggatatcgct 44220
   tacattactt tagaaaatga aattgcgaga aataatgcgc tgcctttttt tacccgctaa 44280
   aaattaagaa atcgtcgaaa ccgcgtcttt tacttcaaat cttttttcct tctgcgaatt 44340
   gatacgaaaa caagagagat attaaagaat gtaggaaggt agagaaagag aagaattatt 44400
   ttaacgttat ttcatcaact atattgatat tggtttacaa ttaaaaatcg atcatttcaa 44460
   actatttcat taaacgagaa aaacgtcatt ctctaccttt ctctaacata tctctcgcta 44520
   ataacgcaac gacaataatt gacggtctac aattatatat cgacaagttg ttttatcgta 44580
   ttgtacatat gtacatagcc cgtacttatt aatttcgcta agcctgtatc acctctaaat 44640
   gccgtcgtcc cttacggaca gccttaagat ccgatctatc gacgttttat tcgcgttaca 44700
   atcgtaaaca ttcccctatc ctgatccgaa gcagagaaca cacgggccaa gtgacggctt 44760
   agcaggccac cgtagatatc gtagagctgt aatagcacgt aggcgaatta atctgcgcga 44820
   aagcagaagg cttcggacgc tccgtcgggc gatattattg tgatactcat agagcgaacg 44880
   agagagaaag aatgcgagag agaatattat cgcgcgataa gatttatcga gtcccctcgt 44940
   agcgccctct tggaaaagtc gtgaatcacg attaataacg ataacgcgtt aagaaaagaa 45000
   aagtcttaaa atttggtttg ctaactagtt tgcaacggtt tgcatccgtt tgccgtagta 45060
   atcagtagaa ttaacgcacg gttctatcat tgcgagccgt gatttaattg cacaaaaatt 45120
   aattaattat tcatgggatg tatcgtcgta tccacgagtc gatctataat gaaaaggaag 45180
   tattcttttt tttttttttt tatttttcct gcaaagaggg tgaagggagc gtgtaggaat 45240
   cgataagatg gcgacacatg gcatggccat tcgcgtgatc gaaacgttgt atgatgtaaa 45300
   taaacccatt acatagatcc agataattcc ccgtcccact ttggattgta ttttatctta 45360
   ttccctcctt tcttccctct gttagatgtg caattttatt attatttttt tttccttttt 45420
   tttttttttt ctctcccctt ttcccttaat atcgttccaa gcgtaaggcg acgcgcaacg 45480
   acgggcgcat gaaacgcgcg tattactaga ctttaaaacg agaaacgaga accgtattgg 45540
   gcgtactgta agatgataac ggaagaaatt ttttataaca gatagtttat tttataaaga 45600
   aatctatatt attatacaaa atatatagaa gcgatgaaga aaaaaaaata tatatatata 45660
   tatattatat tattttatat tatattatat tattttttat attttattgc cttgtatttt 45720
   aattgttgag agatcatgga caatagttcg aaatactata ctatactata tacgatcgat 45780
   cataacgatc tcgtgaacat ctttttgttc tgttcttcga ttcaacgaac taatgtccct 45840
   ggtctccgat ttagatagct aatccacgcg ttgtgatcaa tcccgtgtca aataatcgcg 45900
   aaggaagcgt gaatgagtgc gtgtaaataa agttaaagct ccttccggtc ccacaatttc 45960
   tttttttccg cctaagttta caacaacgac taggacgacg acgatgacta taacaacgac 46020
   aacgacataa tgatgatgat gatgatgatg atgatgatga tgatgaagaa atttcaataa 46080
   aaagtacaac ccttatgaca ccgtttcgtt tttacttctg gtttattgca cccgaacagt 46140
   aacaggccga acgaattctc tcacgtggtg agagcccgag tcgatgcccg cgcaagttgc 46200
   attcaaatca ccgggaacgc agaatcgtag gtagacgtgc cttccgggag caactggcac 46260
   agtccaacga tgagaaacgg aacgagcaac gctagattga atacattttt atttaaatgg 46320
   taaataatcg tataaaataa tgataataag tacggaattt taacgattat tttaattatt 46380
   ttattttatt catttgcgtg cgccagtcgc aaaggtatca aaatttattt agagaaatca 46440
   tgattgtaag aaaaaaggat aaaatttatc attaattgta aaattaatga agagatattt 46500
   c 46501
<210> 3
   <211> 1917
   <212> PRT
   <213> Apis mellifera
<400> 3
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
               20                  25                  30
   Phe Ile Lys Thr Glu Gly Lys Cys Lys Thr Phe Ser Tyr Gly Gly Cys
           35                  40                  45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
   275 280 285
   Ile Ile Glu Ser Phe Val Gly Cys Ile Arg Asp Met Val Leu Ser Ser
   290 295 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
   325 330 335
   Glu Asn Leu Cys Phe Ile Ser Ser Gln Cys Val Asn His Tyr Asn Ser
   340 345 350
   Leu Thr Cys Asp Cys Phe Gly Thr Lys Tyr Glu Gly Glu Arg Cys Asp
   355 360 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385 390 395 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                 1005
   Glu Lys  Ala Ile Leu Leu Tyr  Gln Pro Pro Ile Arg  Asn Asn Tyr
       1010                 1015                 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
       1730                1735                1740
   Gln Ser Gln Gln Tyr Thr Asp Pro Leu Thr Gly Gly Val Lys Thr
       1745                1750                1755
   Val Gly Phe Lys Asn Val Ala Glu Asp Glu Lys Arg Pro Asn Gly
       1760                1765                1770
   Thr His Met Lys Ala Ala Val Lys Glu Tyr Lys Pro Leu Ala Asn
       1775                1780                1785
   Ala Glu Ser Lys Glu Leu Ile Asn Asp Lys Arg Val His Ile Ile
       1790                1795                1800
   Ala Asp Glu Glu Pro Glu Lys Lys Glu Leu Leu Gly Val Asn Thr
       1805                1810                1815
   Gly Ile Ile Thr Lys Ser Pro Lys Leu Asn Pro Phe Ser Met Glu
       1820                1825                1830
   Asp Leu Gln Glu Glu Pro Glu Leu Glu Glu Cys Glu Glu Glu Glu
       1835                1840                1845
   Pro Asp Glu Glu Asp Glu Asp Glu Asp Glu Asp Asn Pro Lys Glu
       1850                1855                1860
   Gln Asn Thr Asp Glu Glu Glu Ile His Gln Lys Thr Glu Arg Thr
       1865                1870                1875
   Asp Asp Asn Asp Phe Val Lys Ser Pro Glu Asn Ser Ser Arg Ile
       1880                1885                1890
   Ser Val Glu Glu Ser Val Asp Ser Leu Asn Asn Asn Pro Lys His
       1895                1900                1905
   Asp Glu Thr Lys Thr Gln Tyr Glu Val
       1910                1915
<210> 4
   <211> 1203
   <212> PRT
   <213> Apis mellifera
<400> 4
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Ala Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145                 150                 155                 160
   Leu Ala Tyr Arg Val Arg Val Gln Cys Ala Asp His Tyr Tyr Asn Ala
                   165                 170                 175
   Thr Cys Thr Lys Phe Cys Arg Pro Arg Asn Asp Ile Phe Gly His Tyr
               180                 185                 190
   Thr Cys Asp Glu Asn Gly Asp Lys Val Cys Ile Gln Gly Trp Lys Gly
           195                 200                 205
   Ala Asp Cys Glu Ile Ala Val Cys Lys Glu Gly Cys His Pro Val His
       210                 215                 220
   Gly His Cys Asn Val Ser Gly Glu Cys Lys Cys Arg His Gly Trp Arg
   225                 230                 235                 240
   Gly Glu Leu Cys Asp Gln Cys Thr Pro Tyr Pro Gly Cys Lys His Gly
                   245                 250                 255
   Tyr Cys Asn Gly Ser Ser Trp Gln Cys Ile Cys Asp Thr Asn Trp Gly
               260                 265                 270
   Gly Ile Leu Cys Asp Gln Asp Leu Asn Tyr Cys Gly Thr His Glu Pro
           275                 280                 285
   Cys Gln Asn Gly Gly Thr Cys Glu Asn Thr Ala Pro Asp Gln Tyr Lys
       290                 295                 300
   Cys Thr Cys Pro Glu Gly Phe Ser Gly Pro Thr Cys Glu Lys Val Asp
   305                 310                 315                 320
   Asn Pro Cys Ala Ser Asn Pro Cys Ala Asn Gly Ala Thr Cys Arg Glu
                   325                 330                 335
   Leu Gly Glu Ser Ala His Cys Glu Cys Ala Pro Gly Phe Ser Gly Pro
               340                 345                 350
   Tyr Cys Ala Thr Asp Ile Asp Glu Cys Ala Ser Gln Pro Cys Gln Asn
           355                 360                 365
   Gly Gly Thr Cys Val Asp Gly Lys Asn Glu Phe Val Cys Asn Cys Pro
       370                 375                 380
   Pro Ala Trp Gln Gly Val Leu Cys Gln Phe Asp Val Asp Glu Cys Ala
   385                 390                 395                 400
   Leu Lys Glu Ser Pro Cys Lys Asn Ser Leu Thr Cys Val Asn Leu Ala
                   405                 410                 415
   Gly Asp Tyr Lys Cys Arg Cys Arg Ser Gly Phe Thr Gly Lys Asn Cys
               420                 425                 430
   Thr Lys Asn Ile Asn Asp Cys Ile Gly Gln Cys Gln His Gly Ala Leu
           435                 440                 445
   Cys Ile Asp Leu Val Asp Asp Tyr His Cys Ser Cys Thr Ala Gly Tyr
       450                 455                 460
   Ser Gly Lys Asp Cys Asp Val Asp Ile Asp Glu Cys Ala Ser Lys Pro
   465                 470                 475                 480
   Cys Gln Asn Gly Gly Glu Cys Arg Asp Leu Val Asn Ala Tyr Glu Cys
                   485                 490                 495
   Val Cys Pro Val Gly Phe Thr Gly Tyr Gln Cys Glu Ile Asp Lys Asp
               500                 505                 510
   His Cys Ser Pro Asn Pro Cys Arg Asn Ser Ala Pro Cys Phe Asn Thr
           515                 520                 525
   Gln Thr Asp Tyr Tyr Cys His Cys Pro Glu Gln Trp Gln Gly Lys Asn
       530                 535                 540
   Cys Ser Glu Pro Ala Ser His Asn Pro Gln Phe Gly Met Met Asp Glu
   545                 550                 555                 560
   Glu Ser Gly Cys Gly Ser Glu Gly Thr Pro Cys Gly Gly Arg Gly Arg
                   565                 570                 575
   Cys Ser Gly Gly Arg Cys Ile Cys Asp Ala Gly Tyr Thr Gly Met His
               580                 585                 590
   Cys His Glu Asn Ile Asn Asp Cys Arg Gly Asn Pro Cys Leu Asn Gly
           595                 600                 605
   Gly Thr Cys Val Asp Leu Val Asn Ser Phe Gln Cys Ile Cys Arg Glu
       610                 615                 620
   Gly Trp Thr Gly Asp Leu Cys Asp Gln Asp Val Asp Glu Cys Thr Asn
   625                 630                 635                 640
   Ser Pro Cys Arg Asn Asn Gly Thr Cys Val Asp Gly Val Ala Asp Phe
                   645                 650                 655
   Thr Cys Ile Cys Arg Gly Gly Trp Lys Gly Lys Thr Cys Thr Leu Arg
               660                 665                 670
   Ala Gly His Cys Glu Pro Gly Thr Cys Arg His Gly Gly Thr Cys Gln
           675                 680                 685
   Asp Arg Gly Asp Gly Phe Thr Cys His Cys Pro Pro Gly Trp Glu Gly
       690                 695                 700
   Ala Ala Cys His Ile Ala Ser Pro Ala Cys Ala Ser Asn Pro Cys Glu
   705                 710                 715                 720
   Asn Gly Ala Thr Cys Val Asn Thr Ala Asp Gly Asn Tyr Arg Cys Val
                   725                 730                 735
   Cys Arg Glu Gly Phe Glu Gly Pro Asn Cys Arg Arg Asp Val Asp Asp
               740                 745                 750
   Cys Gln Pro Leu Pro Cys Leu Asn Gly Gly Lys Cys Val Asp Gly Ile
           755                 760                 765
   Asn Trp Phe Arg Cys Glu Cys Ala Pro Gly Phe Thr Gly Pro Asp Cys
       770                 775                 780
   Arg Ile Asn Val Asn Glu Cys Ala Ser Asp Pro Cys Thr Thr Gly Ser
   785                 790                 795                 800
   Thr Cys Val Asp Gly Ile Ala Ser Tyr Thr Cys Ile Cys Pro Pro Gly
                   805                 810                 815
   Arg Thr Gly Val Arg Cys Glu Ile Arg Thr Ala Gly Gly Pro Gly Cys
               820                 825                 830
   Pro Val Ala Ser Trp Asp Asp Asp Cys Asn Val Cys Glu Cys Arg Asn
           835                 840                 845
   Gly Lys Asn Gln Cys Ser Asn Ile Trp Cys Gly Pro Gly Asn Cys Leu
       850                 855                 860
   Asn Gly Thr Ser Cys Leu Ala His Glu Val Cys Val Pro Ser Pro Asn
   865                 870                 875                 880
   Glu Ser Cys Leu Ala Pro Pro Cys Pro Ala Trp Gly Glu Cys Arg Pro
                   885                 890                 895
   Val Glu Thr Gly Arg Arg Val Gly Pro Pro Ala Leu Pro Ala Pro Pro
               900                 905                 910
   Ser Cys Trp Pro Gly Gln Ala Thr Pro Gly Pro Thr Cys Ser Arg Leu
           915                 920                 925
   Thr Ile Leu Leu Arg Arg Asp Thr Leu Ala Ala Ser Thr Ser Val Glu
       930                 935                 940
   Ile Leu Cys Arg Arg Leu Arg Lys Leu Leu Ser Asp Pro Arg Arg Pro
   945                 950                 955                 960
   Gln Ser Ile Val Leu Leu Cys Asp Leu Lys Pro Gly Asp Asn Asp Thr
                   965                 970                 975
   Val Glu Val Thr Ile Phe Ser Glu Ala Ala Ala Asp Ala Ala Arg Asp
               980                 985                 990
   Leu Gly Glu Ala Leu Ser Arg Pro  Leu Gly Arg Pro Leu  Ala Leu Ala
           995                 1000                1005
   Ser Val  Leu Glu Val Lys Val  Glu Thr Ala Leu Leu  Ser Glu Pro
       1010                1015                1020
   Ser Ser Val Asn Ser Ser Ser Ala Gly Ser Tyr Val Ala Val Leu
   1025 1030 1035
   Gly Gly Ala Leu Ala Thr Val Leu Val Leu Ala Leu Phe Gly Gly
   1040 1045 1050
   Leu Trp Tyr Leu Arg Thr Val Arg Gln Arg Ser Ser Leu Thr Ala
   1055 1060 1065
   Thr Thr Ser Ser Glu Thr Ser Leu His Arg His Arg Ser Asp Leu
   1070 1075 1080
   Asp Glu Lys Ser Asn Asn Leu Gln Asn Glu Glu Asn Leu Arg Arg
   1085 1090 1095
   Tyr Ala Asn Pro Leu Lys Glu Gln Asp Gln Gly Glu Pro Arg Val
   1100 1105 1110
   Ser Val Val Arg Pro Leu Ser Gly Thr Ser Leu Gly Ala Leu Gly
   1115 1120 1125
   Ala Thr Glu Glu Ser Leu Glu Met Val Ser Asp Glu Ser Arg His
   1130 1135 1140
   Arg Leu Pro Pro Leu Tyr Lys Ala Pro Ser Ala Glu Ala Arg Asn
   1145 1150 1155
   Asn Thr Ala Ser Phe Thr Tyr Glu Glu Gly Pro His Lys Pro Tyr
   1160 1165 1170
   Ser Lys Pro Arg Leu Gln Glu Pro Thr Tyr Ser Gln Gln Ala Ser
   1175 1180 1185
   Ser Ser Gln Thr Ser Gly Pro His Gln Val Leu Thr Val His Val
   1190 1195 1200
<210> 5
   <211> 1917
   <212> PRT
   <213> Apis mellifera
<400> 5
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
           35                  40                  45
   Leu Gly Asn Glu Asn Arg Phe Asn Ser Glu Ala Glu Cys Leu Tyr Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
   805 810 815
   Ser Thr Lys Thr Ile Val Glu His Asn Leu Pro Ser Gln Val Asp Val
   820 825 830
   Arg Ser Ile Ala Glu Ser Asp Phe Ser Phe Asn Ile Lys Tyr Arg Gln
   835 840 845
   Phe Thr Ala Glu Met Leu Gln Glu Leu Ile Ser His Ser Leu Tyr Cys
   850 855 860
   Ser Gln Tyr Val Lys Tyr Asp Cys Tyr Lys Ala Pro Leu Glu Leu His
   865 870 875 880
   Ser Ala Thr Trp Phe Leu Asn Ser Lys Gly Thr Thr Val Asp Tyr Ile
   885 890 895
   Gly Asn Val Asn Arg Gly Ser Cys Pro Cys Gly Met Asn Lys Thr Cys
   900 905 910
   Ile Asn Pro Asn Leu Ser Cys Asn Cys Asp Phe Thr Gly Asn Gly Asp
   915 920 925
   Lys Trp Leu Ser Asp Glu Gly Tyr Tyr Glu Thr Pro Asp Ser Leu Gly
   930 935 940
   Ile Thr Gly Met Val Phe Leu Gln Gln Arg Asp Leu Glu Glu Asp Ala
   945 950 955 960
   Gln Gly Arg Ile Thr Leu Gly Pro Leu Glu Cys Val Glu Thr Asn Thr
   965 970 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe  Thr Leu Ser Thr Gly  Val Ile Lys Gly Ile  Glu Val Glu
       1040                 1045                 1050
   Ser Lys  Arg Lys Leu Asn Asn  Gly Glu Trp Gln Lys  Ile Trp Ile
       1055                 1060                 1065
   Asp Tyr  Asn Asn Tyr His Val  Arg Leu Met Ile Asn  Thr Asp Ser
       1070                 1075                 1080
   Gln Met  Leu Asp Leu Glu Asp  Lys Phe Gly Pro Phe  Glu Gly Ser
       1085                 1090                 1095
   Met Phe  Ile Gly Gly Ala Thr  Ala Glu His Leu Arg  Thr Ser Ser
       1100                 1105                 1110
   Val Gln  Gln Gly Leu Ile Gly  Cys Phe Arg Gly Leu  Val Val Asn
       1115                 1120                 1125
   Gly Glu  Ile Leu Asp Ile His  Ser Tyr Met Ser Val  His Leu Ser
       1130                 1135                 1140
   Glu Ile  Ile Lys Asp Cys Lys  Pro Phe Cys Gln Pro  Asn Lys Cys
       1145                 1150                 1155
   Gln Asn  Gly Ala Arg Cys Val  Glu Leu Trp Ser Asn  Tyr Lys Cys
       1160                 1165                 1170
   Val Cys  Glu Asn Lys Trp Ala  His Phe Gly Asp Tyr  Cys Glu Asn
       1175                 1180                 1185
   Asn Ile  Asn Asn Glu Ala Leu  Thr Phe Thr Ser Gln  Ser Ala Phe
       1190                 1195                 1200
   Leu Lys  Lys Asn Tyr Phe Gly  Thr Asp Glu Lys Asp  Glu Glu Lys
       1205                 1210                 1215
   Ser Leu  Leu Lys Asn Ile Leu  Met Lys Asn Ile Leu  Ile Asn Leu
       1220                 1225                 1230
   Arg Thr  Tyr Asp Thr Tyr Ser  Ser Ile Leu Tyr Ala  Asn Asp Asn
       1235                 1240                 1245
   Leu Asn  Asn Phe Ala His Leu  Tyr Ile Ser Asn Gly  Thr Ser Ile
       1250                 1255                 1260
   Val Tyr  Leu Phe Asn Ala Gly  Asn Glu Ile Lys Asn  Ile Thr Val
       1265                 1270                 1275
   Glu Tyr  Pro Val Asn Thr Gly  Ile Ser Ile Gln Ile  Ala Ile Ile
       1280                 1285                 1290
   Arg Asn  Glu Ser Ser Thr Thr  Leu His Val Asn Glu  Tyr Asn Ser
       1295                 1300                 1305
   Thr Leu  Asn Ala Thr Leu Val  Leu Leu Asp Thr Tyr  Ser Asn Lys
       1310                 1315                 1320
   Pro Trp  Ile Asn Pro Glu Lys  Glu Val Leu Ala Pro  Gln Arg Pro
       1325                 1330                 1335
   Pro Ala  Ser Pro Thr Ser Tyr  Phe Gln Val Asn Leu  Gly Gly Phe
       1340                 1345                 1350
   Asp Pro  Asp Asp Leu Leu Arg  Val Gly Lys Glu Asp  Val Gln Ile
       1355                 1360                 1365
   Lys Gly  Tyr Val Gly Cys Leu  Gly Gly Leu Met Ile  Gly Glu Tyr
       1370                 1375                 1380
   Ala Val  Asn Leu Ser Lys Leu  Ala Asn Glu Ala Asn  His Glu Gly
       1385                 1390                 1395
   Asn Lys  Glu Ile Leu Pro Asn  Cys Gln Thr Lys Cys  Asp Ala Met
       1400                 1405                 1410
   Pro Cys  Lys Asn Leu Gly Ile  Cys Thr Glu Asp Phe  Glu Arg Gln
       1415                 1420                 1425
   Glu Ser  Leu Cys Asn Cys Glu  Leu Thr Ser Tyr Phe  Gly Glu His
       1430                 1435                 1440
   Cys Ala  Asp Glu Lys Gly Ala  Asp Phe Ser Gly Glu  Ser Val Val
       1445                 1450                 1455
   Gln Arg  Glu Phe Asp Leu Val  Gly Lys Val Asn Gln  Val Lys Val
       1460                 1465                 1470
   Gln Leu  Ala Phe Ser Thr Asn  Glu Leu Arg Gln Arg  Thr Thr Ala
       1475                 1480                 1485
   Leu Leu  Leu Leu Gln Thr Glu  Asn Lys Arg Ser Tyr  Tyr Leu Leu
       1490                 1495                 1500
   Val Ala  Leu Thr Ser Glu Gly  Gln Leu Ile Phe Glu  Glu Asp Arg
       1505                 1510                 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
       1745                1750                1755
   Val Gly Phe Lys Asn Val Ala Glu Asp Glu Lys Arg Pro Asn Gly
       1760                1765                1770
   Thr His Met Lys Ala Ala Val Lys Glu Tyr Lys Pro Leu Ala Asn
       1775                1780                1785
   Ala Glu Ser Lys Glu Leu Ile Asn Asp Lys Arg Val His Ile Ile
       1790                1795                1800
   Ala Asp Glu Glu Pro Glu Lys Lys Glu Leu Leu Gly Val Asn Thr
       1805                1810                1815
   Gly Ile Ile Thr Lys Ser Pro Lys Leu Asn Pro Phe Ser Met Glu
       1820                1825                1830
   Asp Leu Gln Glu Glu Pro Glu Leu Glu Glu Cys Glu Glu Glu Glu
       1835                1840                1845
   Pro Asp Glu Glu Asp Glu Asp Glu Asp Glu Asp Asn Pro Lys Glu
       1850                1855                1860
   Gln Asn Thr Asp Glu Glu Glu Ile His Gln Lys Thr Glu Arg Thr
       1865                1870                1875
   Asp Asp Asn Asp Phe Val Lys Ser Pro Glu Asn Ser Ser Arg Ile
       1880                1885                1890
   Ser Val Glu Glu Ser Val Asp Ser Leu Asn Asn Asn Pro Lys His
       1895                1900                1905
   Asp Glu Thr Lys Thr Gln Tyr Glu Val
       1910                1915
<210> 6
   <211> 1917
   <212> PRT
   <213> Apis mellifera
<400> 6
   Met Asp Ser Phe Thr Thr Thr Lys Arg Thr Phe Leu Asp Arg Cys Leu
   1               5                   10                  15
   Val Lys Thr Glu Pro Gly Pro Cys Lys Gln Tyr Val Thr Arg Trp Thr
               20                  25                  30
   Phe Ile Lys Thr Glu Gly Lys Cys Lys Thr Phe Ser Tyr Gly Gly Cys
           35                  40                  45
   Leu Gly Asn Glu Asn Arg Phe Asn Ser Glu Ala Glu Cys Leu His Tyr
       50                  55                  60
   Cys Val Gly Val Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                 1005
   Glu Lys  Ala Ile Leu Leu Tyr  Gln Pro Pro Ile Arg  Asn Asn Tyr
       1010                 1015                 1020
   Pro Ser  Phe Met Val Ala Leu  Thr Ser Glu Tyr Arg  Leu Thr Phe
       1025                 1030                 1035
   Asn Phe  Thr Leu Ser Thr Gly  Val Ile Lys Gly Ile  Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly  Ser Ala Phe Gly Val  Arg Leu Asn Asp Ile  Asn Phe Leu
       1520                 1525                 1530
   Asn Gly  Ala Arg His Ser Ile  Tyr Tyr Val Arg Asp  Asn Ser Thr
       1535                 1540                 1545
   Val Thr  Phe Leu Ile Asp Arg  Glu Pro Val Thr Leu  Leu Pro Ile
       1550                 1555                 1560
   Pro Ala  Leu Thr Leu Gly Glu  Asp Glu Ser Pro Gly  Val Thr Glu
       1565                 1570                 1575
   Ile Gln  Leu Gly Gly Leu Asn  Thr Thr Asp Ser Arg  Phe Ser Ala
       1580                 1585                 1590
   Tyr Lys  Gly Tyr Thr Gly Cys  Leu Ser Asn Val Val  Val Ser Ile
       1595                 1600                 1605
   Asn Gly  Gly Pro Asp Met Lys  Pro Leu Glu Glu Tyr  Met Leu Phe
       1610                 1615                 1620
   Thr Lys  Gln Gly Ser Glu Thr  Val Arg Ala Thr Met  Pro Ala Gly
       1625                 1630                 1635
   Val Arg  Ser Ala Gln Cys Ala  Val Phe His Val Gln  Pro His Gly
       1640                 1645                 1650
   Leu Asp  Pro Pro Lys Asn Asp  Ser Asn Val Gly Val  Asp Lys Val
       1655                 1660                 1665
   Trp Val  Glu Asp Pro Pro Glu  Arg Ile Pro Tyr Lys  Ser Gln Tyr
       1670                 1675                 1680
   Thr Asn  Ala Thr Gln Glu Glu  Gln Gly Ala Gly Thr  Tyr Ile Phe
       1685                 1690                 1695
   Ile Ala  Leu Cys Cys Val Phe  Val Thr Ala Val Ile  Gly Cys Ile
       1700                 1705                 1710
   Tyr Glu  Val Trp Arg Ser Ala  Arg Lys Asp Arg Arg  Arg Arg Arg
       1715                 1720                 1725
   Ala Ser  Val Ala Ser Thr Val  Pro Ala Pro Gly Ser  Gln Arg Trp
       1730                 1735                 1740
   Gln Ser  Gln Gln Tyr Thr Asp  Pro Leu Thr Gly Gly  Val Lys Thr
       1745                 1750                 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 7
   <211> 1917
   <212> PRT
   <213> Apis mellifera
<400> 7
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Thr Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
   770 775 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                1005
   Glu Lys  Ala Ile Leu Leu Tyr  Gln Pro Pro Ile Arg  Asn Asn Tyr
       1010                1015                1020
   Pro Ser  Phe Met Val Ala Leu  Thr Ser Glu Tyr Arg  Leu Thr Phe
       1025                1030                1035
   Asn Phe  Thr Leu Ser Thr Gly  Val Ile Lys Gly Ile  Glu Val Glu
       1040                1045                1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly  Ser Ala Phe Gly Val  Arg Leu Asn Asp Ile  Asn Phe Leu
       1520                 1525                 1530
   Asn Gly  Ala Arg His Ser Ile  Tyr Tyr Val Arg Asp  Asn Ser Thr
       1535                 1540                 1545
   Val Thr  Phe Leu Ile Asp Arg  Glu Pro Val Thr Leu  Leu Pro Ile
       1550                 1555                 1560
   Pro Ala  Leu Thr Leu Gly Glu  Asp Glu Ser Pro Gly  Val Thr Glu
       1565                 1570                 1575
   Ile Gln  Leu Gly Gly Leu Asn  Thr Thr Asp Ser Arg  Phe Ser Ala
       1580                 1585                 1590
   Tyr Lys  Gly Tyr Thr Gly Cys  Leu Ser Asn Val Val  Val Ser Ile
       1595                 1600                 1605
   Asn Gly  Gly Pro Asp Met Lys  Pro Leu Glu Glu Tyr  Met Leu Phe
       1610                 1615                 1620
   Thr Lys  Gln Gly Ser Glu Thr  Val Arg Ala Thr Met  Pro Ala Gly
       1625                 1630                 1635
   Val Arg  Ser Ala Gln Cys Ala  Val Phe His Val Gln  Pro His Gly
       1640                 1645                 1650
   Leu Asp  Pro Pro Lys Asn Asp  Ser Asn Val Gly Val  Asp Lys Val
       1655                 1660                 1665
   Trp Val  Glu Asp Pro Pro Glu  Arg Ile Pro Tyr Lys  Ser Gln Tyr
       1670                 1675                 1680
   Thr Asn  Ala Thr Gln Glu Glu  Gln Gly Ala Gly Thr  Tyr Ile Phe
       1685                 1690                 1695
   Ile Ala  Leu Cys Cys Val Phe  Val Thr Ala Val Ile  Gly Cys Ile
       1700                 1705                 1710
   Tyr Glu  Val Trp Arg Ser Ala  Arg Lys Asp Arg Arg  Arg Arg Arg
       1715                 1720                 1725
   Ala Ser  Val Ala Ser Thr Val  Pro Ala Pro Gly Ser  Gln Arg Trp
       1730                 1735                 1740
   Gln Ser  Gln Gln Tyr Thr Asp  Pro Leu Thr Gly Gly  Val Lys Thr
       1745                 1750                 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 8
   <211> 1917
   <212> PRT
   <213> Apis mellifera
<400> 8
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Glu Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
   770 775 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                 1005
   Glu Lys  Ala Ile Leu Leu Tyr  Gln Pro Pro Ile Arg  Asn Asn Tyr
       1010                 1015                 1020
   Pro Ser  Phe Met Val Ala Leu  Thr Ser Glu Tyr Arg  Leu Thr Phe
       1025                 1030                 1035
   Asn Phe  Thr Leu Ser Thr Gly  Val Ile Lys Gly Ile  Glu Val Glu
       1040                 1045                 1050
   Ser Lys  Arg Lys Leu Asn Asn  Gly Glu Trp Gln Lys  Ile Trp Ile
       1055                 1060                 1065
   Asp Tyr  Asn Asn Tyr His Val  Arg Leu Met Ile Asn  Thr Asp Ser
       1070                 1075                 1080
   Gln Met  Leu Asp Leu Glu Asp  Lys Phe Gly Pro Phe  Glu Gly Ser
       1085                 1090                 1095
   Met Phe  Ile Gly Gly Ala Thr  Ala Glu His Leu Arg  Thr Ser Ser
       1100                 1105                 1110
   Val Gln  Gln Gly Leu Ile Gly  Cys Phe Arg Gly Leu  Val Val Asn
       1115                 1120                 1125
   Gly Glu  Ile Leu Asp Ile His  Ser Tyr Met Ser Val  His Leu Ser
       1130                 1135                 1140
   Glu Ile  Ile Lys Asp Cys Lys  Pro Phe Cys Gln Pro  Asn Lys Cys
       1145                 1150                 1155
   Gln Asn  Gly Ala Arg Cys Val  Glu Leu Trp Ser Asn  Tyr Lys Cys
       1160                 1165                 1170
   Val Cys  Glu Asn Lys Trp Ala  His Phe Gly Asp Tyr  Cys Glu Asn
       1175                 1180                 1185
   Asn Ile  Asn Asn Glu Ala Leu  Thr Phe Thr Ser Gln  Ser Ala Phe
       1190                 1195                 1200
   Leu Lys  Lys Asn Tyr Phe Gly  Thr Asp Glu Lys Asp  Glu Glu Lys
       1205                 1210                 1215
   Ser Leu  Leu Lys Asn Ile Leu  Met Lys Asn Ile Leu  Ile Asn Leu
       1220                 1225                 1230
   Arg Thr  Tyr Asp Thr Tyr Ser  Ser Ile Leu Tyr Ala  Asn Asp Asn
       1235                 1240                 1245
   Leu Asn  Asn Phe Ala His Leu  Tyr Ile Ser Asn Gly  Thr Ser Ile
       1250                 1255                 1260
   Val Tyr  Leu Phe Asn Ala Gly  Asn Glu Ile Lys Asn  Ile Thr Val
       1265                 1270                 1275
   Glu Tyr  Pro Val Asn Thr Gly  Ile Ser Ile Gln Ile  Ala Ile Ile
       1280                 1285                 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 9
   <211> 1917
   <212> PRT
   <213> Apis mellifera
<400> 9
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Leu Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                1005
   Glu Lys  Ala Ile Leu Leu Tyr  Gln Pro Pro Ile Arg  Asn Asn Tyr
       1010                 1015                 1020
   Pro Ser  Phe Met Val Ala Leu  Thr Ser Glu Tyr Arg  Leu Thr Phe
       1025                 1030                 1035
   Asn Phe  Thr Leu Ser Thr Gly  Val Ile Lys Gly Ile  Glu Val Glu
       1040                 1045                 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn  Glu Ser Ser Thr Thr  Leu His Val Asn Glu  Tyr Asn Ser
       1295                 1300                 1305
   Thr Leu  Asn Ala Thr Leu Val  Leu Leu Asp Thr Tyr  Ser Asn Lys
       1310                 1315                 1320
   Pro Trp  Ile Asn Pro Glu Lys  Glu Val Leu Ala Pro  Gln Arg Pro
       1325                 1330                 1335
   Pro Ala  Ser Pro Thr Ser Tyr  Phe Gln Val Asn Leu  Gly Gly Phe
       1340                 1345                 1350
   Asp Pro  Asp Asp Leu Leu Arg  Val Gly Lys Glu Asp  Val Gln Ile
       1355                 1360                 1365
   Lys Gly  Tyr Val Gly Cys Leu  Gly Gly Leu Met Ile  Gly Glu Tyr
       1370                 1375                 1380
   Ala Val  Asn Leu Ser Lys Leu  Ala Asn Glu Ala Asn  His Glu Gly
       1385                 1390                 1395
   Asn Lys  Glu Ile Leu Pro Asn  Cys Gln Thr Lys Cys  Asp Ala Met
       1400                 1405                 1410
   Pro Cys  Lys Asn Leu Gly Ile  Cys Thr Glu Asp Phe  Glu Arg Gln
       1415                 1420                 1425
   Glu Ser  Leu Cys Asn Cys Glu  Leu Thr Ser Tyr Phe  Gly Glu His
       1430                 1435                 1440
   Cys Ala  Asp Glu Lys Gly Ala  Asp Phe Ser Gly Glu  Ser Val Val
       1445                 1450                 1455
   Gln Arg  Glu Phe Asp Leu Val  Gly Lys Val Asn Gln  Val Lys Val
       1460                 1465                 1470
   Gln Leu  Ala Phe Ser Thr Asn  Glu Leu Arg Gln Arg  Thr Thr Ala
       1475                 1480                 1485
   Leu Leu  Leu Leu Gln Thr Glu  Asn Lys Arg Ser Tyr  Tyr Leu Leu
       1490                 1495                 1500
   Val Ala  Leu Thr Ser Glu Gly  Gln Leu Ile Phe Glu  Glu Asp Arg
       1505                 1510                 1515
   Glu Gly  Ser Ala Phe Gly Val  Arg Leu Asn Asp Ile  Asn Phe Leu
       1520                 1525                 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
           1760                1765                1770
   Thr His  Met Lys Ala Ala Val  Lys Glu Tyr Lys Pro  Leu Ala Asn
       1775                1780                1785
   Ala Glu  Ser Lys Glu Leu Ile  Asn Asp Lys Arg Val  His Ile Ile
       1790                1795                1800
   Ala Asp  Glu Glu Pro Glu Lys  Lys Glu Leu Leu Gly  Val Asn Thr
       1805                1810                1815
   Gly Ile  Ile Thr Lys Ser Pro  Lys Leu Asn Pro Phe  Ser Met Glu
       1820                1825                1830
   Asp Leu  Gln Glu Glu Pro Glu  Leu Glu Glu Cys Glu  Glu Glu Glu
       1835                1840                1845
   Pro Asp  Glu Glu Asp Glu Asp  Glu Asp Glu Asp Asn  Pro Lys Glu
       1850                1855                1860
   Gln Asn  Thr Asp Glu Glu Glu  Ile His Gln Lys Thr  Glu Arg Thr
       1865                1870                1875
   Asp Asp  Asn Asp Phe Val Lys  Ser Pro Glu Asn Ser  Ser Arg Ile
       1880                1885                1890
   Ser Val  Glu Glu Ser Val Asp  Ser Leu Asn Asn Asn  Pro Lys His
       1895                1900                1905
   Asp Glu  Thr Lys Thr Gln Tyr  Glu Val
       1910                1915
<210> 10
   <211> 1917
   <212> PRT
   <213> Apis mellifera
<400> 10
   Met Asp Ser Phe Thr Thr Thr Lys Arg Thr Phe Leu Asp Arg Cys Leu
   1               5                   10                  15
   Val Lys Thr Glu Pro Gly Pro Cys Lys Gln Tyr Val Thr Arg Trp Thr
               20                  25                  30
   Phe Ile Lys Thr Glu Gly Lys Cys Lys Thr Phe Ser Tyr Gly Gly Cys
           35                  40                  45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
   325 330 335
   Glu Asn Leu Cys Phe Ile Ser Ser Gln Cys Val Asn His Tyr Asn Ser
   340 345 350
   Leu Thr Cys Asp Cys Phe Gly Thr Lys Tyr Glu Gly Glu Arg Cys Asp
   355 360 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385 390 395 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Lys Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                 1005
   Glu Lys  Ala Ile Leu Leu Tyr  Gln Pro Pro Ile Arg  Asn Asn Tyr
       1010                 1015                 1020
   Pro Ser  Phe Met Val Ala Leu  Thr Ser Glu Tyr Arg  Leu Thr Phe
       1025                 1030                 1035
   Asn Phe  Thr Leu Ser Thr Gly  Val Ile Lys Gly Ile  Glu Val Glu
       1040                 1045                 1050
   Ser Lys  Arg Lys Leu Asn Asn  Gly Glu Trp Gln Lys  Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn  Glu Ser Ser Thr Thr  Leu His Val Asn Glu  Tyr Asn Ser
       1295                 1300                 1305
   Thr Leu  Asn Ala Thr Leu Val  Leu Leu Asp Thr Tyr  Ser Asn Lys
       1310                 1315                 1320
   Pro Trp  Ile Asn Pro Glu Lys  Glu Val Leu Ala Pro  Gln Arg Pro
       1325                 1330                 1335
   Pro Ala  Ser Pro Thr Ser Tyr  Phe Gln Val Asn Leu  Gly Gly Phe
       1340                 1345                 1350
   Asp Pro  Asp Asp Leu Leu Arg  Val Gly Lys Glu Asp  Val Gln Ile
       1355                 1360                 1365
   Lys Gly  Tyr Val Gly Cys Leu  Gly Gly Leu Met Ile  Gly Glu Tyr
       1370                 1375                 1380
   Ala Val  Asn Leu Ser Lys Leu  Ala Asn Glu Ala Asn  His Glu Gly
       1385                 1390                 1395
   Asn Lys  Glu Ile Leu Pro Asn  Cys Gln Thr Lys Cys  Asp Ala Met
       1400                 1405                 1410
   Pro Cys  Lys Asn Leu Gly Ile  Cys Thr Glu Asp Phe  Glu Arg Gln
       1415                 1420                 1425
   Glu Ser  Leu Cys Asn Cys Glu  Leu Thr Ser Tyr Phe  Gly Glu His
       1430                 1435                 1440
   Cys Ala  Asp Glu Lys Gly Ala  Asp Phe Ser Gly Glu  Ser Val Val
       1445                 1450                 1455
   Gln Arg  Glu Phe Asp Leu Val  Gly Lys Val Asn Gln  Val Lys Val
       1460                 1465                 1470
   Gln Leu  Ala Phe Ser Thr Asn  Glu Leu Arg Gln Arg  Thr Thr Ala
       1475                 1480                 1485
   Leu Leu  Leu Leu Gln Thr Glu  Asn Lys Arg Ser Tyr  Tyr Leu Leu
       1490                 1495                 1500
   Val Ala  Leu Thr Ser Glu Gly  Gln Leu Ile Phe Glu  Glu Asp Arg
       1505                 1510                 1515
   Glu Gly  Ser Ala Phe Gly Val  Arg Leu Asn Asp Ile  Asn Phe Leu
       1520                 1525                 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 11
   <211> 1917
   <212> PRT
   <213> Apis mellifera
<400> 11
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
   325 330 335
   Glu Asn Leu Cys Phe Ile Ser Ser Gln Cys Val Asn His Tyr Asn Ser
   340 345 350
   Leu Thr Cys Asp Cys Phe Gly Thr Lys Tyr Glu Gly Glu Arg Cys Asp
   355 360 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385 390 395 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Lys Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                1005
   Glu Lys  Ala Ile Leu Leu Tyr  Gln Pro Pro Ile Arg  Asn Asn Tyr
       1010                1015                1020
   Pro Ser  Phe Met Val Ala Leu  Thr Ser Glu Tyr Arg  Leu Thr Phe
       1025                1030                1035
   Asn Phe  Thr Leu Ser Thr Gly  Val Ile Lys Gly Ile  Glu Val Glu
       1040                1045                1050
   Ser Lys  Arg Lys Leu Asn Asn  Gly Glu Trp Gln Lys  Ile Trp Ile
       1055                1060                1065
   Asp Tyr  Asn Asn Tyr His Val  Arg Leu Met Ile Asn  Thr Asp Ser
       1070                 1075                 1080
   Gln Met  Leu Asp Leu Glu Asp  Lys Phe Gly Pro Phe  Glu Gly Ser
       1085                 1090                 1095
   Met Phe  Ile Gly Gly Ala Thr  Ala Glu His Leu Arg  Thr Ser Ser
       1100                 1105                 1110
   Val Gln  Gln Gly Leu Ile Gly  Cys Phe Arg Gly Leu  Val Val Asn
       1115                 1120                 1125
   Gly Glu  Ile Leu Asp Ile His  Ser Tyr Met Ser Val  His Leu Ser
       1130                 1135                 1140
   Glu Ile  Ile Lys Asp Cys Lys  Pro Phe Cys Gln Pro  Asn Lys Cys
       1145                 1150                 1155
   Gln Asn  Gly Ala Arg Cys Val  Glu Leu Trp Ser Asn  Tyr Lys Cys
       1160                 1165                 1170
   Val Cys  Glu Asn Lys Trp Ala  His Phe Gly Asp Tyr  Cys Glu Asn
       1175                 1180                 1185
   Asn Ile  Asn Asn Glu Ala Leu  Thr Phe Thr Ser Gln  Ser Ala Phe
       1190                 1195                 1200
   Leu Lys  Lys Asn Tyr Phe Gly  Thr Asp Glu Lys Asp  Glu Glu Lys
       1205                 1210                 1215
   Ser Leu  Leu Lys Asn Ile Leu  Met Lys Asn Ile Leu  Ile Asn Leu
       1220                 1225                 1230
   Arg Thr  Tyr Asp Thr Tyr Ser  Ser Ile Leu Tyr Ala  Asn Asp Asn
       1235                 1240                 1245
   Leu Asn  Asn Phe Ala His Leu  Tyr Ile Ser Asn Gly  Thr Ser Ile
       1250                 1255                 1260
   Val Tyr  Leu Phe Asn Ala Gly  Asn Glu Ile Lys Asn  Ile Thr Val
       1265                 1270                 1275
   Glu Tyr  Pro Val Asn Thr Gly  Ile Ser Ile Gln Ile  Ala Ile Ile
       1280                 1285                 1290
   Arg Asn  Glu Ser Ser Thr Thr  Leu His Val Asn Glu  Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 12
   <211> 1308
   <212> PRT
   <213> Apis mellifera
<400> 12
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
   130 135 140
   Gln Leu Asp Gly Ser Gly Ile Lys Pro Phe Gln Leu Arg Leu Cys Arg
   145 150 155 160
   Glu Ile Ser Phe Lys Phe Arg Thr Lys Leu Pro His Gly Leu Leu Val
   165 170 175
   Tyr His Ser Val Lys Asp Arg Pro Glu Ile Arg Leu Asp Pro Tyr Ala
   180 185 190
   Leu Tyr Val Ile Val Glu Lys Gly Gln Leu Lys Val Val His Val Phe
   195 200 205
   Gly Lys Arg Ser Thr Ser Leu Thr Val Gly Glu Gly Leu Asn Arg Asp
   210 215 220
   Glu Trp His Ser Val Leu Val Arg Ile Asp Val His Gly Ala Lys Leu
   225 230 235 240
   Ile Ala Arg Val Asp Asp Lys Gln Glu Glu Thr Thr Leu Glu Val Leu
   245 250 255
   Glu His Val Ile Asn Tyr Gly Val Ser Glu Glu Leu Ala Ser Val Val
   260 265 270
   Leu Ile Gly Gly Leu Ser Ser Glu Glu Arg Leu His Gly Val Lys Tyr
   275 280 285
   Ile Ile Glu Ser Phe Val Gly Cys Ile Arg Asp Met Val Leu Ser Ser
   290 295 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
   820 825 830
   Arg Ser Ile Ala Glu Ser Asp Phe Ser Phe Asn Ile Lys Tyr Arg Gln
   835 840 845
   Phe Thr Ala Glu Met Leu Gln Glu Leu Ile Ser His Ser Leu Tyr Cys
   850 855 860
   Ser Gln Tyr Val Lys Tyr Asp Cys Tyr Lys Ala Pro Leu Glu Leu His
   865 870 875 880
   Ser Ala Thr Trp Phe Leu Asn Ser Lys Gly Thr Thr Val Asp Tyr Ile
   885 890 895
   Gly Asn Val Asn Arg Gly Ser Cys Pro Cys Gly Met Asn Lys Thr Cys
   900 905 910
   Ile Asn Pro Asn Leu Ser Cys Asn Cys Asp Phe Thr Gly Asn Gly Asp
   915 920 925
   Lys Trp Leu Ser Asp Glu Gly Tyr Tyr Glu Thr Pro Asp Ser Leu Gly
   930 935 940
   Ile Thr Gly Met Val Phe Leu Gln Gln Arg Asp Leu Glu Glu Asp Ala
   945 950 955 960
   Gln Gly Arg Ile Thr Leu Gly Pro Leu Glu Cys Val Glu Thr Asn Thr
   965 970 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr  Asn Asn Tyr His Val  Arg Leu Met Ile Asn  Thr Asp Ser
       1070                 1075                 1080
   Gln Met  Leu Asp Leu Glu Asp  Lys Phe Gly Pro Phe  Glu Gly Ser
       1085                 1090                 1095
   Met Phe  Ile Gly Gly Ala Thr  Ala Glu His Leu Arg  Thr Ser Ser
       1100                 1105                 1110
   Val Gln  Gln Gly Leu Ile Gly  Cys Phe Arg Gly Leu  Val Val Asn
       1115                 1120                 1125
   Gly Glu  Ile Leu Asp Ile His  Ser Tyr Met Ser Val  His Leu Ser
       1130                 1135                 1140
   Glu Ile  Ile Lys Asp Cys Lys  Pro Phe Cys Gln Pro  Asn Lys Cys
       1145                 1150                 1155
   Gln Asn  Gly Ala Arg Cys Val  Glu Leu Trp Ser Asn  Tyr Lys Cys
       1160                 1165                 1170
   Val Cys  Glu Asn Lys Trp Ala  His Phe Gly Asp Tyr  Cys Glu Asn
       1175                 1180                 1185
   Asn Ile  Asn Asn Glu Ala Leu  Thr Phe Thr Ser Gln  Ser Ala Phe
       1190                 1195                 1200
   Leu Lys  Lys Asn Tyr Phe Gly  Thr Asp Glu Lys Asp  Glu Glu Lys
       1205                 1210                 1215
   Ser Leu  Leu Lys Asn Ile Leu  Met Lys Asn Ile Leu  Ile Asn Leu
       1220                 1225                 1230
   Arg Thr  Tyr Asp Thr Tyr Ser  Ser Ile Leu Tyr Ala  Asn Asp Asn
       1235                 1240                 1245
   Leu Asn  Asn Phe Ala His Leu  Tyr Ile Ser Asn Gly  Thr Ser Ile
       1250                 1255                 1260
   Val Tyr  Leu Phe Asn Ala Gly  Asn Glu Ile Lys Asn  Ile Thr Val
       1265                 1270                 1275
   Glu Tyr  Pro Val Asn Thr Gly  Ile Ser Ile Gln Ile  Ala Ile Ile
       1280                 1285                 1290
   Arg Asn  Glu Ser Ser Thr Thr  Leu His Val Asn Glu  Tyr Asn Ser
       1295                 1300                 1305
<210> 13
   <211> 1308
   <212> PRT
   <213> Apis mellifera
<400> 13
   Met Asp Ser Phe Thr Thr Thr Lys Arg Thr Phe Leu Asp Arg Cys Leu
   1               5                   10                  15
   Val Lys Thr Glu Pro Gly Pro Cys Lys Gln Tyr Val Thr Arg Trp Thr
               20                  25                  30
   Phe Ile Lys Thr Glu Gly Lys Cys Lys Thr Phe Ser Tyr Gly Gly Cys
           35                  40                  45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225 230 235 240
   Ile Ala Arg Val Asp Asp Lys Gln Glu Glu Thr Thr Leu Glu Val Leu
   245 250 255
   Glu His Val Ile Asn Tyr Gly Val Ser Glu Glu Leu Ala Ser Val Val
   260 265 270
   Leu Ile Gly Gly Leu Ser Ser Glu Glu Arg Leu His Gly Val Lys Tyr
   275 280 285
   Ile Ile Glu Ser Phe Val Gly Cys Ile Arg Asp Met Val Leu Ser Ser
   290 295 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
   325 330 335
   Glu Asn Leu Cys Phe Ile Ser Ser Gln Cys Val Asn His Tyr Asn Ser
   340 345 350
   Leu Thr Cys Asp Cys Phe Gly Thr Lys Tyr Glu Gly Glu Arg Cys Asp
   355 360 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385 390 395 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
<210> 14
   <211> 1917
   <212> PRT
   <213> Apis mellifera
<400> 14
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385 390 395 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
   770 775 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
   805 810 815
   Ser Thr Lys Thr Ile Val Glu His Asn Leu Pro Ser Gln Val Asp Val
   820 825 830
   Arg Ser Ile Ala Glu Ser Asp Phe Ser Phe Asn Ile Lys Tyr Arg Gln
   835 840 845
   Phe Thr Ala Glu Met Leu Gln Glu Leu Ile Ser His Ser Leu Tyr Cys
   850 855 860
   Ser Gln Tyr Val Lys Tyr Asp Cys Tyr Lys Ala Pro Leu Glu Leu His
   865 870 875 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                1005
   Glu Lys  Ala Ile Leu Leu Tyr  Gln Pro Pro Ile Arg  Asn Asn Tyr
       1010                 1015                 1020
   Pro Ser  Phe Met Val Ala Leu  Thr Ser Glu Tyr Arg  Leu Thr Phe
       1025                 1030                 1035
   Asn Phe  Thr Leu Ser Thr Gly  Val Ile Lys Gly Ile  Glu Val Glu
       1040                 1045                 1050
   Ser Lys  Arg Lys Leu Asn Asn  Gly Glu Trp Gln Lys  Ile Trp Ile
       1055                 1060                 1065
   Asp Tyr  Asn Asn Tyr His Val  Arg Leu Met Ile Asn  Thr Asp Ser
       1070                 1075                 1080
   Gln Met  Leu Asp Leu Glu Asp  Lys Phe Gly Pro Phe  Glu Gly Ser
       1085                 1090                 1095
   Met Phe  Ile Gly Gly Ala Thr  Ala Glu His Leu Arg  Thr Ser Ser
       1100                 1105                 1110
   Val Gln  Gln Gly Leu Ile Gly  Cys Phe Arg Gly Leu  Val Val Asn
       1115                 1120                 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Met Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 15
   <211> 1917
   <212> PRT
   <213> Apis mellifera
<400> 15
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
   770 775 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
   805 810 815
   Ser Thr Lys Thr Ile Val Glu His Asn Leu Pro Ser Gln Val Asp Val
   820 825 830
   Arg Ser Ile Ala Glu Ser Asp Phe Ser Phe Asn Ile Lys Tyr Arg Gln
   835 840 845
   Phe Thr Ala Glu Met Leu Gln Glu Leu Ile Ser His Ser Leu Tyr Cys
   850 855 860
   Ser Gln Tyr Val Lys Tyr Asp Cys Tyr Lys Ala Pro Leu Glu Leu His
   865 870 875 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                1005
   Glu Lys  Ala Ile Leu Leu Tyr  Gln Pro Pro Ile Arg  Asn Asn Tyr
       1010                1015                1020
   Pro Ser  Phe Met Val Ala Leu  Thr Ser Glu Tyr Arg  Leu Thr Phe
       1025                1030                1035
   Asn Phe  Thr Leu Ser Thr Gly  Val Ile Lys Gly Ile  Glu Val Glu
       1040                1045                1050
   Ser Lys  Arg Lys Leu Asn Asn  Gly Glu Trp Gln Lys  Ile Trp Ile
       1055                1060                1065
   Asp Tyr  Asn Asn Tyr His Val  Arg Leu Met Ile Asn  Thr Asp Ser
       1070                1075                1080
   Gln Met  Leu Asp Leu Glu Asp  Lys Phe Gly Pro Phe  Glu Gly Ser
       1085                1090                1095
   Met Phe  Ile Gly Gly Ala Thr  Ala Glu His Leu Arg  Thr Ser Ser
       1100                1105                1110
   Val Gln  Gln Gly Leu Ile Gly  Cys Phe Arg Gly Leu  Val Val Asn
       1115                1120                1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Thr Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 16
   <211> 1917
   <212> PRT
   <213> Apis mellifera
<400> 16
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
       130                 135                 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385 390 395 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
   885 890 895
   Gly Asn Val Asn Arg Gly Ser Cys Pro Cys Gly Met Asn Lys Thr Cys
   900 905 910
   Ile Asn Pro Asn Leu Ser Cys Asn Cys Asp Phe Thr Gly Asn Gly Asp
   915 920 925
   Lys Trp Leu Ser Asp Glu Gly Tyr Tyr Glu Thr Pro Asp Ser Leu Gly
   930 935 940
   Ile Thr Gly Met Val Phe Leu Gln Gln Arg Asp Leu Glu Glu Asp Ala
   945 950 955 960
   Gln Gly Arg Ile Thr Leu Gly Pro Leu Glu Cys Val Glu Thr Asn Thr
   965 970 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly  Gly Pro Asp Met Lys  Pro Leu Glu Glu Tyr  Met Leu Phe
       1610                 1615                 1620
   Thr Lys  Gln Gly Ser Glu Thr  Val Arg Ala Thr Met  Pro Ala Gly
       1625                 1630                 1635
   Val Arg  Ser Ala Gln Cys Ala  Val Phe His Val Gln  Pro His Gly
       1640                 1645                 1650
   Leu Asp  Pro Pro Lys Asn Asp  Ser Asn Val Gly Val  Asp Lys Met
       1655                 1660                 1665
   Trp Val  Glu Asp Pro Pro Glu  Arg Ile Pro Tyr Lys  Ser Gln Tyr
       1670                 1675                 1680
   Thr Asn  Ala Thr Gln Glu Glu  Gln Gly Ala Gly Thr  Tyr Ile Phe
       1685                 1690                 1695
   Ile Ala  Leu Cys Cys Val Phe  Val Thr Ala Val Ile  Gly Cys Ile
       1700                 1705                 1710
   Tyr Glu  Val Trp Arg Ser Ala  Arg Lys Asp Arg Arg  Arg Arg Arg
       1715                 1720                 1725
   Ala Ser  Val Ala Ser Thr Val  Pro Ala Pro Gly Ser  Gln Arg Trp
       1730                 1735                 1740
   Gln Ser  Gln Gln Tyr Thr Asp  Pro Leu Thr Gly Gly  Val Lys Thr
       1745                 1750                 1755
   Val Gly  Phe Lys Asn Val Ala  Glu Asp Glu Lys Arg  Pro Asn Gly
       1760                 1765                 1770
   Thr His  Met Lys Ala Ala Val  Lys Glu Tyr Lys Pro  Leu Ala Asn
       1775                 1780                 1785
   Ala Glu  Ser Lys Glu Leu Ile  Asn Asp Lys Arg Val  His Ile Ile
       1790                 1795                 1800
   Ala Asp  Glu Glu Pro Glu Lys  Lys Glu Leu Leu Gly  Val Asn Thr
       1805                 1810                 1815
   Gly Ile  Ile Thr Lys Ser Pro  Lys Leu Asn Pro Phe  Ser Met Glu
       1820                 1825                 1830
   Asp Leu  Gln Glu Glu Pro Glu  Leu Glu Glu Cys Glu  Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 17
   <211> 1917
   <212> PRT
   <213> Apis mellifera
<400> 17
   Met Asp Ser Phe Thr Thr Thr Lys Arg Thr Phe Leu Asp Arg Cys Leu
   1               5                   10                  15
   Val Lys Thr Glu Pro Gly Pro Cys Lys Gln Tyr Val Thr Arg Trp Thr
               20                  25                  30
   Phe Ile Lys Thr Glu Gly Lys Cys Lys Thr Phe Ser Tyr Gly Gly Cys
           35                  40                  45
   Leu Gly Asn Glu Asn Arg Phe Asn Ser Glu Ala Glu Cys Leu His Tyr
       50                  55                  60
   Cys Val Gly Gly Pro Glu His Thr Leu Pro His Met Val Thr Asn Gly
   65                  70                  75                  80
   Asn Val Leu Val Thr Ser Thr Ala Thr Thr Asn Thr Leu Pro Ser Thr
                   85                  90                  95
   Thr Ala Ile Thr Ser Arg Pro Thr Phe Ser Pro Pro Glu Pro Thr Lys
               100                 105                 110
   Pro Pro Val Pro Lys Tyr Lys Arg Gly Lys Glu Leu Thr Phe Met Glu
           115                 120                 125
   Ser Gly Tyr Glu Lys Thr Phe Met Phe Ala Gln Ser Asn Thr Phe Ile
   130 135 140
   Gln Leu Asp Gly Ser Gly Ile Lys Pro Phe Gln Leu Arg Leu Cys Arg
   145 150 155 160
   Glu Ile Ser Phe Lys Phe Arg Thr Lys Leu Pro His Gly Leu Leu Val
   165 170 175
   Tyr His Ser Val Lys Asp Arg Pro Glu Ile Arg Leu Asp Pro Tyr Ala
   180 185 190
   Leu Tyr Val Ile Val Glu Lys Gly Gln Leu Lys Val Val His Val Phe
   195 200 205
   Gly Lys Arg Ser Thr Ser Leu Thr Val Gly Glu Gly Leu Asn Arg Asp
   210 215 220
   Glu Trp His Ser Val Leu Val Arg Ile Asp Val His Gly Ala Lys Leu
   225 230 235 240
   Ile Ala Arg Val Asp Asp Lys Gln Glu Glu Thr Thr Leu Glu Val Leu
   245 250 255
   Glu His Val Ile Asn Tyr Gly Val Ser Glu Glu Leu Ala Ser Val Val
   260 265 270
   Leu Ile Gly Gly Leu Ser Ser Glu Glu Arg Leu His Gly Val Lys Tyr
   275 280 285
   Ile Ile Glu Ser Phe Val Gly Cys Ile Arg Asp Met Val Leu Ser Ser
   290 295 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
   325 330 335
   Glu Asn Leu Cys Phe Ile Ser Ser Gln Cys Val Asn His Tyr Asn Ser
   340 345 350
   Leu Thr Cys Asp Cys Phe Gly Thr Lys Tyr Glu Gly Glu Arg Cys Asp
   355 360 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
               900                 905                 910
   Ile Asn Pro Asn Leu Ser Cys Asn Cys Asp Phe Thr Gly Asn Gly Asp
           915                 920                 925
   Lys Trp Leu Ser Asp Glu Gly Tyr Tyr Glu Thr Pro Asp Ser Leu Gly
       930                 935                 940
   Ile Thr Gly Met Val Phe Leu Gln Gln Arg Asp Leu Glu Glu Asp Ala
   945                 950                 955                 960
   Gln Gly Arg Ile Thr Leu Gly Pro Leu Glu Cys Val Glu Thr Asn Thr
                   965                 970                 975
   Gln Lys Tyr Val Val Thr Phe Thr Thr Ser Gln Ser Tyr Ile Glu Val
               980                 985                 990
   Pro Gly Trp Arg Lys Gly Asp Ile  Ala Phe Ser Phe Arg  Thr Thr Gly
           995                 1000                 1005
   Glu Lys  Ala Ile Leu Leu Tyr  Gln Pro Pro Ile Arg  Asn Asn Tyr
       1010                 1015                 1020
   Pro Ser  Phe Met Val Ala Leu  Thr Ser Glu Tyr Arg  Leu Thr Phe
       1025                 1030                 1035
   Asn Phe  Thr Leu Ser Thr Gly  Val Ile Lys Gly Ile  Glu Val Glu
       1040                 1045                 1050
   Ser Lys  Arg Lys Leu Asn Asn  Gly Glu Trp Gln Lys  Ile Trp Ile
       1055                 1060                 1065
   Asp Tyr  Asn Asn Tyr His Val  Arg Leu Met Ile Asn  Thr Asp Ser
       1070                 1075                 1080
   Gln Met  Leu Asp Leu Glu Asp  Lys Phe Gly Pro Phe  Glu Gly Ser
       1085                 1090                 1095
   Met Phe  Ile Gly Gly Ala Thr  Ala Glu His Leu Arg  Thr Ser Ser
       1100                 1105                 1110
   Val Gln  Gln Gly Leu Ile Gly  Cys Phe Arg Gly Leu  Val Val Asn
       1115                 1120                 1125
   Gly Glu  Ile Leu Asp Ile His  Ser Tyr Met Ser Val  His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly  Gly Pro Asp Met Lys  Pro Leu Glu Glu Tyr  Met Leu Phe
       1610                 1615                 1620
   Thr Lys  Gln Gly Ser Glu Thr  Val Arg Ala Thr Met  Pro Ala Gly
       1625                 1630                 1635
   Val Arg  Ser Ala Gln Cys Ala  Val Phe His Val Gln  Pro His Gly
       1640                 1645                 1650
   Leu Asp  Pro Pro Lys Asn Asp  Ser Asn Val Gly Val  Asp Lys Val
       1655                 1660                 1665
   Trp Val  Glu Asp Pro Pro Glu  Arg Ile Pro Tyr Lys  Ser Gln Tyr
       1670                 1675                 1680
   Thr Asn  Ala Thr Gln Glu Glu  Gln Gly Ala Gly Thr  Tyr Ile Phe
       1685                 1690                 1695
   Ile Ala  Leu Cys Cys Val Phe  Val Thr Ala Val Ile  Gly Cys Ile
       1700                 1705                 1710
   Tyr Glu  Val Trp Arg Ser Ala  Arg Lys Asp Arg Arg  Arg Arg Arg
       1715                 1720                 1725
   Ala Ser  Val Ala Ser Thr Val  Pro Ala Pro Gly Ser  Gln Arg Trp
       1730                 1735                 1740
   Gln Ser  Gln Gln Tyr Thr Asp  Pro Leu Thr Gly Gly  Val Lys Thr
       1745                 1750                 1755
   Val Gly  Phe Lys Asn Val Ala  Glu Asp Glu Lys Arg  Pro Asn Gly
       1760                 1765                 1770
   Thr His  Met Lys Ala Ala Val  Lys Glu Tyr Lys Pro  Leu Ala Asn
       1775                 1780                 1785
   Ala Glu  Ser Lys Glu Leu Ile  Asn Asp Lys Arg Val  His Ile Ile
       1790                 1795                 1800
   Ala Asp  Glu Glu Pro Glu Lys  Lys Glu Leu Leu Gly  Val Asn Thr
       1805                 1810                 1815
   Gly Ile  Ile Thr Lys Ser Pro  Lys Leu Asn Pro Phe  Ser Met Glu
       1820                 1825                 1830
   Asp Leu  Gln Glu Glu Pro Glu  Leu Glu Glu Cys Glu  Glu Glu Glu
       1835                 1840                 1845
   Pro Asp Glu Glu Asp Glu Asn Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 18
   <211> 1917
   <212> PRT
   <213> Apis mellifera
<400> 18
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
   130 135 140
   Gln Leu Asp Gly Ser Gly Ile Lys Pro Phe Gln Leu Arg Leu Cys Arg
   145 150 155 160
   Glu Ile Ser Phe Lys Phe Arg Thr Lys Leu Pro His Gly Leu Leu Val
   165 170 175
   Tyr His Ser Val Lys Asp Arg Pro Glu Ile Arg Leu Asp Pro Tyr Ala
   180 185 190
   Leu Tyr Val Ile Val Glu Lys Gly Gln Leu Lys Val Val His Val Phe
   195 200 205
   Gly Lys Arg Ser Thr Ser Leu Thr Val Gly Glu Gly Leu Asn Arg Asp
   210 215 220
   Glu Trp His Ser Val Leu Val Arg Ile Asp Val His Gly Ala Lys Leu
   225 230 235 240
   Ile Ala Arg Val Asp Asp Lys Gln Glu Glu Thr Thr Leu Glu Val Leu
   245 250 255
   Glu His Val Ile Asn Tyr Gly Val Ser Glu Glu Leu Ala Ser Val Val
   260 265 270
   Leu Ile Gly Gly Leu Ser Ser Glu Glu Arg Leu His Gly Val Lys Tyr
   275 280 285
   Ile Ile Glu Ser Phe Val Gly Cys Ile Arg Asp Met Val Leu Ser Ser
   290 295 300
   Gly Lys Ser Ala Ser Asp Leu Leu Pro Ile Gln Pro Leu Ile Ala Thr
   305 310 315 320
   Lys His Glu Asn Val Arg Glu Gly Cys Ile Asp Lys Cys Arg Thr Arg
   325 330 335
   Glu Asn Leu Cys Phe Ile Ser Ser Gln Cys Val Asn His Tyr Asn Ser
   340 345 350
   Leu Thr Cys Asp Cys Phe Gly Thr Lys Tyr Glu Gly Glu Arg Cys Asp
   355 360 365
   Val Tyr Thr Ala Thr Ile Leu Thr Leu Arg Gly Ser Ser Tyr Val Ser
   370 375 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
                   405                 410                 415
   Ala Ser Gly Glu Ile Asp Gly Thr Pro His Tyr Val Ala Ala Ser Ile
               420                 425                 430
   Val Asn Gly Ser Val His Ile Glu Leu Asp Phe Gly His Asn Ser Lys
           435                 440                 445
   Ile Ala Thr Val Leu Gly Asp Tyr Ile Thr Ser Asn His Trp Asn Asn
       450                 455                 460
   Leu Thr Ile Phe His Asn Gly Ser Leu Val Phe Val Ser Leu Asp Asp
   465                 470                 475                 480
   Glu Ile Lys Val Leu Glu Ile Pro Gly Glu Asn Tyr Asn Met Ile Ile
                   485                 490                 495
   Asp Pro Glu Ile Tyr Ile Gly Gly Gly Pro Glu Leu Tyr Lys Lys Lys
               500                 505                 510
   Gly Leu Ser Ser His Asn Asn Phe Ala Gly Ser Leu Lys Tyr Val Phe
           515                 520                 525
   Phe Asn Asp Lys Ser Ile Ile Tyr Glu Leu Lys Arg Ser Asn Pro Met
       530                 535                 540
   Val His Tyr Ile Gly Val Leu Glu Pro Glu Tyr Tyr Glu Ala Asp Val
   545                 550                 555                 560
   Asp Val Ile Pro Ile Thr Tyr Pro Phe Ala Thr Ser His Ile Trp Trp
                   565                 570                 575
   Pro Ile Ala Arg Thr Asp Ser Leu Lys Leu Asn Phe Asp Phe Lys Ser
               580                 585                 590
   Ser Lys Pro Val Ala Val Val Ala Ser Gly Asp Val Lys Cys Asn Arg
           595                 600                 605
   Ser Phe Gly Tyr Trp Glu Leu Gln Leu Val Asn Asp Glu Ile Arg Phe
       610                 615                 620
   Gln Leu Ile Pro Val Leu Gly Glu Asn Val Thr Val Thr Thr Ala Val
   625                 630                 635                 640
   Lys Phe Pro Pro Tyr Asn Thr Ser Trp His Ala Val Glu Leu Asn Tyr
                   645                 650                 655
   Thr Arg Gly Glu Ile Ser Val Leu Val Asp Tyr Arg Asn Lys Gln Ser
               660                 665                 670
   Lys Leu Phe Ser Met Thr Phe Glu Leu Gly Asp Lys Val Ile Ile Gly
           675                 680                 685
   Ser Gly Lys Asn Asn Thr Gly Leu Val Gly Cys Met Arg Glu Ile Arg
       690                 695                 700
   Val Asn Glu Glu Arg Ile Glu Pro Arg Tyr Val Met Asn Thr Asp Arg
   705                 710                 715                 720
   Val Met Gly Glu Val Ala Leu Asp Asn Cys Gln Phe Ile Asp Pro Cys
                   725                 730                 735
   Thr Arg Pro Asn Thr Cys Glu His Gly Gly Lys Cys Ser Val Lys Lys
               740                 745                 750
   Asp Glu Ile Ser Cys Asp Cys Thr Asp Thr Gly Tyr Val Gly Lys Asn
           755                 760                 765
   Cys His Phe Thr Lys Phe Arg Lys Thr Cys Glu Glu Leu Ala Leu Leu
       770                 775                 780
   Gly Tyr Thr Gln Asp Asp Val Tyr Lys Ile Asp Ile Asp Gly Asn Gly
   785                 790                 795                 800
   Arg Phe Pro Pro Ala Leu Val Lys Cys Glu Phe Gln Ser Ile Glu Asn
                   805                 810                 815
   Ser Thr Lys Thr Ile Val Glu His Asn Leu Pro Ser Gln Val Asp Val
               820                 825                 830
   Arg Ser Ile Ala Glu Ser Asp Phe Ser Phe Asn Ile Lys Tyr Arg Gln
           835                 840                 845
   Phe Thr Ala Glu Met Leu Gln Glu Leu Ile Ser His Ser Leu Tyr Cys
       850                 855                 860
   Ser Gln Tyr Val Lys Tyr Asp Cys Tyr Lys Ala Pro Leu Glu Leu His
   865                 870                 875                 880
   Ser Ala Thr Trp Phe Leu Asn Ser Lys Gly Thr Thr Val Asp Tyr Ile
                   885                 890                 895
   Gly Asn Val Asn Arg Gly Ser Cys Pro Cys Gly Met Asn Lys Thr Cys
   900 905 910
   Ile Asn Pro Asn Leu Ser Cys Asn Cys Asp Phe Thr Gly Asn Gly Asp
   915 920 925
   Lys Trp Leu Ser Asp Glu Gly Tyr Tyr Glu Thr Pro Asp Ser Leu Gly
   930 935 940
   Ile Thr Gly Met Val Phe Leu Gln Gln Arg Asp Leu Glu Glu Asp Ala
   945 950 955 960
   Gln Gly Arg Ile Thr Leu Gly Pro Leu Glu Cys Val Glu Thr Asn Thr
   965 970 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asp Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 19
   <211> 1917
   <212> PRT
   <213> Apis mellifera
<400> 19
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
   130 135 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
   770 775 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
   805 810 815
   Ser Thr Lys Thr Ile Val Glu His Asn Leu Pro Ser Gln Val Asp Val
   820 825 830
   Arg Ser Ile Ala Glu Ser Asp Phe Ser Phe Asn Ile Lys Tyr Arg Gln
   835 840 845
   Phe Thr Ala Glu Met Leu Gln Glu Leu Ile Ser His Ser Leu Tyr Cys
   850 855 860
   Ser Gln Tyr Val Lys Tyr Asp Cys Tyr Lys Ala Pro Leu Glu Leu His
   865 870 875 880
   Ser Ala Thr Trp Phe Leu Asn Ser Lys Gly Thr Thr Val Asp Tyr Ile
   885 890 895
   Gly Asn Val Asn Arg Gly Ser Cys Pro Cys Gly Met Asn Lys Thr Cys
   900 905 910
   Ile Asn Pro Asn Leu Ser Cys Asn Cys Asp Phe Thr Gly Asn Gly Asp
   915 920 925
   Lys Trp Leu Ser Asp Glu Gly Tyr Tyr Glu Thr Pro Asp Ser Leu Gly
   930 935 940
   Ile Thr Gly Met Val Phe Leu Gln Gln Arg Asp Leu Glu Glu Asp Ala
   945 950 955 960
   Gln Gly Arg Ile Thr Leu Gly Pro Leu Glu Cys Val Glu Thr Asn Thr
   965 970 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asp Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Val Asp Ser Leu Asn Asn Asn Pro Lys His
   1895 1900 1905
   Asp Glu Thr Lys Thr Gln Tyr Glu Val
   1910 1915
<210> 20
   <211> 1917
   <212> PRT
   <213> Apis mellifera
<400> 20
   Met Asp Ser Phe Thr Thr Thr Lys Arg Thr Phe Leu Asp Arg Cys Leu
   1 5 10 15
   Val Lys Thr Glu Pro Gly Pro Cys Lys Gln Tyr Val Thr Arg Trp Thr
   20 25 30
   Phe Ile Lys Thr Glu Gly Lys Cys Lys Thr Phe Ser Tyr Gly Gly Cys
   35 40 45
   Leu Gly Asn Glu Asn Arg Phe Asn Ser Glu Ala Glu Cys Leu His Tyr
   50 55 60
   Cys Val Gly Gly Pro Glu His Thr Leu Pro His Met Val Thr Asn Gly
   65 70 75 80
   Asn Val Leu Val Thr Ser Thr Ala Thr Thr Asn Thr Leu Pro Ser Thr
   85 90 95
   Thr Ala Ile Thr Ser Arg Pro Thr Phe Ser Pro Pro Glu Pro Thr Lys
   100 105 110
   Pro Pro Val Pro Lys Tyr Lys Arg Gly Lys Glu Leu Thr Phe Met Glu
   115 120 125
   Ser Gly Tyr Glu Lys Thr Phe Met Phe Ala Gln Ser Asn Thr Phe Ile
   130 135 140
   Gln Leu Asp Gly Ser Gly Ile Lys Pro Phe Gln Leu Arg Leu Cys Arg
   145                 150                 155                 160
   Glu Ile Ser Phe Lys Phe Arg Thr Lys Leu Pro His Gly Leu Leu Val
                   165                 170                 175
   Tyr His Ser Val Lys Asp Arg Pro Glu Ile Arg Leu Asp Pro Tyr Ala
               180                 185                 190
   Leu Tyr Val Ile Val Glu Lys Gly Gln Leu Lys Val Val His Val Phe
           195                 200                 205
   Gly Lys Arg Ser Thr Ser Leu Thr Val Gly Glu Gly Leu Asn Arg Asp
       210                 215                 220
   Glu Trp His Ser Val Leu Val Arg Ile Asp Val His Gly Ala Lys Leu
   225                 230                 235                 240
   Ile Ala Arg Val Asp Asp Lys Gln Glu Glu Thr Thr Leu Glu Val Leu
                   245                 250                 255
   Glu His Val Ile Asn Tyr Gly Val Ser Glu Glu Leu Ala Ser Val Val
               260                 265                 270
   Leu Ile Gly Gly Leu Ser Ser Glu Glu Arg Leu His Gly Val Lys Tyr
           275                 280                 285
   Ile Ile Glu Ser Phe Val Gly Cys Ile Arg Asp Met Val Leu Ser Ser
       290                 295                 300
   Gly Lys Ser Ala Ser Asp Leu Leu Pro Ile Gln Pro Leu Ile Ala Thr
   305                 310                 315                 320
   Lys His Glu Asn Val Arg Glu Gly Cys Ile Asp Lys Cys Arg Thr Arg
                   325                 330                 335
   Glu Asn Leu Cys Phe Ile Ser Ser Gln Cys Val Asn His Tyr Asn Ser
               340                 345                 350
   Leu Thr Cys Asp Cys Phe Gly Thr Lys Tyr Glu Gly Glu Arg Cys Asp
           355                 360                 365
   Val Tyr Thr Ala Thr Ile Leu Thr Leu Arg Gly Ser Ser Tyr Val Ser
       370                 375                 380
   Phe Arg Val Tyr Asp Trp Lys Asp Arg Val His Ser Ser Val Asn Arg
   385                 390                 395                 400
   Ile Ser Leu Ala Phe Lys Thr Lys Trp Asp Asp Ser Ala Leu Phe Tyr
   405 410 415
   Ala Ser Gly Glu Ile Asp Gly Thr Pro His Tyr Val Ala Ala Ser Ile
   420 425 430
   Val Asn Gly Ser Val His Ile Glu Leu Asp Phe Gly His Asn Ser Lys
   435 440 445
   Ile Ala Thr Val Leu Gly Asp Tyr Ile Thr Ser Asn His Trp Asn Asn
   450 455 460
   Leu Thr Ile Phe His Asn Gly Ser Leu Val Phe Val Ser Leu Asp Asp
   465 470 475 480
   Glu Ile Lys Val Leu Glu Ile Pro Gly Glu Asn Tyr Asn Met Ile Ile
   485 490 495
   Asp Pro Glu Ile Tyr Ile Gly Gly Gly Pro Glu Leu Tyr Lys Lys Lys
   500 505 510
   Gly Leu Ser Ser His Asn Asn Phe Ala Gly Ser Leu Lys Tyr Val Phe
   515 520 525
   Phe Asn Asp Lys Ser Ile Ile Tyr Glu Leu Lys Arg Ser Asn Pro Met
   530 535 540
   Val His Tyr Ile Gly Val Leu Glu Pro Glu Tyr Tyr Glu Ala Asp Val
   545 550 555 560
   Asp Val Ile Pro Ile Thr Tyr Pro Phe Ala Thr Ser His Ile Trp Trp
   565 570 575
   Pro Ile Ala Arg Thr Asp Ser Leu Lys Leu Asn Phe Asp Phe Lys Ser
   580 585 590
   Ser Lys Pro Val Ala Val Val Ala Ser Gly Asp Val Lys Cys Asn Arg
   595 600 605
   Ser Phe Gly Tyr Trp Glu Leu Gln Leu Val Asn Asp Glu Ile Arg Phe
   610 615 620
   Gln Leu Ile Pro Val Leu Gly Glu Asn Val Thr Val Thr Thr Ala Val
   625 630 635 640
   Lys Phe Pro Pro Tyr Asn Thr Ser Trp His Ala Val Glu Leu Asn Tyr
   645 650 655
   Thr Arg Gly Glu Ile Ser Val Leu Val Asp Tyr Arg Asn Lys Gln Ser
   660 665 670
   Lys Leu Phe Ser Met Thr Phe Glu Leu Gly Asp Lys Val Ile Ile Gly
   675 680 685
   Ser Gly Lys Asn Asn Thr Gly Leu Val Gly Cys Met Arg Glu Ile Arg
   690 695 700
   Val Asn Glu Glu Arg Ile Glu Pro Arg Tyr Val Met Asn Thr Asp Arg
   705 710 715 720
   Val Met Gly Glu Val Ala Leu Asp Asn Cys Gln Phe Ile Asp Pro Cys
   725 730 735
   Thr Arg Pro Asn Thr Cys Glu His Gly Gly Lys Cys Ser Val Lys Lys
   740 745 750
   Asp Glu Ile Ser Cys Asp Cys Thr Asp Thr Gly Tyr Val Gly Lys Asn
   755 760 765
   Cys His Phe Thr Lys Phe Arg Lys Thr Cys Glu Glu Leu Ala Leu Leu
   770 775 780
   Gly Tyr Thr Gln Asp Asp Val Tyr Lys Ile Asp Ile Asp Gly Asn Gly
   785 790 795 800
   Arg Phe Pro Pro Ala Leu Val Lys Cys Glu Phe Gln Ser Ile Glu Asn
   805 810 815
   Ser Thr Lys Thr Ile Val Glu His Asn Leu Pro Ser Gln Val Asp Val
   820 825 830
   Arg Ser Ile Ala Glu Ser Asp Phe Ser Phe Asn Ile Lys Tyr Arg Gln
   835 840 845
   Phe Thr Ala Glu Met Leu Gln Glu Leu Ile Ser His Ser Leu Tyr Cys
   850 855 860
   Ser Gln Tyr Val Lys Tyr Asp Cys Tyr Lys Ala Pro Leu Glu Leu His
   865 870 875 880
   Ser Ala Thr Trp Phe Leu Asn Ser Lys Gly Thr Thr Val Asp Tyr Ile
   885 890 895
   Gly Asn Val Asn Arg Gly Ser Cys Pro Cys Gly Met Asn Lys Thr Cys
   900 905 910
   Ile Asn Pro Asn Leu Ser Cys Asn Cys Asp Phe Thr Gly Asn Gly Asp
   915 920 925
   Lys Trp Leu Ser Asp Glu Gly Tyr Tyr Glu Thr Pro Asp Ser Leu Gly
   930 935 940
   Ile Thr Gly Met Val Phe Leu Gln Gln Arg Asp Leu Glu Glu Asp Ala
   945 950 955 960
   Gln Gly Arg Ile Thr Leu Gly Pro Leu Glu Cys Val Glu Thr Asn Thr
   965 970 975
   Gln Lys Tyr Val Val Thr Phe Thr Thr Ser Gln Ser Tyr Ile Glu Val
   980 985 990
   Pro Gly Trp Arg Lys Gly Asp Ile Ala Phe Ser Phe Arg Thr Thr Gly
   995 1000 1005
   Glu Lys Ala Ile Leu Leu Tyr Gln Pro Pro Ile Arg Asn Asn Tyr
   1010 1015 1020
   Pro Ser Phe Met Val Ala Leu Thr Ser Glu Tyr Arg Leu Thr Phe
   1025 1030 1035
   Asn Phe Thr Leu Ser Thr Gly Val Ile Lys Gly Ile Glu Val Glu
   1040 1045 1050
   Ser Lys Arg Lys Leu Asn Asn Gly Glu Trp Gln Lys Ile Trp Ile
   1055 1060 1065
   Asp Tyr Asn Asn Tyr His Val Arg Leu Met Ile Asn Thr Asp Ser
   1070 1075 1080
   Gln Met Leu Asp Leu Glu Asp Lys Phe Gly Pro Phe Glu Gly Ser
   1085 1090 1095
   Met Phe Ile Gly Gly Ala Thr Ala Glu His Leu Arg Thr Ser Ser
   1100 1105 1110
   Val Gln Gln Gly Leu Ile Gly Cys Phe Arg Gly Leu Val Val Asn
   1115 1120 1125
   Gly Glu Ile Leu Asp Ile His Ser Tyr Met Ser Val His Leu Ser
   1130 1135 1140
   Glu Ile Ile Lys Asp Cys Lys Pro Phe Cys Gln Pro Asn Lys Cys
   1145 1150 1155
   Gln Asn Gly Ala Arg Cys Val Glu Leu Trp Ser Asn Tyr Lys Cys
   1160 1165 1170
   Val Cys Glu Asn Lys Trp Ala His Phe Gly Asp Tyr Cys Glu Asn
   1175 1180 1185
   Asn Ile Asn Asn Glu Ala Leu Thr Phe Thr Ser Gln Ser Ala Phe
   1190 1195 1200
   Leu Lys Lys Asn Tyr Phe Gly Thr Asp Glu Lys Asp Glu Glu Lys
   1205 1210 1215
   Ser Leu Leu Lys Asn Ile Leu Met Lys Asn Ile Leu Ile Asn Leu
   1220 1225 1230
   Arg Thr Tyr Asp Thr Tyr Ser Ser Ile Leu Tyr Ala Asn Asp Asn
   1235 1240 1245
   Leu Asn Asn Phe Ala His Leu Tyr Ile Ser Asn Gly Thr Ser Ile
   1250 1255 1260
   Val Tyr Leu Phe Asn Ala Gly Asn Glu Ile Lys Asn Ile Thr Val
   1265 1270 1275
   Glu Tyr Pro Val Asn Thr Gly Ile Ser Ile Gln Ile Ala Ile Ile
   1280 1285 1290
   Arg Asn Glu Ser Ser Thr Thr Leu His Val Asn Glu Tyr Asn Ser
   1295 1300 1305
   Thr Leu Asn Ala Thr Leu Val Leu Leu Asp Thr Tyr Ser Asn Lys
   1310 1315 1320
   Pro Trp Ile Asn Pro Glu Lys Glu Val Leu Ala Pro Gln Arg Pro
   1325 1330 1335
   Pro Ala Ser Pro Thr Ser Tyr Phe Gln Val Asn Leu Gly Gly Phe
   1340 1345 1350
   Asp Pro Asp Asp Leu Leu Arg Val Gly Lys Glu Asp Val Gln Ile
   1355 1360 1365
   Lys Gly Tyr Val Gly Cys Leu Gly Gly Leu Met Ile Gly Glu Tyr
   1370 1375 1380
   Ala Val Asn Leu Ser Lys Leu Ala Asn Glu Ala Asn His Glu Gly
   1385 1390 1395
   Asn Lys Glu Ile Leu Pro Asn Cys Gln Thr Lys Cys Asp Ala Met
   1400 1405 1410
   Pro Cys Lys Asn Leu Gly Ile Cys Thr Glu Asp Phe Glu Arg Gln
   1415 1420 1425
   Glu Ser Leu Cys Asn Cys Glu Leu Thr Ser Tyr Phe Gly Glu His
   1430 1435 1440
   Cys Ala Asp Glu Lys Gly Ala Asp Phe Ser Gly Glu Ser Val Val
   1445 1450 1455
   Gln Arg Glu Phe Asp Leu Val Gly Lys Val Asn Gln Val Lys Val
   1460 1465 1470
   Gln Leu Ala Phe Ser Thr Asn Glu Leu Arg Gln Arg Thr Thr Ala
   1475 1480 1485
   Leu Leu Leu Leu Gln Thr Glu Asn Lys Arg Ser Tyr Tyr Leu Leu
   1490 1495 1500
   Val Ala Leu Thr Ser Glu Gly Gln Leu Ile Phe Glu Glu Asp Arg
   1505 1510 1515
   Glu Gly Ser Ala Phe Gly Val Arg Leu Asn Asp Ile Asn Phe Leu
   1520 1525 1530
   Asn Gly Ala Arg His Ser Ile Tyr Tyr Val Arg Asp Asn Ser Thr
   1535 1540 1545
   Val Thr Phe Leu Ile Asp Arg Glu Pro Val Thr Leu Leu Pro Ile
   1550 1555 1560
   Pro Ala Leu Thr Leu Gly Glu Asp Glu Ser Pro Gly Val Thr Glu
   1565 1570 1575
   Ile Gln Leu Gly Gly Leu Asn Thr Thr Asp Ser Arg Phe Ser Ala
   1580 1585 1590
   Tyr Lys Gly Tyr Thr Gly Cys Leu Ser Asn Val Val Val Ser Ile
   1595 1600 1605
   Asn Gly Gly Pro Asp Met Lys Pro Leu Glu Glu Tyr Met Leu Phe
   1610 1615 1620
   Thr Lys Gln Gly Ser Glu Thr Val Arg Ala Thr Met Pro Ala Gly
   1625 1630 1635
   Val Arg Ser Ala Gln Cys Ala Val Phe His Val Gln Pro His Gly
   1640 1645 1650
   Leu Asp Pro Pro Lys Asn Asp Ser Asn Val Gly Val Asp Lys Val
   1655 1660 1665
   Trp Val Glu Asp Pro Pro Glu Arg Ile Pro Tyr Lys Ser Gln Tyr
   1670 1675 1680
   Thr Asn Ala Thr Gln Glu Glu Gln Gly Ala Gly Thr Tyr Ile Phe
   1685 1690 1695
   Ile Ala Leu Cys Cys Val Phe Val Thr Ala Val Ile Gly Cys Ile
   1700 1705 1710
   Tyr Glu Val Trp Arg Ser Ala Arg Lys Asp Arg Arg Arg Arg Arg
   1715 1720 1725
   Ala Ser Val Ala Ser Thr Val Pro Ala Pro Gly Ser Gln Arg Trp
   1730 1735 1740
   Gln Ser Gln Gln Tyr Thr Asp Pro Leu Thr Gly Gly Val Lys Thr
   1745 1750 1755
   Val Gly Phe Lys Asn Val Ala Glu Asp Glu Lys Arg Pro Asn Gly
   1760 1765 1770
   Thr His Met Lys Ala Ala Val Lys Glu Tyr Lys Pro Leu Ala Asn
   1775 1780 1785
   Ala Glu Ser Lys Glu Leu Ile Asn Asp Lys Arg Val His Ile Ile
   1790 1795 1800
   Ala Asp Glu Glu Pro Glu Lys Lys Glu Leu Leu Gly Val Asn Thr
   1805 1810 1815
   Gly Ile Ile Thr Lys Ser Pro Lys Leu Asn Pro Phe Ser Met Glu
   1820 1825 1830
   Asp Leu Gln Glu Glu Pro Glu Leu Glu Glu Cys Glu Glu Glu Glu
   1835 1840 1845
   Pro Asp Glu Glu Asp Glu Asp Glu Asp Glu Asp Asn Pro Lys Glu
   1850 1855 1860
   Gln Asn Thr Asp Glu Glu Glu Ile His Gln Lys Thr Glu Arg Thr
   1865 1870 1875
   Asp Asp Asn Asp Phe Val Lys Ser Pro Glu Asn Ser Ser Arg Ile
   1880 1885 1890
   Ser Val Glu Glu Ser Ile Asp Ser Leu Asn Asn Asn Pro Lys His
   1895 1900 1905
   Asp Glu Thr Lys Thr Gln Tyr Glu Val
   1910 1915
<210> 21
   <211> 1203
   <212> PRT
   <213> Apis mellifera
<400> 21
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Thr Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145 150 155 160
   Leu Ala Tyr Arg Val Arg Val Gln Cys Ala Asp His Tyr Tyr Asn Ala
   165 170 175
   Thr Cys Thr Lys Phe Cys Arg Pro Arg Asn Asp Ile Phe Gly His Tyr
   180 185 190
   Thr Cys Asp Glu Asn Gly Asp Lys Val Cys Ile Gln Gly Trp Lys Gly
   195 200 205
   Ala Asp Cys Glu Ile Ala Val Cys Lys Glu Gly Cys His Pro Val His
   210 215 220
   Gly His Cys Asn Val Ser Gly Glu Cys Lys Cys Arg His Gly Trp Arg
   225 230 235 240
   Gly Glu Leu Cys Asp Gln Cys Thr Pro Tyr Pro Gly Cys Lys His Gly
   245 250 255
   Tyr Cys Asn Gly Ser Ser Trp Gln Cys Ile Cys Asp Thr Asn Trp Gly
   260 265 270
   Gly Ile Leu Cys Asp Gln Asp Leu Asn Tyr Cys Gly Thr His Glu Pro
   275 280 285
   Cys Gln Asn Gly Gly Thr Cys Glu Asn Thr Ala Pro Asp Gln Tyr Lys
   290 295 300
   Cys Thr Cys Pro Glu Gly Phe Ser Gly Pro Thr Cys Glu Lys Val Asp
   305 310 315 320
   Asn Pro Cys Ala Ser Asn Pro Cys Ala Asn Gly Ala Thr Cys Arg Glu
   325 330 335
   Leu Gly Glu Ser Ala His Cys Glu Cys Ala Pro Gly Phe Ser Gly Pro
   340 345 350
   Tyr Cys Ala Thr Asp Ile Asp Glu Cys Ala Ser Gln Pro Cys Gln Asn
   355 360 365
   Gly Gly Thr Cys Val Asp Gly Lys Asn Glu Phe Val Cys Asn Cys Pro
   370 375 380
   Pro Ala Trp Gln Gly Val Leu Cys Gln Phe Asp Val Asp Glu Cys Ala
   385 390 395 400
   Leu Lys Glu Ser Pro Cys Lys Asn Ser Leu Thr Cys Val Asn Leu Ala
   405 410 415
   Gly Asp Tyr Lys Cys Arg Cys Arg Ser Gly Phe Thr Gly Lys Asn Cys
   420 425 430
   Thr Lys Asn Ile Asn Asp Cys Ile Gly Gln Cys Gln His Gly Ala Leu
   435 440 445
   Cys Ile Asp Leu Val Asp Asp Tyr His Cys Ser Cys Thr Ala Gly Tyr
   450 455 460
   Ser Gly Lys Asp Cys Asp Val Asp Ile Asp Glu Cys Ala Ser Lys Pro
   465 470 475 480
   Cys Gln Asn Gly Gly Glu Cys Arg Asp Leu Val Asn Ala Tyr Glu Cys
   485 490 495
   Val Cys Pro Val Gly Phe Thr Gly Tyr Gln Cys Glu Ile Asp Lys Asp
   500 505 510
   His Cys Ser Pro Asn Pro Cys Arg Asn Ser Ala Pro Cys Phe Asn Thr
   515 520 525
   Gln Thr Asp Tyr Tyr Cys His Cys Pro Glu Gln Trp Gln Gly Lys Asn
   530 535 540
   Cys Ser Glu Pro Ala Ser His Asn Pro Gln Phe Gly Met Met Asp Glu
   545 550 555 560
   Glu Ser Gly Cys Gly Ser Glu Gly Thr Pro Cys Gly Gly Arg Gly Arg
   565 570 575
   Cys Ser Gly Gly Arg Cys Ile Cys Asp Ala Gly Tyr Thr Gly Met His
   580 585 590
   Cys His Glu Asn Ile Asn Asp Cys Arg Gly Asn Pro Cys Leu Asn Gly
   595 600 605
   Gly Thr Cys Val Asp Leu Val Asn Ser Phe Gln Cys Ile Cys Arg Glu
   610 615 620
   Gly Trp Thr Gly Asp Leu Cys Asp Gln Asp Val Asp Glu Cys Thr Asn
   625 630 635 640
   Ser Pro Cys Arg Asn Asn Gly Thr Cys Val Asp Gly Val Ala Asp Phe
   645 650 655
   Thr Cys Ile Cys Arg Gly Gly Trp Lys Gly Lys Thr Cys Thr Leu Arg
   660 665 670
   Ala Gly His Cys Glu Pro Gly Thr Cys Arg His Gly Gly Thr Cys Gln
   675 680 685
   Asp Arg Gly Asp Gly Phe Thr Cys His Cys Pro Pro Gly Trp Glu Gly
   690 695 700
   Ala Ala Cys His Ile Ala Ser Pro Ala Cys Ala Ser Asn Pro Cys Glu
   705 710 715 720
   Asn Gly Ala Thr Cys Val Asn Thr Ala Asp Gly Asn Tyr Arg Cys Val
   725 730 735
   Cys Arg Glu Gly Phe Glu Gly Pro Asn Cys Arg Arg Asp Val Asp Asp
   740 745 750
   Cys Gln Pro Leu Pro Cys Leu Asn Gly Gly Lys Cys Val Asp Gly Ile
   755 760 765
   Asn Trp Phe Arg Cys Glu Cys Ala Pro Gly Phe Thr Gly Pro Asp Cys
   770 775 780
   Arg Ile Asn Val Asn Glu Cys Ala Ser Asp Pro Cys Thr Thr Gly Ser
   785 790 795 800
   Thr Cys Val Asp Gly Ile Ala Ser Tyr Thr Cys Ile Cys Pro Pro Gly
   805 810 815
   Arg Thr Gly Val Arg Cys Glu Ile Arg Thr Ala Gly Gly Pro Gly Cys
   820 825 830
   Pro Val Ala Ser Trp Asp Asp Asp Cys Asn Val Cys Glu Cys Arg Asn
   835 840 845
   Gly Lys Asn Gln Cys Ser Asn Ile Trp Cys Gly Pro Gly Asn Cys Leu
   850 855 860
   Asn Gly Thr Ser Cys Leu Ala His Glu Val Cys Val Pro Ser Pro Asn
   865 870 875 880
   Glu Ser Cys Leu Ala Pro Pro Cys Pro Ala Trp Gly Glu Cys Arg Pro
   885 890 895
   Val Glu Thr Gly Arg Arg Val Gly Pro Pro Ala Leu Pro Ala Pro Pro
   900 905 910
   Ser Cys Trp Pro Gly Gln Ala Thr Pro Gly Pro Thr Cys Ser Arg Leu
           915                 920                 925
   Thr Ile Leu Leu Arg Arg Asp Thr Leu Ala Ala Ser Thr Ser Val Glu
       930                 935                 940
   Ile Leu Cys Arg Arg Leu Arg Lys Leu Leu Ser Asp Pro Arg Arg Pro
   945                 950                 955                 960
   Gln Ser Ile Val Leu Leu Cys Asp Leu Lys Pro Gly Asp Asn Asp Thr
                   965                 970                 975
   Val Glu Val Thr Ile Phe Ser Glu Ala Ala Ala Asp Ala Ala Arg Asp
               980                 985                 990
   Leu Gly Glu Ala Leu Ser Arg Pro  Leu Gly Arg Pro Leu  Ala Leu Ala
           995                 1000                1005
   Ser Val  Leu Glu Val Lys Val  Glu Thr Ala Leu Leu  Ser Glu Pro
       1010                 1015                 1020
   Ser Ser  Val Asn Ser Ser Ser  Ala Gly Ser Tyr Val  Ala Val Leu
       1025                 1030                 1035
   Gly Gly  Ala Leu Ala Thr Val  Leu Val Leu Ala Leu  Phe Gly Gly
       1040                 1045                 1050
   Leu Trp  Tyr Leu Arg Thr Val  Arg Gln Arg Ser Ser  Leu Thr Ala
       1055                 1060                 1065
   Thr Thr  Ser Ser Glu Thr Ser  Leu His Arg His Arg  Ser Asp Leu
       1070                 1075                 1080
   Asp Glu  Lys Ser Asn Asn Leu  Gln Asn Glu Glu Asn  Leu Arg Arg
       1085                 1090                 1095
   Tyr Ala  Asn Pro Leu Lys Glu  Gln Asp Gln Gly Glu  Pro Arg Val
       1100                 1105                 1110
   Ser Val  Val Arg Pro Leu Ser  Gly Thr Ser Leu Gly  Ala Leu Gly
       1115                 1120                 1125
   Ala Thr  Glu Glu Ser Leu Glu  Met Val Ser Asp Glu  Ser Arg His
       1130                 1135                 1140
   Arg Leu  Pro Pro Leu Tyr Lys  Ala Pro Ser Ala Glu  Ala Arg Asn
   1145 1150 1155
   Asn Thr Ala Ser Phe Thr Tyr Glu Glu Gly Pro His Lys Pro Tyr
   1160 1165 1170
   Ser Lys Pro Arg Leu Gln Glu Pro Thr Tyr Ser Gln Gln Ala Ser
   1175 1180 1185
   Ser Ser Gln Thr Ser Gly Pro His Gln Val Leu Thr Val His Val
   1190 1195 1200
<210> 22
   <211> 1203
   <212> PRT
   <213> Apis mellifera
<400> 22
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Ala Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145                 150                 155                 160
   Leu Ala Tyr Arg Val Arg Val Gln Cys Ala Asp His Tyr Tyr Asn Ala
   165 170 175
   Thr Cys Thr Lys Phe Cys Arg Pro Arg Asn Asp Ile Phe Gly His Tyr
   180 185 190
   Thr Cys Asp Glu Asn Gly Asp Lys Val Cys Ile Gln Gly Trp Lys Gly
   195 200 205
   Ala Asp Cys Glu Ile Ala Val Cys Lys Glu Gly Cys His Pro Val His
   210 215 220
   Gly His Cys Asn Val Ser Gly Glu Cys Lys Cys Arg His Gly Trp Arg
   225 230 235 240
   Gly Glu Leu Cys Asp Gln Cys Thr Pro Tyr Pro Gly Cys Lys His Gly
   245 250 255
   Tyr Cys Asn Gly Ser Ser Trp Gln Cys Ile Cys Asp Thr Asn Trp Gly
   260 265 270
   Gly Ile Leu Cys Asp Gln Asp Leu Asn Tyr Cys Gly Thr His Glu Pro
   275 280 285
   Cys Gln Asn Gly Gly Thr Cys Glu Asn Thr Ala Pro Asp Gln Tyr Lys
   290 295 300
   Cys Thr Cys Pro Glu Gly Phe Ser Gly Pro Thr Cys Glu Lys Val Asp
   305 310 315 320
   Asn Pro Cys Ala Ser Asn Pro Cys Ala Asn Gly Ala Thr Cys Arg Glu
   325 330 335
   Leu Gly Glu Ser Ala His Cys Glu Cys Ala Pro Gly Phe Ser Gly Pro
   340 345 350
   Tyr Cys Ala Thr Asp Ile Asp Glu Cys Ala Ser Gln Pro Cys Gln Asn
   355 360 365
   Gly Gly Thr Cys Val Asp Gly Lys Asn Glu Phe Val Cys Asn Cys Pro
   370 375 380
   Pro Ala Trp Gln Gly Val Leu Cys Gln Phe Asp Val Asp Glu Cys Ala
   385 390 395 400
   Leu Lys Glu Ser Pro Cys Lys Asn Ser Leu Thr Cys Val Asn Leu Ala
   405 410 415
   Gly Asp Tyr Lys Cys Arg Cys Arg Ser Gly Phe Thr Gly Lys Asn Cys
   420 425 430
   Thr Lys Asn Ile Asn Asp Cys Ile Gly Gln Cys Gln His Gly Ala Leu
   435 440 445
   Cys Ile Asp Leu Val Asp Asp Tyr His Cys Ser Cys Thr Ala Gly Tyr
   450 455 460
   Ser Gly Lys Asp Cys Asp Val Asp Ile Asp Glu Cys Ala Ser Lys Pro
   465 470 475 480
   Cys Gln Asn Gly Gly Glu Cys Arg Asp Leu Val Asn Ala Tyr Glu Cys
   485 490 495
   Val Cys Pro Val Gly Phe Thr Gly Tyr Gln Cys Glu Ile Asp Lys Asp
   500 505 510
   His Cys Ser Pro Asn Pro Cys Arg Asn Ser Ala Pro Cys Phe Asn Thr
   515 520 525
   Gln Thr Asp Tyr Tyr Cys His Cys Pro Glu Gln Trp Gln Gly Lys Asn
   530 535 540
   Cys Ser Glu Pro Ala Ser His Asn Pro Gln Phe Gly Met Met Asp Glu
   545 550 555 560
   Glu Ser Gly Cys Gly Ser Glu Gly Thr Pro Cys Gly Gly Arg Gly Arg
   565 570 575
   Cys Ser Gly Gly Arg Cys Ile Cys Asp Ala Gly Tyr Thr Gly Met His
   580 585 590
   Cys His Glu Asn Ile Asn Asp Cys Arg Gly Asn Pro Cys Leu Asn Gly
   595 600 605
   Gly Thr Cys Val Asp Leu Val Asn Ser Phe Gln Cys Ile Cys Arg Glu
   610 615 620
   Gly Trp Thr Gly Asp Leu Cys Asp Gln Asp Val Asp Glu Cys Thr Asn
   625 630 635 640
   Ser Pro Cys Arg Asn Asn Gly Thr Cys Val Asp Gly Val Ala Asp Phe
   645 650 655
   Thr Cys Ile Cys Arg Gly Gly Trp Lys Gly Lys Thr Cys Thr Leu Arg
   660 665 670
   Ala Gly His Cys Glu Pro Gly Thr Cys Arg His Gly Gly Thr Cys Gln
           675                 680                 685
   Asp Arg Gly Asp Gly Phe Thr Cys His Cys Pro Pro Gly Trp Glu Gly
       690                 695                 700
   Ala Ala Cys His Ile Ala Ser Pro Ala Cys Ala Ser Asn Pro Cys Glu
   705                 710                 715                 720
   Asn Gly Ala Thr Cys Val Asn Thr Ala Asp Gly Asn Tyr Arg Cys Val
                   725                 730                 735
   Cys Arg Glu Gly Phe Glu Gly Pro Asn Cys Arg Arg Asp Val Asp Asp
               740                 745                 750
   Cys Gln Pro Leu Pro Cys Leu Asn Gly Gly Lys Cys Val Asp Gly Ile
           755                 760                 765
   Asn Trp Phe Arg Cys Glu Cys Ala Pro Gly Phe Thr Gly Pro Asp Cys
       770                 775                 780
   Arg Ile Asn Val Asn Glu Cys Ala Ser Asp Pro Cys Thr Thr Gly Ser
   785                 790                 795                 800
   Thr Cys Val Asp Gly Ile Ala Ser Tyr Thr Cys Ile Cys Pro Pro Gly
                   805                 810                 815
   Arg Thr Gly Val Arg Cys Glu Ile Arg Thr Ala Gly Gly Pro Gly Cys
               820                 825                 830
   Pro Val Ala Ser Trp Asp Asp Asp Cys Asn Val Cys Glu Cys Arg Asn
           835                 840                 845
   Gly Lys Asn Gln Cys Ser Asn Ile Trp Cys Gly Pro Gly Asn Cys Leu
       850                 855                 860
   Asn Gly Thr Ser Cys Leu Ala His Glu Val Cys Val Pro Ser Pro Asn
   865                 870                 875                 880
   Glu Ser Cys Leu Ala Pro Pro Cys Pro Ala Trp Gly Glu Cys Arg Pro
                   885                 890                 895
   Val Glu Thr Gly Arg Arg Val Gly Pro Pro Ala Leu Pro Ala Pro Pro
               900                 905                 910
   Ser Cys Trp Pro Gly Gln Ala Thr Pro Gly Pro Thr Cys Ser Arg Leu
           915                 920                 925
   Thr Ile Leu Leu Arg Arg Asp Thr Leu Ala Ala Ser Thr Ser Val Glu
   930 935 940
   Ile Leu Cys Arg Arg Leu Arg Lys Leu Leu Ser Asp Pro Arg Arg Pro
   945 950 955 960
   Gln Ser Ile Val Leu Leu Cys Asp Leu Lys Pro Gly Asp Asn Asp Thr
   965 970 975
   Val Glu Val Thr Ile Phe Ser Glu Ala Ala Ala Asp Ala Ala Arg Asp
   980 985 990
   Leu Gly Glu Ala Leu Ser Arg Pro Leu Gly Arg Pro Leu Ala Leu Ala
   995 1000 1005
   Ser Val Leu Glu Val Lys Val Glu Thr Ala Leu Leu Ser Glu Pro
   1010 1015 1020
   Ser Ser Val Asn Ser Ser Ser Ala Gly Ser Tyr Val Ala Val Leu
   1025 1030 1035
   Gly Gly Ala Leu Ala Thr Val Leu Val Leu Ala Leu Phe Gly Gly
   1040 1045 1050
   Leu Trp Tyr Leu Arg Thr Val Arg Gln Arg Ser Ser Leu Thr Ala
   1055 1060 1065
   Thr Thr Ser Ser Glu Thr Ser Leu His Arg His Arg Ser Asp Leu
   1070 1075 1080
   Asp Glu Lys Ser Asn Asn Phe Gln Asn Glu Glu Asn Leu Arg Arg
   1085 1090 1095
   Tyr Ala Asn Pro Leu Lys Glu Gln Asp Gln Gly Glu Pro Arg Val
   1100 1105 1110
   Ser Val Val Arg Pro Leu Ser Gly Thr Ser Leu Gly Ala Leu Gly
   1115 1120 1125
   Ala Thr Glu Glu Ser Leu Glu Met Val Ser Asp Glu Ser Arg His
   1130 1135 1140
   Arg Leu Pro Pro Leu Tyr Lys Ala Pro Ser Ala Glu Ala Arg Asn
   1145 1150 1155
   Asn Thr Ala Ser Phe Thr Tyr Glu Glu Gly Pro His Lys Pro Tyr
   1160 1165 1170
   Ser Lys Pro Arg Leu Gln Glu Pro Thr Tyr Ser Gln Gln Ala Ser
   1175 1180 1185
   Ser Ser Gln Thr Ser Gly Pro His Gln Val Leu Thr Val His Val
   1190 1195 1200
<210> 23
   <211> 1201
   <212> PRT
   <213> Apis mellifera
<400> 23
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Ala Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145                 150                 155                 160
   Leu Ala Tyr Arg Val Arg Val Gln Cys Ala Asp His Tyr Tyr Asn Ala
                   165                 170                 175
   Thr Cys Thr Lys Phe Cys Arg Pro Arg Asn Asp Ile Phe Gly His Tyr
   180 185 190
   Thr Cys Asp Glu Asn Gly Asp Lys Val Cys Ile Gln Gly Trp Lys Gly
   195 200 205
   Ala Asp Cys Glu Ile Ala Val Cys Lys Glu Gly Cys His Pro Val His
   210 215 220
   Gly His Cys Asn Val Ser Gly Glu Cys Lys Cys Arg His Gly Trp Arg
   225 230 235 240
   Gly Glu Leu Cys Asp Gln Cys Thr Pro Tyr Pro Gly Cys Lys His Gly
   245 250 255
   Tyr Cys Asn Gly Ser Ser Trp Gln Cys Ile Cys Asp Thr Asn Trp Gly
   260 265 270
   Gly Ile Leu Cys Asp Gln Asp Leu Asn Tyr Cys Gly Thr His Glu Pro
   275 280 285
   Cys Gln Asn Gly Gly Thr Cys Glu Asn Thr Ala Pro Asp Gln Tyr Lys
   290 295 300
   Cys Thr Cys Pro Glu Gly Phe Ser Gly Pro Thr Cys Glu Lys Val Asp
   305 310 315 320
   Asn Pro Cys Ala Ser Asn Pro Cys Ala Asn Gly Ala Thr Cys Arg Glu
   325 330 335
   Leu Gly Glu Ser Ala His Cys Glu Cys Ala Pro Gly Phe Ser Gly Pro
   340 345 350
   Tyr Cys Ala Thr Asp Ile Asp Glu Cys Ala Ser Gln Pro Cys Gln Asn
   355 360 365
   Gly Gly Thr Cys Val Asp Gly Lys Asn Glu Phe Val Cys Asn Cys Pro
   370 375 380
   Pro Ala Trp Gln Gly Val Leu Cys Gln Phe Asp Val Asp Glu Cys Ala
   385 390 395 400
   Leu Lys Glu Ser Pro Cys Lys Asn Ser Leu Thr Cys Val Asn Leu Ala
   405 410 415
   Gly Asp Tyr Lys Cys Arg Cys Arg Ser Gly Phe Thr Gly Lys Asn Cys
   420 425 430
   Thr Lys Asn Ile Asn Asp Cys Ile Gly Gln Cys Gln His Gly Ala Leu
           435                 440                 445
   Cys Ile Asp Leu Val Asp Asp Tyr His Cys Ser Cys Thr Ala Gly Tyr
       450                 455                 460
   Ser Gly Lys Asp Cys Asp Val Asp Ile Asp Glu Cys Ala Ser Lys Pro
   465                 470                 475                 480
   Cys Gln Asn Gly Gly Glu Cys Arg Asp Leu Val Asn Ala Tyr Glu Cys
                   485                 490                 495
   Val Cys Pro Val Gly Phe Thr Gly Tyr Gln Cys Glu Ile Asp Lys Asp
               500                 505                 510
   His Cys Ser Pro Asn Pro Cys Arg Asn Ser Ala Pro Cys Phe Asn Thr
           515                 520                 525
   Gln Thr Asp Tyr Tyr Cys His Cys Pro Glu Gln Trp Gln Gly Lys Asn
       530                 535                 540
   Cys Ser Glu Pro Ala Ser His Asn Pro Gln Phe Gly Met Met Asp Glu
   545                 550                 555                 560
   Glu Ser Gly Cys Gly Ser Glu Gly Thr Pro Cys Gly Gly Arg Gly Arg
                   565                 570                 575
   Cys Ser Gly Gly Arg Cys Ile Cys Asp Ala Gly Tyr Thr Gly Met His
               580                 585                 590
   Cys His Glu Asn Ile Asn Asp Cys Arg Gly Asn Pro Cys Leu Asn Gly
           595                 600                 605
   Gly Thr Cys Val Asp Leu Val Asn Ser Phe Gln Cys Ile Cys Arg Glu
       610                 615                 620
   Gly Trp Thr Gly Asp Leu Cys Asp Gln Asp Val Asp Glu Cys Thr Asn
   625                 630                 635                 640
   Ser Pro Cys Arg Asn Asn Gly Thr Cys Val Asp Gly Val Ala Asp Phe
                   645                 650                 655
   Thr Cys Ile Cys Arg Gly Gly Trp Lys Gly Lys Thr Cys Thr Leu Arg
               660                 665                 670
   Ala Gly His Cys Glu Pro Gly Thr Cys Arg His Gly Gly Thr Cys Gln
           675                 680                 685
   Asp Arg Gly Asp Gly Phe Thr Cys His Cys Pro Pro Gly Trp Glu Gly
       690                 695                 700
   Ala Ala Cys His Ile Ala Ser Pro Ala Cys Ala Ser Asn Pro Cys Glu
   705                 710                 715                 720
   Asn Gly Ala Thr Cys Val Asn Thr Ala Asp Gly Asn Tyr Arg Cys Val
                   725                 730                 735
   Cys Arg Glu Gly Phe Glu Gly Pro Asn Cys Arg Arg Asp Val Asp Asp
               740                 745                 750
   Cys Gln Pro Leu Pro Cys Leu Asn Gly Gly Lys Cys Val Asp Gly Ile
           755                 760                 765
   Asn Trp Phe Arg Cys Glu Cys Ala Pro Gly Phe Thr Gly Pro Asp Cys
       770                 775                 780
   Arg Ile Asn Val Asn Glu Cys Ala Ser Asp Pro Cys Thr Thr Gly Ser
   785                 790                 795                 800
   Thr Cys Val Asp Gly Ile Ala Ser Tyr Thr Cys Ile Cys Pro Pro Gly
                   805                 810                 815
   Arg Thr Gly Val Arg Cys Glu Ile Arg Thr Ala Gly Gly Pro Gly Cys
               820                 825                 830
   Pro Val Ala Ser Trp Asp Asp Asp Cys Asn Val Cys Glu Cys Arg Asn
           835                 840                 845
   Gly Lys Asn Gln Cys Ser Asn Ile Trp Cys Gly Pro Gly Asn Cys Leu
       850                 855                 860
   Asn Gly Thr Ser Cys Leu Ala His Glu Val Cys Val Pro Ser Pro Asn
   865                 870                 875                 880
   Glu Ser Cys Leu Ala Pro Pro Cys Pro Ala Trp Gly Glu Cys Arg Pro
                   885                 890                 895
   Val Glu Thr Gly Arg Arg Val Gly Pro Pro Ala Leu Pro Ala Pro Pro
               900                 905                 910
   Ser Cys Trp Pro Gly Gln Ala Thr Pro Gly Pro Thr Cys Ser Arg Leu
           915                 920                 925
   Thr Ile Leu Leu Arg Arg Asp Thr Leu Ala Ala Ser Thr Ser Val Glu
       930                 935                 940
   Ile Leu Cys Arg Arg Leu Arg Lys Leu Leu Ser Asp Pro Arg Arg Pro
   945                 950                 955                 960
   Gln Ser Ile Val Leu Leu Cys Asp Leu Lys Pro Gly Asp Asn Asp Thr
                   965                 970                 975
   Val Glu Val Thr Ile Phe Ser Glu Ala Ala Ala Asp Ala Ala Arg Asp
               980                 985                 990
   Leu Gly Glu Ala Leu Ser Arg Pro  Leu Gly Arg Pro Leu  Ala Leu Ala
           995                 1000                1005
   Ser Val  Leu Glu Val Lys Val  Glu Thr Ala Leu Leu  Ser Glu Pro
       1010                1015                1020
   Ser Ser  Val Asn Ser Ser Ser  Ala Gly Ser Tyr Val  Ala Val Leu
       1025                1030                1035
   Gly Gly  Ala Leu Ala Thr Val  Leu Val Leu Ala Leu  Phe Gly Gly
       1040                1045                1050
   Leu Trp  Tyr Leu Arg Thr Val  Arg Gln Arg Ser Ser  Leu Thr Ala
       1055                1060                1065
   Thr Thr  Ser Ser Glu Thr Ser  Leu His Arg His Arg  Ser Asp Leu
       1070                1075                1080
   Asp Glu  Lys Ser Asn Asn Phe  Gln Asn Glu Glu Asn  Leu Arg Arg
       1085                1090                1095
   Asn Pro  Leu Lys Glu Gln Asp  Gln Gly Glu Pro Arg  Val Ser Val
       1100                1105                1110
   Val Arg  Pro Leu Ser Gly Thr  Ser Leu Gly Ala Leu  Gly Ala Thr
       1115                1120                1125
   Glu Glu  Ser Leu Glu Met Val  Ser Asp Glu Ser Arg  His Arg Leu
       1130                1135                1140
   Pro Pro  Leu Tyr Lys Ala Pro  Ser Ala Glu Ala Arg  Asn Asn Thr
       1145                1150                1155
   Ala Ser  Phe Thr Tyr Glu Glu  Gly Pro His Lys Pro  Tyr Ser Lys
       1160                1165                1170
   Pro Arg  Leu Gln Glu Pro Thr  Tyr Ser Gln Gln Ala  Ser Ser Ser
   1175 1180 1185
   Gln Thr Ser Gly Pro His Gln Val Leu Thr Val His Val
   1190 1195 1200
<210> 24
   <211> 1203
   <212> PRT
   <213> Apis mellifera
<400> 24
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Ala Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145                 150                 155                 160
   Leu Ala Tyr Arg Val Arg Val Gln Cys Ala Asp His Tyr Tyr Asn Ala
                   165                 170                 175
   Thr Cys Thr Lys Phe Cys Arg Pro Arg Asn Asp Ile Phe Gly His Tyr
               180                 185                 190
   Thr Cys Asp Glu Asn Gly Asp Lys Val Cys Ile Gln Gly Trp Lys Gly
   195 200 205
   Ala Asp Cys Glu Ile Ala Val Cys Lys Glu Gly Cys His Pro Val His
   210 215 220
   Gly His Cys Asn Val Ser Gly Glu Cys Lys Cys Arg His Gly Trp Arg
   225 230 235 240
   Gly Glu Leu Cys Asp Gln Cys Thr Pro Tyr Pro Gly Cys Lys His Gly
   245 250 255
   Tyr Cys Asn Gly Ser Ser Trp Gln Cys Ile Cys Asp Thr Asn Trp Gly
   260 265 270
   Gly Ile Leu Cys Asp Gln Asp Leu Asn Tyr Cys Gly Thr His Glu Pro
   275 280 285
   Cys Gln Asn Gly Gly Thr Cys Glu Asn Thr Ala Pro Asp Gln Tyr Lys
   290 295 300
   Cys Thr Cys Pro Glu Gly Phe Ser Gly Pro Thr Cys Glu Lys Val Asp
   305 310 315 320
   Asn Pro Cys Ala Ser Asn Pro Cys Ala Asn Gly Ala Thr Cys Arg Glu
   325 330 335
   Leu Gly Glu Ser Ala His Cys Glu Cys Ala Pro Gly Phe Ser Gly Pro
   340 345 350
   Tyr Cys Ala Thr Asp Ile Asp Glu Cys Ala Ser Gln Pro Cys Gln Asn
   355 360 365
   Gly Gly Thr Cys Val Asp Gly Lys Asn Glu Phe Val Cys Asn Cys Pro
   370 375 380
   Pro Ala Trp Gln Gly Val Leu Cys Gln Phe Asp Val Asp Glu Cys Ala
   385 390 395 400
   Leu Lys Glu Ser Pro Cys Lys Asn Ser Leu Thr Cys Val Asn Leu Ala
   405 410 415
   Gly Asp Tyr Lys Cys Arg Cys Arg Ser Gly Phe Thr Gly Lys Asn Cys
   420 425 430
   Thr Lys Asn Ile Asn Asp Cys Ile Gly Gln Cys Gln His Gly Ala Leu
   435 440 445
   Cys Ile Asp Leu Val Asp Asp Tyr His Cys Ser Cys Thr Ala Gly Tyr
       450                 455                 460
   Ser Gly Lys Asp Cys Asp Val Asp Ile Asp Glu Cys Ala Ser Lys Pro
   465                 470                 475                 480
   Cys Gln Asn Gly Gly Glu Cys Arg Asp Leu Val Asn Ala Tyr Glu Cys
                   485                 490                 495
   Val Cys Pro Val Gly Phe Thr Gly Tyr Gln Cys Glu Ile Asp Lys Asp
               500                 505                 510
   His Cys Ser Pro Asn Pro Cys Arg Asn Ser Ala Pro Cys Phe Asn Thr
           515                 520                 525
   Gln Thr Asp Tyr Tyr Cys His Cys Pro Glu Gln Trp Gln Gly Lys Asn
       530                 535                 540
   Cys Ser Glu Pro Ala Ser His Asn Pro Gln Phe Gly Met Met Asp Glu
   545                 550                 555                 560
   Glu Ser Gly Cys Gly Ser Glu Gly Thr Pro Cys Gly Gly Arg Gly Arg
                   565                 570                 575
   Cys Ser Gly Gly Arg Cys Ile Cys Asp Ala Gly Tyr Thr Gly Met His
               580                 585                 590
   Cys His Glu Asn Ile Asn Asp Cys Arg Gly Asn Pro Cys Leu Asn Gly
           595                 600                 605
   Gly Thr Cys Val Asp Leu Val Asn Ser Phe Gln Cys Ile Cys Arg Glu
       610                 615                 620
   Gly Trp Thr Gly Asp Leu Cys Asp Gln Asp Val Asp Glu Cys Thr Asn
   625                 630                 635                 640
   Ser Pro Cys Arg Asn Asn Gly Thr Cys Val Asp Gly Val Ala Asp Phe
                   645                 650                 655
   Thr Cys Ile Cys Arg Gly Gly Trp Lys Gly Lys Thr Cys Thr Leu Arg
               660                 665                 670
   Ala Gly His Cys Glu Pro Gly Thr Cys Arg His Gly Gly Thr Cys Gln
           675                 680                 685
   Asp Arg Gly Asp Gly Phe Thr Cys His Cys Pro Pro Gly Trp Glu Gly
       690                 695                 700
   Ala Ala Cys His Ile Ala Ser Pro Ala Cys Ala Ser Asn Pro Cys Glu
   705 710 715 720
   Asn Gly Ala Thr Cys Val Asn Thr Ala Asp Gly Asn Tyr Arg Cys Val
   725 730 735
   Cys Arg Glu Gly Phe Glu Gly Pro Asn Cys Arg Arg Asp Val Asp Asp
   740 745 750
   Cys Gln Pro Leu Pro Cys Leu Asn Gly Gly Lys Cys Val Asp Gly Ile
   755 760 765
   Asn Trp Phe Arg Cys Glu Cys Ala Pro Gly Phe Thr Gly Pro Asp Cys
   770 775 780
   Arg Ile Asn Val Asn Glu Cys Ala Ser Asp Pro Cys Thr Thr Gly Ser
   785 790 795 800
   Thr Cys Val Asp Gly Ile Ala Ser Tyr Thr Cys Ile Cys Pro Pro Gly
   805 810 815
   Arg Thr Gly Val Arg Cys Glu Ile Arg Thr Ala Gly Gly Pro Gly Cys
   820 825 830
   Pro Val Ala Ser Trp Asp Asp Asp Cys Asn Val Cys Glu Cys Arg Asn
   835 840 845
   Gly Lys Asn Gln Cys Ser Asn Ile Trp Cys Gly Pro Gly Asn Cys Leu
   850 855 860
   Asn Gly Thr Ser Cys Leu Ala His Glu Val Cys Val Pro Ser Pro Asn
   865 870 875 880
   Glu Ser Cys Leu Ala Pro Pro Cys Pro Ala Trp Gly Glu Cys Arg Pro
   885 890 895
   Val Glu Thr Gly Arg Arg Val Gly Pro Pro Ala Leu Pro Ala Pro Pro
   900 905 910
   Ser Cys Trp Pro Gly Gln Ala Thr Pro Gly Pro Thr Cys Ser Arg Leu
   915 920 925
   Thr Ile Leu Leu Arg Arg Asp Thr Leu Ala Ala Ser Thr Ser Val Glu
   930 935 940
   Ile Leu Cys Arg Arg Leu Arg Lys Leu Leu Ser Asp Pro Arg Arg Pro
   945 950 955 960
   Gln Ser Ile Val Leu Leu Cys Asp Leu Lys Pro Gly Asp Asn Asp Thr
   965 970 975
   Val Glu Val Thr Ile Phe Ser Glu Ala Ala Ala Asp Ala Ala Arg Asp
   980 985 990
   Leu Gly Glu Ala Leu Ser Arg Pro Leu Gly Arg Pro Leu Ala Leu Ala
   995 1000 1005
   Ser Val Leu Glu Val Lys Val Glu Thr Ala Leu Leu Ser Glu Pro
   1010 1015 1020
   Ser Ser Val Asn Ser Ser Ser Ala Gly Ser Tyr Val Ala Val Leu
   1025 1030 1035
   Gly Gly Ala Leu Ala Thr Val Leu Val Leu Ala Leu Phe Gly Gly
   1040 1045 1050
   Leu Trp Tyr Leu Arg Thr Val Arg Gln Arg Ser Ser Leu Thr Ala
   1055 1060 1065
   Thr Thr Ser Ser Glu Thr Ser Leu His Arg His Arg Ser Asp Leu
   1070 1075 1080
   Asp Glu Lys Ser Asn Asn Phe Gln Asn Glu Glu Asn Leu Arg Arg
   1085 1090 1095
   Tyr Thr Asn Pro Leu Lys Glu Gln Asp Gln Gly Glu Pro Arg Val
   1100 1105 1110
   Ser Val Val Arg Pro Leu Ser Gly Thr Ser Leu Gly Ala Leu Gly
   1115 1120 1125
   Ala Thr Glu Glu Ser Leu Glu Met Val Ser Asp Glu Ser Arg His
   1130 1135 1140
   Arg Leu Pro Pro Leu Tyr Lys Ala Pro Ser Ala Glu Ala Arg Asn
   1145 1150 1155
   Asn Thr Ala Ser Phe Thr Tyr Glu Glu Gly Pro His Lys Pro Tyr
   1160 1165 1170
   Ser Lys Pro Arg Leu Gln Glu Pro Thr Tyr Ser Gln Gln Ala Ser
   1175 1180 1185
   Ser Ser Gln Thr Ser Gly Pro His Gln Val Leu Thr Val His Val
   1190 1195 1200
<210> 25
   <211> 1203
   <212> PRT
   <213> Apis mellifera
<400> 25
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Ala Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145                 150                 155                 160
   Leu Ala Tyr Arg Val Arg Val Gln Cys Ala Asp His Tyr Tyr Asn Ala
                   165                 170                 175
   Thr Cys Thr Lys Phe Cys Arg Pro Arg Asn Asp Ile Phe Gly His Tyr
               180                 185                 190
   Thr Cys Asp Glu Asn Gly Asp Lys Val Cys Ile Gln Gly Trp Lys Gly
           195                 200                 205
   Ala Asp Cys Glu Ile Ala Val Cys Lys Glu Gly Cys His Pro Val His
       210                 215                 220
   Gly His Cys Asn Val Ser Gly Glu Cys Lys Cys Arg His Gly Trp Arg
   225                 230                 235                 240
   Gly Glu Leu Cys Asp Gln Cys Thr Pro Tyr Pro Gly Cys Lys His Gly
                   245                 250                 255
   Tyr Cys Asn Gly Ser Ser Trp Gln Cys Ile Cys Asp Thr Asn Trp Gly
               260                 265                 270
   Gly Ile Leu Cys Asp Gln Asp Leu Asn Tyr Cys Gly Thr His Glu Pro
           275                 280                 285
   Cys Gln Asn Gly Gly Thr Cys Glu Asn Thr Ala Pro Asp Gln Tyr Lys
       290                 295                 300
   Cys Thr Cys Pro Glu Gly Phe Ser Gly Pro Thr Cys Glu Lys Val Asp
   305                 310                 315                 320
   Asn Pro Cys Ala Ser Asn Pro Cys Ala Asn Gly Ala Thr Cys Arg Glu
                   325                 330                 335
   Leu Gly Glu Ser Ala His Cys Glu Cys Ala Pro Gly Phe Ser Gly Pro
               340                 345                 350
   Tyr Cys Ala Thr Asp Ile Asp Glu Cys Ala Ser Gln Pro Cys Gln Asn
           355                 360                 365
   Gly Gly Thr Cys Val Asp Gly Lys Asn Glu Phe Val Cys Asn Cys Pro
       370                 375                 380
   Pro Ala Trp Gln Gly Val Leu Cys Gln Phe Asp Val Asp Glu Cys Ala
   385                 390                 395                 400
   Leu Lys Glu Ser Pro Cys Lys Asn Ser Leu Thr Cys Val Asn Leu Ala
                   405                 410                 415
   Gly Asp Tyr Lys Cys Arg Cys Arg Ser Gly Phe Thr Gly Lys Asn Cys
               420                 425                 430
   Thr Lys Asn Ile Asn Asp Cys Ile Gly Gln Cys Gln His Gly Ala Leu
           435                 440                 445
   Cys Ile Asp Leu Val Asp Asp Tyr His Cys Ser Cys Thr Ala Gly Tyr
       450                 455                 460
   Ser Gly Lys Asp Cys Asp Val Asp Ile Asp Glu Cys Ala Ser Lys Pro
   465                 470                 475                 480
   Cys Gln Asn Gly Gly Glu Cys Arg Asp Leu Val Asn Ala Tyr Glu Cys
                   485                 490                 495
   Val Cys Pro Val Gly Phe Thr Gly Tyr Gln Cys Glu Ile Asp Lys Asp
               500                 505                 510
   His Cys Ser Pro Asn Pro Cys Arg Asn Ser Ala Pro Cys Phe Asn Thr
           515                 520                 525
   Gln Thr Asp Tyr Tyr Cys His Cys Pro Glu Gln Trp Gln Gly Lys Asn
       530                 535                 540
   Cys Ser Glu Pro Ala Ser His Asn Pro Gln Phe Gly Met Met Asp Glu
   545                 550                 555                 560
   Glu Ser Gly Cys Gly Ser Glu Gly Thr Pro Cys Gly Gly Arg Gly Arg
                   565                 570                 575
   Cys Ser Gly Gly Arg Cys Ile Cys Asp Ala Gly Tyr Thr Gly Met His
               580                 585                 590
   Cys His Glu Asn Ile Asn Asp Cys Arg Gly Asn Pro Cys Leu Asn Gly
           595                 600                 605
   Gly Thr Cys Val Asp Leu Val Asn Ser Phe Gln Cys Ile Cys Arg Glu
       610                 615                 620
   Gly Trp Thr Gly Asp Leu Cys Asp Gln Asp Val Asp Glu Cys Thr Asn
   625                 630                 635                 640
   Ser Pro Cys Arg Asn Asn Gly Thr Cys Val Asp Gly Val Ala Asp Phe
                   645                 650                 655
   Thr Cys Ile Cys Arg Gly Gly Trp Lys Gly Lys Thr Cys Thr Leu Arg
               660                 665                 670
   Ala Gly His Cys Glu Pro Gly Thr Cys Arg His Gly Gly Thr Cys Gln
           675                 680                 685
   Asp Arg Gly Asp Gly Phe Thr Cys His Cys Pro Pro Gly Trp Glu Gly
       690                 695                 700
   Ala Ala Cys His Ile Ala Ser Pro Ala Cys Ala Ser Asn Pro Cys Glu
   705                 710                 715                 720
   Asn Gly Ala Thr Cys Val Asn Thr Ala Asp Gly Asn Tyr Arg Cys Val
   725 730 735
   Cys Arg Glu Gly Phe Glu Gly Pro Asn Cys Arg Arg Asp Val Asp Asp
   740 745 750
   Cys Gln Pro Leu Pro Cys Leu Asn Gly Gly Lys Cys Val Asp Gly Ile
   755 760 765
   Asn Trp Phe Arg Cys Glu Cys Ala Pro Gly Phe Thr Gly Pro Asp Cys
   770 775 780
   Arg Ile Asn Val Asn Glu Cys Ala Ser Asp Pro Cys Thr Thr Gly Ser
   785 790 795 800
   Thr Cys Val Asp Gly Ile Ala Ser Tyr Thr Cys Ile Cys Pro Pro Gly
   805 810 815
   Arg Thr Gly Val Arg Cys Glu Ile Arg Thr Ala Gly Gly Pro Gly Cys
   820 825 830
   Pro Val Ala Ser Trp Asp Asp Asp Cys Asn Val Cys Glu Cys Arg Asn
   835 840 845
   Gly Lys Asn Gln Cys Ser Asn Ile Trp Cys Gly Pro Gly Asn Cys Leu
   850 855 860
   Asn Gly Thr Ser Cys Leu Ala His Glu Val Cys Val Pro Ser Pro Asn
   865 870 875 880
   Glu Ser Cys Leu Ala Pro Pro Cys Pro Ala Trp Gly Glu Cys Arg Pro
   885 890 895
   Val Glu Thr Gly Arg Arg Val Gly Pro Pro Ala Leu Pro Ala Pro Pro
   900 905 910
   Ser Cys Trp Pro Gly Gln Ala Thr Pro Gly Pro Thr Cys Ser Arg Leu
   915 920 925
   Thr Ile Leu Leu Arg Arg Asp Thr Leu Ala Ala Ser Thr Ser Val Glu
   930 935 940
   Ile Leu Cys Arg Arg Leu Arg Lys Leu Leu Ser Asp Pro Arg Arg Pro
   945 950 955 960
   Gln Ser Ile Val Leu Leu Cys Asp Leu Lys Pro Gly Asp Asn Asp Thr
   965 970 975
   Val Glu Val Thr Ile Phe Ser Glu Ala Ala Ala Asp Ala Ala Arg Asp
               980                 985                 990
   Leu Gly Glu Ala Leu Ser Arg Pro  Leu Gly Arg Pro Leu  Ala Leu Ala
           995                 1000                 1005
   Ser Val  Leu Glu Val Lys Val  Glu Thr Ala Leu Leu  Ser Glu Pro
       1010                 1015                 1020
   Ser Ser  Val Asn Ser Ser Ser  Ala Gly Ser Tyr Val  Ala Val Leu
       1025                 1030                 1035
   Gly Gly  Ala Leu Ala Thr Val  Leu Val Leu Ala Leu  Phe Gly Gly
       1040                 1045                 1050
   Leu Trp  Tyr Leu Arg Thr Val  Arg Gln Arg Ser Ser  Leu Thr Ala
       1055                 1060                 1065
   Thr Thr  Ser Ser Glu Thr Ser  Leu His Arg His Arg  Ser Asp Leu
       1070                 1075                 1080
   Asp Glu  Lys Ser Asn Asn Phe  Gln Asn Glu Glu Asn  Leu Arg Arg
       1085                 1090                 1095
   Tyr Val  Asn Pro Leu Lys Glu  Gln Asp Gln Gly Glu  Pro Arg Val
       1100                 1105                 1110
   Ser Val  Val Arg Pro Leu Ser  Gly Thr Ser Leu Gly  Ala Leu Gly
       1115                 1120                 1125
   Ala Thr  Glu Glu Ser Leu Glu  Met Val Ser Asp Glu  Ser Arg His
       1130                 1135                 1140
   Arg Leu  Pro Pro Leu Tyr Lys  Ala Pro Ser Ala Glu  Ala Arg Asn
       1145                 1150                 1155
   Asn Thr  Ala Ser Phe Thr Tyr  Glu Glu Gly Pro His  Lys Pro Tyr
       1160                 1165                 1170
   Ser Lys  Pro Arg Leu Gln Glu  Pro Thr Tyr Ser Gln  Gln Ala Ser
       1175                 1180                 1185
   Ser Ser  Gln Thr Ser Gly Pro  His Gln Val Leu Thr  Val His Val
       1190                 1195                 1200
<210> 26
   <211> 1203
   <212> PRT
   <213> Apis mellifera
<400> 26
   Met Arg Ala Ala Ala Ala Tyr Val Leu Phe Leu Ala His Leu Ile Gln
   1               5                   10                  15
   Ala Thr Asn Ala Ser Gly Phe Phe Glu Val Gln Ile Leu Ser Leu Thr
               20                  25                  30
   Asn Asn Arg Gly Thr Leu Val Asp Gly Arg Cys Cys Gly Gly Gly Gly
           35                  40                  45
   Asp Gly Gly Gly Lys Gly Glu Leu Pro Pro Cys Thr Thr Pro Cys Ser
       50                  55                  60
   Thr Ala Phe Trp Leu Cys Leu Lys Glu Tyr Gln Ser Asn Val Thr Ala
   65                  70                  75                  80
   Ile Gly Ser Cys Ser Phe Gly Asn Val Ser Ser His Ala Leu Gly Gln
                   85                  90                  95
   Asn Thr Phe Thr Leu Ser Glu Pro Val Thr Leu Gln Leu His Phe Thr
               100                 105                 110
   Phe Arg Trp Thr Arg Gln Phe Thr Leu Ile Leu Gln Ala Arg Asp Glu
           115                 120                 125
   Ala Ser Thr Gly Val Ile Glu Glu Ala Ser Phe Ser Gly Ile Val Leu
       130                 135                 140
   Pro Gly Pro Thr Trp His Thr Leu Asn His Glu Gly Arg Asn Ala His
   145                 150                 155                 160
   Leu Ala Tyr Arg Val Arg Val Gln Cys Ala Asp His Tyr Tyr Asn Ala
                   165                 170                 175
   Thr Cys Thr Lys Phe Cys Arg Pro Arg Asn Asp Ile Phe Gly His Tyr
               180                 185                 190
   Thr Cys Asp Glu Asn Gly Asp Lys Val Cys Ile Gln Gly Trp Lys Gly
           195                 200                 205
   Ala Asp Cys Glu Ile Ala Val Cys Lys Glu Gly Cys His Pro Val His
       210                 215                 220
   Gly His Cys Asn Val Ser Gly Glu Cys Lys Cys Arg His Gly Trp Arg
   225                 230                 235                 240
   Gly Glu Leu Cys Asp Gln Cys Thr Pro Tyr Pro Gly Cys Lys His Gly
                   245                 250                 255
   Tyr Cys Asn Gly Ser Ser Trp Gln Cys Ile Cys Asp Thr Asn Trp Gly
               260                 265                 270
   Gly Ile Leu Cys Asp Gln Asp Leu Asn Tyr Cys Gly Thr His Glu Pro
           275                 280                 285
   Cys Gln Asn Gly Gly Thr Cys Glu Asn Thr Ala Pro Asp Gln Tyr Lys
       290                 295                 300
   Cys Thr Cys Pro Glu Gly Phe Ser Gly Pro Thr Cys Glu Lys Val Asp
   305                 310                 315                 320
   Asn Pro Cys Ala Ser Asn Pro Cys Ala Asn Gly Ala Thr Cys Arg Glu
                   325                 330                 335
   Leu Gly Glu Ser Ala His Cys Glu Cys Ala Pro Gly Phe Ser Gly Pro
               340                 345                 350
   Tyr Cys Ala Thr Asp Ile Asp Glu Cys Ala Ser Gln Pro Cys Gln Asn
           355                 360                 365
   Gly Gly Thr Cys Val Asp Gly Lys Asn Glu Phe Val Cys Asn Cys Pro
       370                 375                 380
   Pro Ala Trp Gln Gly Val Leu Cys Gln Phe Asp Val Asp Glu Cys Ala
   385                 390                 395                 400
   Leu Lys Glu Ser Pro Cys Lys Asn Ser Leu Thr Cys Val Asn Leu Ala
                   405                 410                 415
   Gly Asp Tyr Lys Cys Arg Cys Arg Ser Gly Phe Thr Gly Lys Asn Cys
               420                 425                 430
   Thr Lys Asn Ile Asn Asp Cys Ile Gly Gln Cys Gln His Gly Ala Leu
           435                 440                 445
   Cys Ile Asp Leu Val Asp Asp Tyr His Cys Ser Cys Thr Ala Gly Tyr
       450                 455                 460
   Ser Gly Lys Asp Cys Asp Val Asp Ile Asp Glu Cys Ala Ser Lys Pro
   465                 470                 475                 480
   Cys Gln Asn Gly Gly Glu Cys Arg Asp Leu Val Asn Ala Tyr Glu Cys
   485 490 495
   Val Cys Pro Val Gly Phe Thr Gly Tyr Gln Cys Glu Ile Asp Lys Asp
   500 505 510
   His Cys Ser Pro Asn Pro Cys Arg Asn Ser Ala Pro Cys Phe Asn Thr
   515 520 525
   Gln Thr Asp Tyr Tyr Cys His Cys Pro Glu Gln Trp Gln Gly Lys Asn
   530 535 540
   Cys Ser Glu Pro Ala Ser His Asn Pro Gln Phe Gly Met Met Asp Glu
   545 550 555 560
   Glu Ser Gly Cys Gly Ser Glu Gly Thr Pro Cys Gly Gly Arg Gly Arg
   565 570 575
   Cys Ser Gly Gly Arg Cys Ile Cys Asp Ala Gly Tyr Thr Gly Met His
   580 585 590
   Cys His Glu Asn Ile Asn Asp Cys Arg Gly Asn Pro Cys Leu Asn Gly
   595 600 605
   Gly Thr Cys Val Asp Leu Val Asn Ser Phe Gln Cys Ile Cys Arg Glu
   610 615 620
   Gly Trp Thr Gly Asp Leu Cys Asp Gln Asp Val Asp Glu Cys Thr Asn
   625 630 635 640
   Ser Pro Cys Arg Asn Asn Gly Thr Cys Val Asp Gly Val Ala Asp Phe
   645 650 655
   Thr Cys Ile Cys Arg Gly Gly Trp Lys Gly Lys Thr Cys Thr Leu Arg
   660 665 670
   Ala Gly His Cys Glu Pro Gly Thr Cys Arg His Gly Gly Thr Cys Gln
   675 680 685
   Asp Arg Gly Asp Gly Phe Thr Cys His Cys Pro Pro Gly Trp Glu Gly
   690 695 700
   Ala Ala Cys His Ile Ala Ser Pro Ala Cys Ala Ser Asn Pro Cys Glu
   705 710 715 720
   Asn Gly Ala Thr Cys Val Asn Thr Ala Asp Gly Asn Tyr Arg Cys Val
   725 730 735
   Cys Arg Glu Gly Phe Glu Gly Pro Asn Cys Arg Arg Asp Val Asp Asp
               740                 745                 750
   Cys Gln Pro Leu Pro Cys Leu Asn Gly Gly Lys Cys Val Asp Gly Ile
           755                 760                 765
   Asn Trp Phe Arg Cys Glu Cys Ala Pro Gly Phe Thr Gly Pro Asp Cys
       770                 775                 780
   Arg Ile Asn Val Asn Glu Cys Ala Ser Asp Pro Cys Thr Thr Gly Ser
   785                 790                 795                 800
   Thr Cys Val Asp Gly Ile Ala Ser Tyr Thr Cys Ile Cys Pro Pro Gly
                   805                 810                 815
   Arg Thr Gly Val Arg Cys Glu Ile Arg Thr Ala Gly Gly Pro Gly Cys
               820                 825                 830
   Pro Val Ala Ser Trp Asp Asp Asp Cys Asn Val Cys Glu Cys Arg Asn
           835                 840                 845
   Gly Lys Asn Gln Cys Ser Asn Ile Trp Cys Gly Pro Gly Asn Cys Leu
       850                 855                 860
   Asn Gly Thr Ser Cys Leu Ala His Glu Val Cys Val Pro Ser Pro Asn
   865                 870                 875                 880
   Glu Ser Cys Leu Ala Pro Pro Cys Pro Ala Trp Gly Glu Cys Arg Pro
                   885                 890                 895
   Val Glu Thr Gly Arg Arg Val Gly Pro Pro Ala Leu Pro Ala Pro Pro
               900                 905                 910
   Ser Cys Trp Pro Gly Gln Ala Thr Pro Gly Pro Thr Cys Ser Arg Leu
           915                 920                 925
   Thr Ile Leu Leu Arg Arg Asp Thr Leu Ala Ala Ser Thr Ser Val Glu
       930                 935                 940
   Ile Leu Cys Arg Arg Leu Arg Lys Leu Leu Ser Asp Pro Arg Arg Pro
   945                 950                 955                 960
   Gln Ser Ile Val Leu Leu Cys Asp Leu Lys Pro Gly Asp Asn Asp Thr
                   965                 970                 975
   Val Glu Val Thr Ile Phe Ser Glu Ala Ala Ala Asp Ala Ala Arg Asp
               980                 985                 990
   Leu Gly Glu Ala Leu Ser Arg Pro  Leu Gly Arg Pro Leu  Ala Leu Ala
           995                 1000                 1005
   Ser Val  Leu Glu Val Lys Val  Glu Thr Ala Leu Leu  Ser Glu Pro
       1010                 1015                 1020
   Ser Ser  Val Asn Ser Ser Ser  Ala Gly Ser Tyr Val  Ala Val Leu
       1025                 1030                 1035
   Gly Gly  Ala Leu Ala Thr Val  Leu Val Leu Ala Leu  Phe Gly Gly
       1040                 1045                 1050
   Leu Trp  Tyr Leu Arg Thr Val  Arg Gln Arg Ser Ser  Leu Thr Ala
       1055                 1060                 1065
   Thr Thr  Ser Ser Glu Thr Ser  Leu His Arg His Arg  Ser Asp Leu
       1070                 1075                 1080
   Asp Glu  Lys Ser Asn Asn Phe  Gln Asn Glu Glu Asn  Leu Arg Arg
       1085                 1090                 1095
   Tyr Ala  Asn Pro Leu Lys Glu  Gln Asp Gln Gly Glu  Pro Arg Val
       1100                 1105                 1110
   Ser Val  Val Arg Pro Leu Ser  Gly Thr Ser Leu Gly  Ala Leu Gly
       1115                 1120                 1125
   Ala Thr  Glu Glu Ser Leu Glu  Met Val Ser Asp Glu  Ser Arg His
       1130                 1135                 1140
   Arg Leu  Pro Pro Leu Tyr Lys  Ala Pro Ser Ala Glu  Ala Arg Asn
       1145                 1150                 1155
   Asn Thr  Ala Ser Phe Thr Tyr  Glu Glu Gly Pro His  Lys Pro Tyr
       1160                 1165                 1170
   Ser Lys  Leu Arg Leu Gln Glu  Pro Thr Tyr Ser Gln  Gln Ala Ser
       1175                 1180                 1185
   Ser Ser  Gln Thr Ser Gly Pro  His Gln Val Leu Thr  Val His Val
       1190                 1195                 1200